# EUROPÄISCHE PATENTSCHRIFT

(11) **EP 4 721 092 B1**
(45) Veröffentlichungstag und Bekanntmachung des Hinweises auf die Patenterteilung: **02.12.2026**
(21) Anmeldenummer: 24728242.9
(22) Anmeldetag: 30.05.2024
(51) Int. Cl.: G16H 30/40, A61B 6/00, A61B 6/50, G06N 3/09, G06N 3/045, G06N 3/088, G06N 3/096, A61B 5/055, A61B 6/03, G16H 50/70

(54) **ERZEUGEN VON KÜNSTLICHEN KONTRASTVERSTÄRKTEN RADIOLOGISCHEN AUFNAHMEN**
GENERATION OF ARTIFICIAL CONTRAST ENHANCED RADIOLOGICAL RECORDINGS
GÉNÉRATION D'ENREGISTREMENTS RADIOLOGIQUES ARTIFICIELS À CONTRASTE AMÉLIORÉ

(30) Priorität: 05.06.2023 EP 23177301; 13.07.2023 EP 23185296
(43) Veröffentlichungstag der Anmeldung: 08.04.2026
(73) Patentinhaber: Bayer Aktiengesellschaft, 51373 Leverkusen (DE)
(72) Erfinder: LENGA, Matthias, 51373 Leverkusen (DE); BALTRUSCHAT, Ivo Matteo, 51373 Leverkusen (DE); JANBAKHSHI, Parvaneh, 51373 Leverkusen (DE); KREIS, Felix Karl, 51373 Leverkusen (DE)
(74) Vertreter: BIP Patents
(86) Internationale Anmeldenummer: PCT/EP2024/064881
(87) Internationale Veröffentlichungsnummer: WO 2024/251601

(56) Entgegenhaltungen:
- EP-A1- 4 044 120
- WO-A1-2022/194777
- US-A1- 2021 241 458
- MONTALT-TORDERA JAVIER ET AL: "Angiography with Deep Learning", JOURNAL OF MAGNETIC RESONANCE IMAGING, vol. 54, no. 3, 22 February 2021 (2021-02-22), US, pages 795 - 805, XP055982847, ISSN: 1053-1807, DOI: 10.1002/jmri.27573
- RINGE KRISTINA I. ET AL: "Gadoxetate Disodium-Enhanced MRI of the Liver: Part 1, Protocol Optimization and Lesion Appearance in the Noncirrhotic Liver", AMERICAN JOURNAL OF ROENTGENOLOGY, vol. 195, no. 1, 1 July 2010 (2010-07-01), US, pages 13 - 28, XP093048379, ISSN: 0361-803X, DOI: 10.2214/AJR.10.4392

## Beschreibung

### TECHNISCHES GEBIET

Die vorliegende Offenbarung befasst sich mit dem technischen Gebiet der Erzeugung von künstlichen kontrastverstärkten radiologischen Aufnahmen.

### EINLEITUNG

EP4044120A1 offenbart ein System zum Synthetisieren von Trainingsdaten für maschinelles Lernen, ein Trainingssystem zum Trainieren eines maschinellen Lernmodells, ein System zur Bildverbesserung, ein Verfahren zum Synthetisieren von Trainingsdaten für maschinelles Lernen, ein Verfahren zum Trainieren eines maschinellen Lernmodells, ein Verfahren zur Bildverbesserung, eine Abbildungsanordnung, ein Computerprogrammelement und ein computerlesbares Medium.

J. Montalt-Tordera *et al.* offenbaren eine Verringerung der Kontrastmitteldosis bei kardiovaskulärer MR-Angiographie mittels Deep Learning (J. Montalt-Tordera et al.: Reducing Contrast Agent Dose in Cardiovascular MR Angiography with Deep Learning, Journal of Magnetic Resonance Imaging Vol. 54, 3, 2021, 795-805).

WO2022/194777A1 offenbart Gd³⁺-Chelatkomplexe, Verfahren zu ihrer Herstellung und die Verwendung dieser Verbindungen als MRT-Kontrastmittel.

K. I. Ringe *et al.* offenbaren Gadoxetat-Dinatrium-verstärkte MRT der Leber (K. I. Ringe et al.: Gadoxetate disodium-enhanced MRI of the liver: part 1, protocol optimization and lesion appearance in the noncirrhotic liver, AJR Am J Roentgenol. 2010, 195(1):13-28).

WO2019/074938A1 offenbart ein Verfahren zur Reduzierung der Menge an Kontrastmittel bei der Erzeugung von radiologischen Aufnahmen mit Hilfe eines künstlichen neuronalen Netzes.

In dem offenbarten Verfahren wird in einem ersten Schritt ein Trainingsdatensatz erzeugt. Der Trainingsdatensatz umfasst für jede Person einer Vielzahl an Personen i) eine native radiologische Aufnahme (*zero-contrast image*), ii) eine radiologische Aufnahme nach der Applikation einer geringen Menge an Kontrastmittel (*low-contrast image*) und iii) eine radiologische Aufnahme nach der Applikation einer Standardmenge an Kontrastmittel (*full-contrast image*). Die Standardmenge ist die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

In einem zweiten Schritt wird ein künstliches neuronales Netz trainiert, für jede Person des Trainingsdatensatzes auf Basis der nativen Aufnahme und der Aufnahme nach Applikation einer geringeren Menge an Kontrastmittel als der Standardmenge eine künstliche radiologische Aufnahme vorherzusagen, die einen Aufnahmebereich nach der Applikation der Standardmenge an Kontrastmittel zeigt. Die gemessene radiologische Aufnahme nach der Applikation einer Standardmenge an Kontrastmittel dient beim Training jeweils als Referenz (*ground truth*).

In einem dritten Schritt kann das trainierte künstliche neuronale Netz verwendet werden, für eine neue Person auf Basis einer nativen Aufnahme und einer radiologischen Aufnahme nach der Applikation einer geringeren Menge an Kontrastmittel als der Standardmenge eine künstliche radiologische Aufnahme vorherzusagen, die den aufgenommenen Bereich so zeigt wie er aussehen würde, wenn die Standardmenge an Kontrastmittel appliziert worden wäre.

Das in WO2019/074938A1 offenbarte Verfahren weist Nachteile auf.

Das in WO2019/074938A1 offenbarte künstliche neuronale Netz wird trainiert, eine radiologische Aufnahme nach der Applikation der Standardmenge eines Kontrastmittels vorherzusagen. Das künstliche neuronale Netz ist nicht konfiguriert und nicht trainiert, eine radiologische Aufnahme nach der Applikation einer geringeren oder höheren Menge als der Standarmenge an Kontrastmittel vorherzusagen. Das in WO2019/074938A1 beschriebene Verfahren kann prinzipiell trainiert werden, eine radiologische Aufnahme nach der Applikation einer anderen Menge als der Standardmenge an Kontrastmittel vorherzusagen - allerdings sind hierfür weitere Trainingsdaten und ein weiteres Training erforderlich.

Die von trainierten Modellen des maschinellen Lernens erzeugten medizinischen Aufnahmen können Fehler aufweisen (siehe z.B.: K. Schwarz et al.: On the Frequency Bias of Generative Models, https://doi.org/10.48550/arXiv.2111.02447).

Solche Fehler (Artefakte) können problematisch sein, da ein Arzt auf Basis der künstlichen medizinischen Aufnahmen eine Diagnose stellen und/oder eine Therapie initiieren könnte. Wenn ein Arzt künstliche medizinische Aufnahmen begutachtet, muss der Arzt wissen, ob Merkmale in den künstlichen medizinischen Aufnahmen auf reale Merkmale des Untersuchungsobjekts zurückgeführt werden können, oder ob es sich um Artefakte handelt, die auf Fehler bei der Vorhersage durch das trainierte Modell des maschinellen Lernens zurückzuführen sind.

Es wäre wünschenswert, radiologische Aufnahmen mit einer variablen Kontrastverstärkung erzeugen zu können, ohne dass für jede einzelne Kontrastverstärkung Trainingsdaten erzeugt und ein künstliches neuronales Netz trainiert werden müssen. Es wäre weiterhin wünschenswert radiologische Aufnahmen mit einer variablen Kontrastverstärkung erzeugen zu können, wobei ein nachvollziehbarer, deterministischer Prozess zur Erzeugung der variablen Kontrastverstärkung angewendet wird. Dies erleichtert Zulassung und Anwendung eines entsprechenden Verfahrens im medizinischen Bereich, in dem falsch negative und falsch positive Befunde zu minimieren sind. Methoden des maschinellen Lernens nutzen statistische Modelle, deren Generalisierbarkeit beschränkt ist, da ihnen üblicherweise eine beschränkte Auswahl an Trainingsdaten zu Grunde liegt. Es wäre weiterhin wünschenswert, radiologische Aufnahmen mit einer variablen Kontrastverstärkung unter Verwendung einer breiten Vielfalt von Kontrastmitteln erzeugen zu können. Es wäre weiterhin wünschenswert, das Verfahren zur Erzeugung radiologischer Aufnahmen mit einer variablen Kontrastverstärkung mit einer breiten Vielfalt verschiedener Kontrastmittel unabhängig von ihren physischen, chemischen, physiologischen oder sonstigen Eigenschaften anwenden zu können. Es wäre weiterhin wünschenswert, radiologische Aufnahmen mit einer variablen Kontrastverstärkung erzeugen zu können, die weniger Fehler (Artefakte) aufweisen.

### ZUSAMMENFASSUNG

Diese und weitere Aufgaben werden durch die Gegenstände der unabhängigen Patentansprüche gelöst. Bevorzugte Ausführungsformen der vorliegenden Offenbarung finden sich in den abhängigen Patentansprüchen, in der vorliegenden Beschreibung und in den Zeichnungen.

Ein erster Gegenstand der vorliegenden Offenbarung ist somit ein computer-implementiertes Verfahren zum Erzeugen einer synthetischen kontrastverstärkten radiologischen Aufnahme, wobei das Verfahren umfasst:
- Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
   wobei das Erzeugen der dritten Repräsentation durch das erste Modell umfasst:
   ∘ Subtrahieren der ersten Repräsentation von der zweiten Repräsentation,
   ∘ Multiplizieren des Ergebnisses des Subtrahierens mit einem Verstärkungsfaktor α,
   ∘ Addieren des Ergebnisses des Multiplizierens auf die erste Repräsentation,
- Zuführen der dritten Repräsentation einem zweiten Modell,
   ∘ wobei das zweite Modell in einem Trainingsverfahren auf Basis von Trainingsdaten trainiert wurde,
   ∘ wobei die Trainingsdaten für jedes Referenzobjekt einer Vielzahl an Referenzobjekten
      (i) eine von dem ersten Modell erzeugte Referenz-Repräsentation, die einen Referenzbereich des Referenzobjekts nach Applikation einer Referenzmenge des Kontrastmittels repräsentiert, und
      (ii) eine gemessene Referenz-Repräsentation des Referenzbereichs des Referenzobjekts nach Applikation der Referenzmenge des Kontrastmittels
      umfasst,
   ∘ wobei das Trainingsverfahren für jedes Referenzobjekt umfasst:
      ▪ Zuführen der von dem ersten Modell erzeugten Referenz-Repräsentation dem zweiten Modell,_wobei das zweite Modell auf Basis der von dem ersten Modell erzeugten Referenz-Repräsentation und auf Basis von Modellparametern eine korrigierte Referenz-Repräsentation erzeugt,
      ▪ Empfangen der korrigierten Referenz-Repräsentation von dem zweiten Modell,
      ▪ Reduzieren von Abweichungen zwischen der korrigierten Referenz-Repräsentation und der gemessenen Referenz-Repräsentation durch Modifizieren von Modellparametern,
- Empfangen einer korrigierten dritten Repräsentation des Untersuchungsbereichs des Untersuchungsobjekts von dem zweiten Modell,
- Ausgeben und/oder Speichern der korrigierten dritten Repräsentation und/oder Übermitteln der korrigierten dritten Repräsentation an ein separates Computersystem.

Ein weiterer Gegenstand der vorliegenden Offenbarung ist ein Computersystem umfassend:
einen Prozessor; und
einen Speicher, der ein Anwendungsprogramm speichert, das so konfiguriert ist, dass es, wenn es vom Prozessor ausgeführt wird, eine Operation durchführt, wobei die Operation umfasst:
   - Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
   - Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
   - Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
wobei das Erzeugen der dritten Repräsentation durch das erste Modell umfasst:
∘ Subtrahieren der ersten Repräsentation von der zweiten Repräsentation,
∘ Multiplizieren des Ergebnisses des Subtrahierens mit einem Verstärkungsfaktor α,
∘ Addieren des Ergebnisses des Multiplizierens auf die erste Repräsentation,
   - Zuführen der dritten Repräsentation einem zweiten Modell,
      ∘ wobei das zweite Modell in einem Trainingsverfahren auf Basis von Trainingsdaten trainiert wurde,
      ∘ wobei die Trainingsdaten für jedes Referenzobjekt einer Vielzahl an Referenzobjekten
         (i) eine von dem ersten Modell erzeugte Referenz-Repräsentation, die einen Referenzbereich des Referenzobjekts nach Applikation einer Referenzmenge des Kontrastmittels repräsentiert, und
         (ii) eine gemessene Referenz-Repräsentation des Referenzbereichs des Referenzobjekts nach Applikation der Referenzmenge des Kontrastmittels
         umfasst,
      ∘ wobei das Trainingsverfahren für jedes Referenzobjekt umfasst:
         ▪ Zuführen der von dem ersten Modell erzeugten Referenz-Repräsentation dem zweiten Modell,_wobei das zweite Modell auf Basis der von dem ersten Modell erzeugten Referenz-Repräsentation und auf Basis von Modellparametern eine korrigierte Referenz-Repräsentation erzeugt,
         ▪ Empfangen der korrigierten Referenz-Repräsentation von dem zweiten Modell,
         ▪ Reduzieren von Abweichungen zwischen der korrigierten Referenz-Repräsentation und der gemessenen Referenz-Repräsentation durch Modifizieren von Modellparametern,
   - Empfangen einer korrigierten dritten Repräsentation des Untersuchungsbereichs des Untersuchungsobjekts von dem zweiten Modell,
   - Ausgeben und/oder Speichern der korrigierten dritten Repräsentation und/oder Übermitteln der korrigierten dritten Repräsentation an ein separates Computersystem.

Ein weiterer Gegenstand der vorliegenden Offenbarung ist ein Computerprogramm, das in einen Arbeitsspeicher eines Computersystems geladen werden kann und dort das Computersystem dazu veranlasst, folgende Schritte ausführen:
- Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
wobei das Erzeugen der dritten Repräsentation durch das erste Modell umfasst:
∘ Subtrahieren der ersten Repräsentation von der zweiten Repräsentation,
∘ Multiplizieren des Ergebnisses des Subtrahierens mit einem Verstärkungsfaktor α,
∘ Addieren des Ergebnisses des Multiplizierens auf die erste Repräsentation,
   - Zuführen der dritten Repräsentation einem zweiten Modell,
      ∘ wobei das zweite Modell in einem Trainingsverfahren auf Basis von Trainingsdaten trainiert wurde,
      ∘ wobei die Trainingsdaten für jedes Referenzobjekt einer Vielzahl an Referenzobjekten
         (i) eine von dem ersten Modell erzeugte Referenz-Repräsentation, die einen Referenzbereich des Referenzobjekts nach Applikation einer Referenzmenge des Kontrastmittels repräsentiert, und
         (ii) eine gemessene Referenz-Repräsentation des Referenzbereichs des Referenzobjekts nach Applikation der Referenzmenge des Kontrastmittels umfasst,
      ∘ wobei das Trainingsverfahren für jedes Referenzobjekt umfasst:
         ▪ Zuführen der von dem ersten Modell erzeugten Referenz-Repräsentation dem zweiten Modell,_wobei das zweite Modell auf Basis der von dem ersten Modell erzeugten Referenz-Repräsentation und auf Basis von Modellparametern eine korrigierte Referenz-Repräsentation erzeugt,
         ▪ Empfangen der korrigierten Referenz-Repräsentation von dem zweiten Modell,
         ▪ Reduzieren von Abweichungen zwischen der korrigierten Referenz-Repräsentation und der gemessenen Referenz-Repräsentation durch Modifizieren von Modellparametern,
   - Empfangen einer korrigierten dritten Repräsentation des Untersuchungsbereichs des Untersuchungsobjekts von dem zweiten Modell,
   - Ausgeben und/oder Speichern der korrigierten dritten Repräsentation und/oder Übermitteln der korrigierten dritten Repräsentation an ein separates Computersystem.

Ein weiterer Gegenstand der vorliegenden Offenbarung ist eine Verwendung eines Kontrastmittels in einem radiologischen Untersuchungsverfahren umfassend:
- Bereitstellen einer ersten Repräsentation, wobei die erste Repräsentation einen Untersuchungsbereich eines Untersuchungsobjekts ohne das Kontrastmittel oder nach Applikation einer ersten Menge des Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
   wobei das Erzeugen der dritten Repräsentation durch das erste Modell umfasst:
   ∘ Subtrahieren der ersten Repräsentation von der zweiten Repräsentation,
   ∘ Multiplizieren des Ergebnisses des Subtrahierens mit einem Verstärkungsfaktor α,
   ∘ Addieren des Ergebnisses des Multiplizierens auf die erste Repräsentation,
- Zuführen der dritten Repräsentation einem zweiten Modell,
   ∘ wobei das zweite Modell in einem Trainingsverfahren auf Basis von Trainingsdaten trainiert wurde,
   ∘ wobei die Trainingsdaten für jedes Referenzobjekt einer Vielzahl an Referenzobjekten
      (i) eine von dem ersten Modell erzeugte Referenz-Repräsentation, die einen Referenzbereich des Referenzobjekts nach Applikation einer Referenzmenge des Kontrastmittels repräsentiert, und
      (ii) eine gemessene Referenz-Repräsentation des Referenzbereichs des Referenzobjekts nach Applikation der Referenzmenge des Kontrastmittels
      umfasst,
   ∘ wobei das Trainingsverfahren für jedes Referenzobjekt umfasst:
      ▪ Zuführen der von dem ersten Modell erzeugten Referenz-Repräsentation dem zweiten Modell,_wobei das zweite Modell auf Basis der von dem ersten Modell erzeugten Referenz-Repräsentation und auf Basis von Modellparametern eine korrigierte Referenz-Repräsentation erzeugt,
      ▪ Empfangen der korrigierten Referenz-Repräsentation von dem zweiten Modell,
      ▪ Reduzieren von Abweichungen zwischen der korrigierten Referenz-Repräsentation und der gemessenen Referenz-Repräsentation durch Modifizieren von Modellparametern,
- Empfangen einer korrigierten dritten Repräsentation des Untersuchungsbereichs des Untersuchungsobjekts von dem zweiten Modell,
- Ausgeben und/oder Speichern der korrigierten dritten Repräsentation und/oder Übermitteln der korrigierten dritten Repräsentation an ein separates Computersystem.

Ein weiterer Gegenstand der vorliegenden Offenbarung ist ein Kit umfassend ein Computerprogrammprodukt und ein Kontrastmittel, wobei das Computerprogrammprodukt ein Computerprogramm umfasst, das in einen Arbeitsspeicher eines Computersystems geladen werden kann und dort das Computersystem dazu veranlasst, folgende Schritte ausführen:
- Bereitstellen einer ersten Repräsentation, wobei die erste Repräsentation einen Untersuchungsbereich eines Untersuchungsobjekts ohne das Kontrastmittel oder nach Applikation einer ersten Menge des Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
   wobei das Erzeugen der dritten Repräsentation durch das erste Modell umfasst:
   ∘ Subtrahieren der ersten Repräsentation von der zweiten Repräsentation,
   ∘ Multiplizieren des Ergebnisses des Subtrahierens mit einem Verstärkungsfaktor α,
   ∘ Addieren des Ergebnisses des Multiplizierens auf die erste Repräsentation,
- Zuführen der dritten Repräsentation einem zweiten Modell,
   ∘ wobei das zweite Modell in einem Trainingsverfahren auf Basis von Trainingsdaten trainiert wurde,
   ∘ wobei die Trainingsdaten für jedes Referenzobjekt einer Vielzahl an Referenzobjekten
      (i) eine von dem ersten Modell erzeugte Referenz-Repräsentation, die einen Referenzbereich des Referenzobjekts nach Applikation einer Referenzmenge des Kontrastmittels repräsentiert, und
      (ii) eine gemessene Referenz-Repräsentation des Referenzbereichs des Referenzobjekts nach Applikation der Referenzmenge des Kontrastmittels
      umfasst,
   ∘ wobei das Trainingsverfahren für jedes Referenzobjekt umfasst:
      ▪ Zuführen der von dem ersten Modell erzeugten Referenz-Repräsentation dem zweiten Modell, Zuführen der von dem ersten Modell erzeugten Referenz-Repräsentation dem zweiten Modell, wobei das zweite Modell auf Basis der von dem ersten Modell erzeugten Referenz-Repräsentation und auf Basis von Modellparametern eine korrigierte Referenz-Repräsentation erzeugt,
      ▪ Empfangen der korrigierten Referenz-Repräsentation von dem zweiten Modell,
      ▪ Reduzieren von Abweichungen zwischen der korrigierten Referenz-Repräsentation und der gemessenen Referenz-Repräsentation durch Modifizieren von Modellparametern,
- Empfangen einer korrigierten dritten Repräsentation des Untersuchungsbereichs des Untersuchungsobjekts von dem zweiten Modell,
- Ausgeben und/oder Speichern der korrigierten dritten Repräsentation und/oder Übermitteln der korrigierten dritten Repräsentation an ein separates Computersystem.

### KURZE BESCHREIBUNG DER ZEICHNUNGEN

Fig. 1 zeigt beispielhaft und schematisch die Erzeugung einer dritten Repräsentation eines Untersuchungsbereichs eines Untersuchungsobjekts auf Basis einer ersten Repräsentation und einer zweiten Repräsentation mit Hilfe eines ersten Modells.
Fig. 2 zeigt schematisch ein weiteres Beispiel der Erzeugung einer dritten Repräsentation eines Untersuchungsbereichs eines Untersuchungsobjekts auf Basis einer ersten und einer zweiten Repräsentation des Untersuchungsbereichs des Untersuchungsobjekt mit Hilfe eines ersten Modells.
Fig. 3 zeigt beispielhaft und schematisch ein Verfahren zum Trainieren eines zweiten Modells.
Fig. 4 zeigt beispielhaft und schematisch die Erzeugung einer korrigierten dritten Repräsentation auf Basis einer dritten Repräsentation mit Hilfe eines trainierten zweiten Modells.
Fig. 5 zeigt beispielhaft und schematisch ein Modell umfassend ein erstes Modell und ein zweites Modell.
Fig. 6 zeigt beispielhaft und schematisch ein Computersystem gemäß der vorliegenden Offenbarung.
Fig. 7 zeigt beispielhaft und schematisch eine weitere Ausführungsform des Computersystems gemäß der vorliegenden Offenbarung.
Fig. 8 zeigt beispielhaft und schematisch eine Ausführungsform des computer-implementierten Verfahrens der vorliegenden Offenbarung in Form eines Ablaufschemas.

### DETAILLIERTE BESCHREIBUNG

Die Gegenstände der vorliegenden Offenbarung werden nachstehend näher erläutert, ohne zwischen den Gegenständen (Verfahren, Computersystem, Computerprogramm(produkt), Verwendung, Kontrastmittel zur Verwendung, Kit) zu unterscheiden. Die nachfolgenden Erläuterungen sollen vielmehr für alle Gegenstände in analoger Weise gelten, unabhängig davon, in welchem Kontext (Verfahren, Computersystem, Computerprogramm(produkt), Verwendung, Kontrastmittel zur Verwendung, Kit) sie erfolgen.

Wenn in der vorliegenden Beschreibung oder in den Patentansprüchen Schritte in einer Reihenfolge genannt sind, bedeutet dies nicht zwingend, dass diese Offenbarung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Die vorliegende Offenbarung beschreibt Mittel, mit denen auf Basis von mindestens zwei Repräsentationen, die einen Untersuchungsbereich eines Untersuchungsobjekts nach Zugabe/Anwendung/Verwendung unterschiedlicher Mengen an Kontrastmittel repräsentieren, eine oder mehrere künstliche radiologische Aufnahmen erzeugt werden, bei der/denen der Kontrast zwischen Bereichen mit Kontrastmittel und Bereichen ohne Kontrastmittel variiert werden kann.

Das "Untersuchungsobjekt" ist üblicherweise ein Lebewesen, vorzugsweise ein Säugetier, ganz besonders bevorzugt ein Mensch.

Der "Untersuchungsbereich" ist ein Teil des Untersuchungsobjekts, zum Beispiel ein Organ oder ein Teil eines Organs oder mehrere Organe oder ein anderer Teil des Untersuchungsobjekts.

Der Untersuchungsbereich kann beispielsweise eine Leber, eine Niere, ein Herz, eine Lunge, ein Gehirn, ein Magen, eine Blase, eine Prostatadrüse, ein Darm oder ein Teil der genannten Teile oder ein anderer Teil des Körpers eines Säugetiers (z.B. eines Menschen) sein.

In einer Ausführungsform umfasst der Untersuchungsbereich eine Leber oder einen Teil einer Leber oder der Untersuchungsbereich ist eine Leber oder ein Teil einer Leber eines Säugetiers, vorzugsweise eines Menschen.

In einer weiteren Ausführungsform umfasst der Untersuchungsbereich ein Gehirn oder einen Teil eines Gehirns oder der Untersuchungsbereich ist ein Gehirn oder ein Teil eines Gehirns eines Säugetiers, vorzugsweise eines Menschen.

In einer weiteren Ausführungsform umfasst der Untersuchungsbereich ein Herz oder ein Teil eines Herzes oder der Untersuchungsbereich ist ein Herz oder ein Teil eines Herzes eines Säugetiers, vorzugsweise eines Menschen.

In einer weiteren Ausführungsform umfasst der Untersuchungsbereich einen Thorax oder einen Teil eines Thorax oder der Untersuchungsbereich ist ein Thorax oder ein Teil eines Thorax eines Säugetiers, vorzugsweise eines Menschen.

In einer weiteren Ausführungsform umfasst der Untersuchungsbereich einen Magen oder einen Teil eines Magens oder der Untersuchungsbereich ist ein Magen oder ein Teil eines Magens eines Säugetiers, vorzugsweise eines Menschen.

In einer weiteren Ausführungsform umfasst der Untersuchungsbereich eine Bauchspeicheldrüse oder einen Teil einer Bauchspeicheldrüse oder der Untersuchungsbereich ist eine Bauchspeicheldrüse oder ein Teil einer Bauchspeicheldrüse eines Säugetiers, vorzugsweise eines Menschen.

In einer weiteren Ausführungsform umfasst der Untersuchungsbereich eine Niere oder einen Teil einer Niere oder der Untersuchungsbereich ist eine Niere oder ein Teil einer Niere eines Säugetiers, vorzugsweise eines Menschen.

In einer weiteren Ausführungsform umfasst der Untersuchungsbereich einen oder beide Lungenflügel oder einen Teil eines Lungenflügels Säugetiers, vorzugsweise eines Menschen.

In einer weiteren Ausführungsform umfasst der Untersuchungsbereich eine Brust oder einen Teil einer Brust oder der Untersuchungsbereich ist eine Brust oder ein Teil einer Brust eines weiblichen Säugetiers, vorzugsweise eines weiblichen Menschen.

In einer weiteren Ausführungsform umfasst der Untersuchungsbereich eine Prostata oder einen Teil einer Prostata oder der Untersuchungsbereich ist eine Prostata oder ein Teil einer Prostata eines männlichen Säugetiers, vorzugsweise eines männlichen Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme festgelegt. Selbstverständlich kann der Untersuchungsbereich alternativ oder zusätzlich auch automatisch, beispielsweise auf Grundlage eines ausgewählten Protokolls, festgelegt werden.

Der Untersuchungsbereich wird einer radiologischen Untersuchung unterzogen.

Die "Radiologie" ist das Teilgebiet der Medizin, das sich mit der Anwendung elektromagnetischer Strahlen und (unter Einbezug etwa der Ultraschalldiagnostik) mechanischer Wellen zu diagnostischen, therapeutischen und/oder wissenschaftlichen Zwecken befasst. Neben Röntgenstrahlen kommen auch andere ionisierende Strahlung wie Gammastrahlung oder Elektronen zum Einsatz. Da ein wesentlicher Einsatzzweck die Bildgebung ist, werden auch andere bildgebende Verfahren wie die Sonographie und die Magnetresonanztomographie (Kernspintomographie) zur Radiologie gerechnet, obwohl bei diesen Verfahren keine ionisierende Strahlung zum Einsatz kommt. Der Begriff "Radiologie" im Sinne der vorliegenden Offenbarung umfasst damit insbesondere die folgenden Untersuchungsmethoden: Computertomographie, Magnetresonanztomographie, Sonographie.

In einer Ausführungsform der vorliegenden Offenbarung handelt es sich bei der radiologischen Untersuchung um eine magnetresonanztomographische Untersuchung.

In einer weiteren Ausführungsform handelt es sich bei der radiologischen Untersuchung um eine computertomographische Untersuchung

In einer weiteren Ausführungsform handelt es sich bei der radiologischen Untersuchung um eine Ultraschalluntersuchung.

Bei radiologischen Untersuchungen werden häufig Kontrastmittel zur Kontrastverstärkung eingesetzt.

"Kontrastmittel" sind Substanzen oder Gemische von Substanzen, die die Darstellung von Strukturen und Funktionen des Körpers bei radiologischen Untersuchungen verbessern.

In der Computertomographie werden meist iodhaltige Lösungen als Kontrastmittel eingesetzt. In der Magnetresonanztomographie (MRT) werden üblicherweise superparamagnetische Substanzen (z.B. Eisenoxidnanopartikel, superparamagnetische Eisen-Platin-Partikel (SIPPs)) oder paramagnetische Substanzen (z.B. Gadolinium-Chelate, Mangan-Chelate, Hafnium-Chelate) als Kontrastmittel verwendet. Im Fall der Sonographie werden üblicherweise Flüssigkeiten, die gasgefüllte Mikrobläschen (*microbubbles*) enthalten, intravenös verabreicht. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review of Risk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

MRT-Kontrastmittel entfalten in einer MRT-Untersuchung ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren. Superparamagnetische führen zu einer überwiegenden T2-Verkürzung, während paramagnetische Kontrastmittel im Wesentlichen zu einer T1-Verkürzung führen. Die Wirkung dieser Kontrastmittel ist indirekt, da das Kontrastmittel selbst kein Signal abgibt, sondern nur die Signalintensität in seiner Umgebung beeinflusst. Ein Beispiel für ein superparamagnetisches Kontrastmittel sind Eisenoxidnanopartikel (SPIO, engl.: *superparamagnetic iron oxide*). Beispiele für paramagnetische Kontrastmittel sind Gadolinium-Chelate wie Gadopentetat-Dimeglumin (Handelsname: Magnevist^{®} u.a.), Gadotersäure (Dotarem^{®}, Dotagita^{®}, Cyclolux^{®}), Gadodiamid (Omniscan^{®}), Gadoteridol (ProHance^{®}), Gadobutrol (Gadovist^{®}), Gadopiclenol (Elucirem, Vueway) und Gadoxetsäure (Primovist^{®}/Eovist^{®}).

In einer Ausführungsform handelt es sich bei der radiologischen Untersuchung um eine MRT-Untersuchung, bei der ein MRT-Kontrastmittel eingesetzt wird.

In einer weiteren Ausführungsform handelt es sich bei der radiologischen Untersuchung um eine CT-Untersuchung, bei der ein CT-Kontrastmittel eingesetzt wird.

In einer weiteren Ausführungsform handelt es sich bei der radiologischen Untersuchung um eine CT-Untersuchung, bei der ein MRT-Kontrastmittel eingesetzt wird.

Die Erzeugung einer künstlichen radiologischen Aufnahme mit einer variablen Kontrastverstärkung basiert auf mindestens zwei Repräsentationen des Untersuchungsbereichs, einer ersten Repräsentation und einer zweiten Repräsentation.

Die erste Repräsentation repräsentiert den Untersuchungsbereich ohne Kontrastmittel oder nach der Applikation einer ersten Menge eines Kontrastmittels. Vorzugsweise repräsentiert die erste Repräsentation den Untersuchungsbereich ohne Kontrastmittel (native Repräsentation).

Die zweite Repräsentation repräsentiert den Untersuchungsbereich nach der Applikation einer zweiten Menge eines Kontrastmittels. Die zweite Menge ist größer als die erste Menge (wobei die erste Menge wie beschrieben auch Null sein kann). Der Ausdruck "nach Applikation einer zweiten Menge eines Kontrastmittels" soll nicht so verstanden werden, dass sich die erste Menge und die zweite Menge in dem Untersuchungsbereich addieren. Der Ausdruck "die Repräsentation repräsentiert den Untersuchungsbereich nach der Applikation einer (ersten oder zweiten) Menge" soll also eher bedeuten: "die Repräsentation repräsentiert den Untersuchungsbereich mit einer (ersten oder zweiten) Menge" oder "die Repräsentation repräsentiert den Untersuchungsbereich umfassend eine (erste oder zweite) Menge".

In einer Ausführungsform ist sowohl die erste Menge als auch die zweite Menge des Kontrastmittels kleiner als die Standardmenge.

In einer weiteren Ausführungsform entspricht die zweite Menge des Kontrastmittels der Standardmenge.

In einer weiteren Ausführungsform ist die erste Menge des Kontrastmittels gleich Null und die zweite Menge des Kontrastmittels ist kleiner als die Standardmenge.

In einer weiteren Ausführungsform ist die erste Menge des Kontrastmittels gleich Null und die zweite Menge des Kontrastmittels entspricht der Standardmenge.

Die Standardmenge ist üblicherweise die vom Hersteller und/oder Vertreiber des Kontrastmittels empfohlene und/oder die von einer Zulassungsbehörde zugelassene und/oder die in einem Beipackzettel zum Kontrastmittel aufgeführte Menge.

So beträgt die Standardmenge von Primovist^{®} beispielsweise 0,025 mmol Gd-EOB-DTPA Dinatrium / kg Körpergewicht.

In einer Ausführungsform der vorliegenden Offenbarung handelt es sich bei dem Kontrastmittel um ein Mittel, das Gadolinium(III) 2-[4,7,10-tris(carboxymethyl)-1,4,7,10-tetrazacyclododec-1-yl]essigsäure (auch als Gadolinium-DOTA oder Gadotersäure bezeichnet) umfasst.

In einer weiteren Ausführungsform handelt es sich bei dem Kontrastmittel um ein Mittel, das Gadolinium(III) Ethoxybenzyl-diethylenetriaminepentaessigsäure (Gd-EOB-DTPA) umfasst; vorzugsweise umfasst das Kontrastmittel das Dinatriumsalz der Gadolinium(III)-ethoxybenzyl-diethylenetriaminpentaessigsäure (auch als Gadoxetsäure bezeichnet).

In einer Ausführungsform der vorliegenden Offenbarung handelt es sich bei dem Kontrastmittel um ein Mittel, das Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat (auch als Gadopiclenol bezeichnet), umfasst (siehe z.B. WO2007/042504 sowie WO2020/030618 und/oder WO2022/013454).

In einer Ausführungsform der vorliegenden Offenbarung handelt es sich bei dem Kontrastmittel um ein Mittel, das Dihydrogen[(±)-4-carboxy-5,8,11-tris(carboxymethyl)-1-phenyl-2-oxa-5,8,11-triazatridecan-13-oato(5-)]gadolinat(2-) (auch als Gadobensäure bezeichnet) umfasst.

In einer Ausführungsform der vorliegenden Offenbarung handelt es sich bei dem Kontrastmittel um ein Mittel, das Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yl]acetat (auch als Gadoquatrane bezeichnet) umfasst (siehe z.B. J. Lohrke et al.: Preclinical Profile of Gadoquatrane: A Novel Tetramerie, Macrocyclic High Relaxivity Gadolinium-Based Contrast Agent. Invest Radiol., 2022, 1, 57(10): 629-638; WO2016193190).

In einer Ausführungsform der vorliegenden Offenbarung handelt es sich bei dem Kontrastmittel um ein Mittel, das einen Gd³⁺-Komplex einer Verbindung der Formel (I) 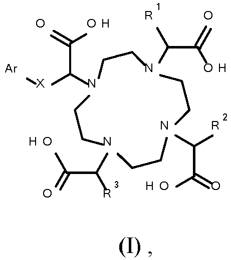 umfasst, wobei
Ar eine Gruppe ausgewählt aus 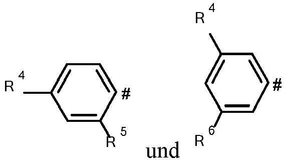 darstellt,
wobei # die Anknüpfung zu X darstellt,
X eine Gruppe darstellt, die aus
CH₂, (CH₂)₂, (CH₂)₃, (CH₂)₄ und *-(CH₂)₂-O-CH₂-^{#} ausgewählt wird,
wobei * die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
   und
R⁶ ein Wasserstoffatom darstellt,
oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen Gd³⁺-Komplex einer Verbindung der Formel (II) 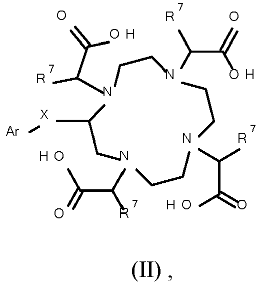 umfasst, wobei
Ar eine Gruppe ausgewählt aus 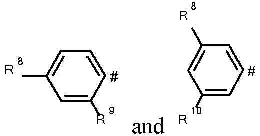 darstellt,
wobei # die Anknüpfung zu X darstellt,
X eine Gruppe darstellt, die aus CH₂, (CH₂)₂, (CH₂)₃, (CH₂)₄ and *-(CH₂)₂-O-CH₂-^{#} ausgewählt wird, wobei * die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon.

Der Begriff "C₁-C₃-Alkyl" bedeutet eine lineare oder verzweigte, gesättigte, einwertige Kohlenwasserstoffgruppe mit 1, 2 oder 3 Kohlenstoffatomen, z.B. Methyl, Ethyl, n-Propyl und Isopropyl. Der Begriff "C₂-C₄-Alkyl" bedeutet eine lineare oder verzweigte, gesättigte, einwertige Kohlenwasserstoffgruppe mit 2, 3 oder 4 Kohlenstoffatomen.

Der Begriff "C₂-C₄-Alkoxy" bedeutet eine lineare oder verzweigte, gesättigte, einwertige Gruppe der Formel (C₂-C₄-Alkyl)-O-, in der der Begriff "C₂-C₄-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl)triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

In einer Ausführungsform der vorliegenden Offenbarung handelt es sich bei dem Kontrastmittel um ein Mittel, das Gadolinium-2,2',2"-{(2S)-10-(carboxymethyl)-2-[4-(2-ethoxyethoxy)benzyl]-1,4,7,10-tetraazacyclododecane-1,4,7-triyl}triacetat umfasst.

In einer Ausführungsform der vorliegenden Offenbarung handelt es sich bei dem Kontrastmittel um ein Mittel, das Gadolinium-2,2',2"-[10-(carboxymethyl)-2-(4-ethoxybenzyl)-1,4,7,10-tetraazacyclododecane-1,4,7-triyl]triacetat umfasst.

In einer Ausführungsform der vorliegenden Offenbarung handelt es sich bei dem Kontrastmittel um ein Mittel, das Gadolinium(III) 5,8-bis(carboxylatomethyl)-2-[2-(methylamino)-2-oxoethyl]-10-oxo-2,5,8,11-tetraazadodecan-1-carboxylat-Hydrat (auch als Gadodiamid bezeichnet) umfasst.

In einer Ausführungsform der vorliegenden Offenbarung handelt es sich bei dem Kontrastmittel um ein Mittel, das Gadolinium(III) 2-[4-(2-hydroxypropyl)-7,10-bis(2-oxido-2-oxoethyl)-1,4,7,10-tetrazacyclododec-1-yl]acetat (auch als Gadoteridol bezeichnet) umfasst.

In einer Ausführungsform der vorliegenden Offenbarung handelt es sich bei dem Kontrastmittel um ein Mittel, das Gadolinium(III) 2,2',2"-(10-((2R,3S)-1,3,4-trihydroxybutan-2-yl)-1,4,7,10-tetraazacyclododecan-1,4,7-triyl)triacetat (auch als Gadobutrol oder Gd-DO3A-butrol bezeichnet) umfasst.

In einem ersten Schritt werden die erste Repräsentation und die zweite Repräsentation bereitgestellt, d.h. z.B. empfangen oder erzeugt.

Der Begriff "Empfangen" umfasst sowohl das Abrufen von Repräsentationen als auch das Entgegennehmen von Repräsentationen, die z.B. an das Computersystem der vorliegenden Offenbarung übermittelt werden. Die Repräsentationen können von einem Computertomographen, von einem Magnetresonanztomographen oder von einem Ultraschall-Scanner empfangen werden. Die radiologischen Aufnahmen können aus einem oder mehreren Datenspeichern ausgelesen und/oder von einem separaten Computersystem übermittelt werden.

Der Begriff "Erzeugen" bedeutet vorzugsweise, dass eine Repräsentation auf Basis einer anderen (z.B. einer empfangenen) Repräsentation oder auf Basis von mehreren anderen (z.B. empfangenen) Repräsentationen erzeugt wird. So kann beispielsweise eine empfangene Repräsentation eine Repräsentation eines Untersuchungsbereichs eines Untersuchungsobjekts im Ortsraum sein. Auf Basis dieser Ortsraum-Repräsentation kann beispielsweise eine Repräsentation des Untersuchungsbereichs des Untersuchungsobjekts im Frequenzraum durch eine Transformation (z.B. eine Fourier-Transformation) erzeugt werden. Weitere Möglichkeiten eine Repräsentation auf Basis einer oder mehrerer anderer Repräsentationen zu erzeugen, sind in dieser Beschreibung beschrieben.

Eine Repräsentation des Untersuchungsbereichs im Sinne der vorliegenden Offenbarung kann eine Repräsentation im Ortsraum (Bildraum), eine Repräsentation im Frequenzraum, eine Repräsentation im Projektionsraum oder eine Repräsentation in einem anderen Raum sein.

In einer Repräsentation im Ortsraum, in dieser Beschreibung auch als Ortsraumdarstellung oder Ortsraum-Repräsentation bezeichnet, wird der Untersuchungsbereich üblicherweise durch eine Vielzahl an Bildelementen (z.B. Pixel oder Voxel oder Doxel) repräsentiert, die beispielsweise rasterförmig angeordnet sein können, wobei jedes Bildelement einen Teil des Untersuchungsbereichs repräsentiert, wobei jedem Bildelement ein Farbwert oder Grauwert zugeordnet sein kann. Der Farbwert oder Grauwert repräsentiert eine Signalintensität, z.B. die Abschwächung von Röntgenstrahlen. Ein in der Radiologie weit verbreitetes Format zur Speicherung und Verarbeitung von Repräsentationen im Ortsraum ist das DICOM-Format. DICOM (Digital Imaging and Communications in Medicine) ist ein offener Standard zur Speicherung und zum Austausch von Informationen im medizinischen Bilddatenmanagement.

In einer Repräsentation im Frequenzraum, in dieser Beschreibung auch als Frequenzraumdarstellung oder Frequenzraum-Repräsentation bezeichnet, wird der Untersuchungsbereich durch eine Überlagerung von Grundschwingungen repräsentiert. Beispielsweise kann der Untersuchungsbereich durch eine Summe von Sinus- und Kosinus-Funktionen mit verschiedenen Amplituden, Frequenzen und Phasen repräsentiert werden. Die Amplituden und Phasen können als Funktion der Frequenzen beispielsweise in einer zwei- oder dreidimensionalen Darstellung aufgetragen werden. Üblicherweise wird die niedrigste Frequenz (Ursprung) in das Zentrum gelegt. Je weiter man sich von diesem Zentrum entfernt, desto höher sind die Frequenzen. Jeder Frequenz kann eine Amplitude, mit der die Frequenz in der Frequenzraumdarstellung vertreten ist, und eine Phase, die angibt, inwieweit die jeweilige Schwingung gegenüber einer Sinus- oder Kosinus-Schwingung verschoben ist, zugeordnet werden.

Eine Repräsentation im Ortsraum lässt sich beispielsweise durch eine Fourier-Transformation in eine Repräsentation im Frequenzraum überführen (transformieren). Umgekehrt lässt sich eine Repräsentation im Frequenzraum beispielsweise durch eine inverse Fourier-Transformation in eine Repräsentation im Ortsraum überführen (transformieren).

Details über Ortsraumdarstellungen und Frequenzraumdarstellungen und ihre jeweilige Umwandlung ineinander sind in zahlreichen Publikationen beschrieben, siehe z.B.: https://see.stanford.edu/materials/lsoftaee261/book-fall-07.pdf.

Eine Repräsentation eines Untersuchungsbereichs im Projektionsraum ist üblicherweise das Ergebnis einer computertomographischen Untersuchung vor einer Bildrekonstruktion. Eine Projektionsraumdarstellung kann als Rohdaten bei der computertomographischen Untersuchung aufgefasst werden. Im Rahmen einer Computertomographie wird die Intensität bzw. die Schwächung der Röntgenstrahlung beim Durchgang durch das Untersuchungsobjekt gemessen. Daraus lassen sich Projektionswerte errechnen. In einem zweiten Schritt wird die durch die Projektion kodierte Objektinformation durch eine computergestützte Rekonstruktion in ein Bild (Ortsraumdarstellung) transformiert. Die Rekonstruktion kann mit der Radon-Transformation erfolgen. Die Radon-Transformation beschreibt die Verknüpfung zwischen dem unbekannten Untersuchungsobjekt und seinen zugehörigen Projektionen.

Details über die Transformation von Projektionsdaten in eine Ortsraumdarstellung sind in zahlreichen Publikationen beschrieben, siehe z.B. K. Fang: The Radon Transformation and Its Application in Tomography, Journal of Physics Conference Series 1903(1):012066.

Eine Repräsentation des Untersuchungsbereichs kann auch eine Repräsentation im Hough-Raum sein. Zur Erkennung von geometrischen Objekten in einem Bild wird nach einer Kantendetektion durch die so genannte Hough-Transformation ein Dualraum erschaffen, in den für jeden Punkt im Bild, der auf einer Kante liegt, alle möglichen Parameter des geometrischen Objekts eingetragen werden. Jeder Punkt im Dualraum entspricht damit einem geometrischen Objekt im Bildraum. Bei einer Geraden kann das z.B. die Steigung und der y-Achsen-Abschnitt der Geraden sein, bei einem Kreis der Mittelpunkt und Radius des Kreises. Details über die Hough-Transformation sind in der Literatur zu finden (siehe z.B.: A.S. Hassanein et al.: A Survey on Hough Transform, Theory, Techniques and Applications, arXiv:1502.02160v1).

Es gibt weitere Räume, in denen Repräsentationen des Untersuchungsbereichs vorliegen können. Der Einfachheit halber und der besseren Verständlichkeit halber wird die Erfindung in weiten Teilen der Beschreibung auf Basis von Ortsraum-Repräsentationen beschrieben. Dies soll jedoch nicht als Einschränkung verstanden werden. Der Fachmann der Bildanalyse weiß, wie er die entsprechenden Teile der Beschreibung auf andere Repräsentationen als Ortsraum-Repräsentationen anwenden kann.

Die erste Repräsentation und die zweite Repräsentation werden einem ersten Modell zugeführt.

Es ist möglich, eine Co-Registrierung der ersten Repräsentation und der zweiten Repräsentation durchzuführen, bevor sie dem ersten Modell zugeführt werden. Die "Co-Registrierung" (im Stand der Technik auch "Bildregistrierung" genannt) dient dazu, zwei oder mehrere Ortsraumdarstellungen desselben Untersuchungsbereichs bestmöglich in Übereinstimmung miteinander zu bringen. Dabei wird eine der Ortsraumdarstellungen als Referenzbild festgelegt, die andere wird Objektbild genannt. Um diese optimal an das Referenzbild anzupassen, wird eine ausgleichende Transformation berechnet.

Es ist auch möglich, eine Co-Registrierung von Repräsentationen im Frequenzraum durchzuführen, wobei hierbei zu beachten ist, dass sich eine Translation im Ortsraum als eine additive lineare Phasenrampe im Frequenzraum darstellt. Skalierung und Rotation hingegen bleiben bei der Fourier- und inversen Fourier-Transformation erhalten - eine Skalierung und Rotation im Frequenzraum ist auch eine Skalierung und Rotation im Ortsraum (siehe z.B.: S. Skare: Rigid Body Image Realignment in Image Space vs. k-Space, ISMRM SCIENTIFIC WORKSHOP on Motion Correction, 2014, https://cds.ismrm.org/protected/Motion_14/Program/Syllabus/Skare.pdf).

Das erste Modell ist konfiguriert, auf Basis der ersten Repräsentation und der zweiten Repräsentation eine dritte Repräsentation zu erzeugen. Die dritte Repräsentation repräsentiert den Untersuchungsbereich des Untersuchungsobjekts nach Applikation einer dritten Menge des Kontrastmittels.

Die dritte Menge des Kontrastmittels unterscheidet sich von der ersten Menge und von der zweiten Menge. Die dritte Menge des Kontrastmittels ist vorzugsweise größer als die zweite Menge; die dritte Menge kann aber auch kleiner als die zweite Menge sein.

Ist die zweite Menge kleiner als die Standardmenge, so kann die dritte Menge beispielsweise gleich der Standardmenge sein. Es ist aber auch möglich, dass die dritte Menge größer als die Standmenge ist.

Ist die dritte Menge kleiner als die zweite Menge, so führt das erste Modell zu einer Kontrastabschwächung. Das bedeutet, dass die durch das Kontrastmittel in der zweiten Repräsentation hervorgerufene Kontrastverstärkung in der dritten Repräsentation abgeschwächt (weniger stark ausgeprägt) ist.

Ist die dritte Menge größer als die zweite Menge, so führt das erste Modell zu einer Kontrastverstärkung. Das bedeutet, dass die durch das Kontrastmittel in der zweiten Repräsentation hervorgerufene Kontrastverstärkung in der dritten Repräsentation verstärkt (stärker ausgeprägt) ist.

Das erste Modell kann ein Modell des maschinellen Lernens sein.

Ein "Modell des maschinellen Lernens" kann als eine computerimplementierte Datenverarbeitungsarchitektur verstanden werden. Ein solches Modell kann Eingabedaten empfangen und Ausgabedaten auf der Grundlage dieser Eingabedaten und Modellparametern liefern. Ein solches Modell kann durch Training eine Beziehung zwischen den Eingabedaten und den Ausgabedaten erlernen. Beim Training können Modellparameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modellparameter werden so verändert, dass die Abweichungen zwischen den Ausgabedaten und den Zieldaten auf ein (definiertes) Minimum reduziert werden. Zur Modifizierung der Modellparameter im Hinblick auf eine Reduzierung der Abweichungen kann ein Optimierungsverfahren wie beispielsweise ein Gradientenverfahren verwendet werden.

Die Abweichungen können mit Hilfe einer Fehlerfunktion (engl.: *loss function*) quantifiziert werden. Eine solche Fehlerfunktion kann verwendet werden, um einen Fehler (engl.: *loss*) für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modellparameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder eine andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Das erste Modell kann ein Modell des maschinellen Lernens sein, wie es beispielsweise in einer der folgenden Publikationen beschrieben ist: WO2019/074938A1, WO2022/253687A1, WO2022/207443A1, WO2022/223383A1, WO20227184297A1, WO2022/179896A2, WO2021/069338A1, EP 22209510.1, EP23159288.2, PCT/EP2023/053324, PCT/EP2023/050207, CN110852993A, CN110853738A, US2021150671A1, arXiv:2303.15938v1, doi:10.1093/jrr/rrz030.

Das erste Modell kann ein mechanistisches (deterministisches) Modell sein. Mechanistische Modelle beruhen auf grundlegenden Prinzipien und bekannten Beziehungen innerhalb eines Systems. Sie werden oft aus wissenschaftlichen Theorien und bereichsspezifischem Wissen abgeleitet. Mechanistische Modelle beschreiben die zugrundeliegenden Mechanismen eines Systems mit Hilfe mathematischer Gleichungen oder physikalischer Gesetze. Sie zielen darauf ab, das Verhalten des Systems auf der Grundlage eines Verständnisses seiner Komponenten und Wechselwirkungen zu simulieren.

Modelle des maschinellen Lernens sind hingegen datengesteuert und lernen Muster und Beziehungen aus Eingabedaten, ohne dass die Beziehungen explizit programmiert werden.

Während also mechanistische Modelle auf grundlegenden Prinzipien beruhen und darauf abzielen, die zugrundeliegenden Systemmechanismen darzustellen, lernen Modelle des maschinellen Lernens Muster und Beziehungen direkt aus Daten, ohne diese Beziehungen explizit zu programmieren.

Das mechanistische Modell beruht also auf physikalischen Gesetzen. In radiologischen Untersuchungen ist ein Signal, das von einem Kontrastmittel hervorgerufen wird, üblicherweise abhängig von der Menge (z.B. der Konzentration) des Kontrastmittels in dem Untersuchungsbereich. Die Signalstärke kann beispielsweise über einen definierten Konzentrationsbereich linear oder in Form einer anderen Abhängigkeit von der Konzentration des Kontrastmittels in dem Untersuchungsbereich abhängen. Die funktionelle Abhängigkeit der Signalstärke von der Konzentration kann man sich zu Nutze machen, um ein mechanistisches Modell zu erstellen.

In einer Ausführungsform ist das erste Modell ein mechanistisches Modell, das in einem ersten Schritt auf Basis der ersten Repräsentation und der zweiten Repräsentation die durch Kontrastmittel in dem Untersuchungsbereich hervorgerufene Signalintensitätsverteilung ermittelt und diese in einem zweiten Schritt α-fach auf die erste oder zweite Repräsentation addiert, wobei α ein Verstärkungsfaktor ist.

Die Erzeugung der durch Kontrastmittel in dem Untersuchungsbereich hervorgerufene Signalintensitätsverteilung kann beispielsweise ein Subtrahieren der ersten Repräsentation von der zweiten Repräsentation umfassen. Repräsentiert die erste Repräsentation den Untersuchungsbereich des Untersuchungsobjekts ohne Kontrastmittel und repräsentiert die zweite Repräsentation den Untersuchungsbereich des Untersuchungsobjekts mit Kontrastmittel, so wird durch das Subtrahieren der ersten Repräsentation von der zweiten Repräsentation eine Repräsentation des Untersuchungsbereichs erzeugt, bei der die Signalintensitätsverteilung allein durch das Kontrastmittel hervorgerufen wird, da die Signale, die nicht durch Kontrastmittel hervorgerufen werden, in der ersten Repräsentation und der zweiten Repräsentation gleich sind und durch das Subtrahieren eliminiert werden.

Wird diese Signalintensitätsverteilung einfach (α = 1) auf die erste Repräsentation addiert, erhält man wieder die zweite Repräsentation.

Wird diese Signalintensitätsverteilung mehrfach (α > 1) auf die erste Repräsentation addiert, erhält man eine dritte Repräsentation des Untersuchungsbereichs, bei der der Kontrast zwischen Bereichen mit Kontrastmittel und Bereichen ohne Kontrastmittel gegenüber der zweiten Repräsentation verstärkt ist. Dabei sind nicht nur ganzzahlige Werte von α möglich, sondern auch andere reelle Werte (z.B. 1,5 oder 3,1416 oder andere Werte).

Wird ein Bruchteil (0 > α > 1) dieser Signalintensitätsverteilung auf die erste Repräsentation addiert, erhält man eine dritte Repräsentation des Untersuchungsbereichs, bei der der Kontrast zwischen Bereichen mit Kontrastmittel und Bereichen ohne Kontrastmittel gegenüber der zweiten Repräsentation abgeschwächt ist.

Es sind auch negative Werte von α möglich, die z.B. so gewählt werden können, dass Bereiche des Untersuchungsbereichs, die eine Kontrastmittel-induzierte Signalverstärkung in der messtechnisch erzeugten Repräsentation erfahren, in den künstlich erzeugten Repräsentationen komplett dunkel (schwarz) sind.

Der Verstärkungsfaktor α ist also eine positive oder negative reelle Zahl. Der Verstärkungsfaktor α kann von einem Nutzer gewählt werden, d.h. variabel sein, oder vordefiniert sein, d.h. vorgegeben sein. Der Verstärkungsfaktor α kann auch automatisch ermittelt werden, z.B. auf Basis des Histogramms der ersten Repräsentation und/oder der zweiten Repräsentation und/oder der Differenz der ersten Repräsentation von der zweiten Repräsentation und/oder mit Hilfe eines initialen Modells (siehe unten).

Durch Variation des Verstärkungsfaktors α kann der Kontrast zwischen Bereichen mit Kontrastmittel und Bereichen ohne Kontrastmittel also variiert werden.

Es ist also möglich, mit Hilfe des ersten Modells auf Basis der ersten Repräsentation und der zweiten Repräsentation eine dritte Repräsentation des Untersuchungsbereichs des Untersuchungsobjekts zu erzeugen, die den Untersuchungsbereich nach Applikation einer dritten Menge repräsentiert, wobei die dritte Menge verschieden von der ersten Menge und der zweiten Menge sein kann.

Es ist möglich, mit Hilfe des ersten Modells auf Basis einer ersten Repräsentation des Untersuchungsbereichs ohne Kontrastmittel oder mit einer ersten Menge eines Kontrastmittels, und einer zweiten Repräsentation des Untersuchungsbereichs mit einer zweiten Menge des Kontrastmittels, die kleiner oder gleich der Standardmenge ist, eine Repräsentation des Untersuchungsbereichs mit einer Menge an Kontrastmittel zu erzeugen, die größer als die Standardmenge ist.

Das zuvor beschriebene mechanistische Modell basiert auf der Annahme, dass die durch Grauwerte oder Farbwerte repräsentierte Signalintensität in einer Repräsentation des Untersuchungsbereichs linear von der Menge des applizierten Kontrastmittels abhängt. Dies ist insbesondere bei vielen MRT-Untersuchungen der Fall. Die lineare Abhängigkeit erlaubt es, den Kontrast durch Variation des Verstärkungsfaktors α zu variieren. Ein Verstärkungsfaktor von α = 2 bedeutet also, dass die dritte Menge an Kontrastmittel der doppelten zweiten Menge entspricht.

Das zuvor beschriebene mechanistische Modell ist offenbart in: EP22207079.9, EP22207080.7, EP23168725.2.

Es sei angemerkt, dass statt einer linearen Abhängigkeit dem mechanistischen Modell auch eine andere Abhängigkeit zugrunde gelegt werden kann. Die Abhängigkeit kann empirisch ermittelt werden.

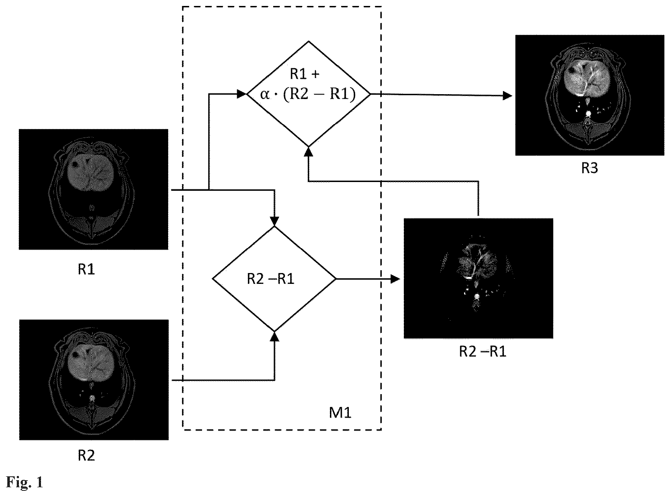

Fig. 1 zeigt beispielhaft und schematisch die Erzeugung einer dritten Repräsentation eines Untersuchungsbereichs eines Untersuchungsobjekts auf Basis einer ersten Repräsentation und einer zweiten Repräsentation mit Hilfe eines ersten Modells.

Das Untersuchungsobjekt ist ein Schwein und der Untersuchungsbereich umfasst die Leber des Schweins.

Die erste Repräsentation R1 ist eine magnetresonanztomografische Aufnahme, die den Untersuchungsbereich im Ortsraum ohne Kontrastmittel repräsentiert.

Die zweite Repräsentation R2 repräsentiert denselben Untersuchungsbereich desselben Untersuchungsobjekts wie die erste Repräsentation R1 im Ortsraum. Die zweite Repräsentation R2 ist ebenfalls eine magnetresonanztomografische Aufnahme.

Die zweite Repräsentation R2 repräsentiert den Untersuchungsbereich nach der Applikation einer zweiten Menge eines Kontrastmittels. Im vorliegenden Beispiel wurde dem Untersuchungsobjekt eine Menge von 25 µmol pro kg Körpergewicht eines hepatobiliären Kontrastmittels intravenös verabreicht. Die zweite Repräsentation R2 repräsentiert den Untersuchungsbereich in der so genannten arteriellen Phase (siehe z.B. DOI:10.1002/jmri.22200).

Ein hepatobiliäres Kontrastmittel zeichnet sich dadurch aus, dass es spezifisch von Leberzellen, den Hepatozyten, aufgenommen wird, sich im Funktionsgewebe (Parenchym) anreichert und den Kontrast in gesundem Lebergewebe verstärkt. Ein Beispiel eines hepatobiliären Kontrastmittels ist das Dinatriumsalz der Gadoxetsäure (Gd-EOB-DTPA Dinatrium), das in dem US-Patent No. 6,039,931A beschrieben ist, und unter den Markennamen Primovist^{®} und Eovist^{®} kommerziell erhältlich ist. Weitere hepatobiliäre Kontrastmittel sind u.a. in WO2022/194777 beschrieben.

Die erste Repräsentation R1 und die zweite Repräsentation R2 werden einem ersten Model M1 zugeführt. Auf Basis der ersten Repräsentation R1 und der zweiten Repräsentation R2 erzeugt das erste Modell M1 eine dritte Repräsentation R3. In dem in Fig. 1 gezeigten Beispiel umfasst die Erzeugung der dritten Repräsentation R3 ein Subtrahieren der ersten Repräsentation R1 von der zweiten Repräsentation R2 (R2-R1). Ferner umfasst das Erzeugen der dritten Repräsentation R3 ein α-faches Addieren der Differenz der ersten Repräsentation von der zweiten Repräsentation auf die erste Repräsentation (R3= R1+α·(R2-R1)).

Falls beim Subtrahieren der ersten Repräsentation R1 von der zweiten Repräsentation R2 negative Grauwerte/Farbwerte entstehen, können diese negativen Werte auf Null (oder einen anderen Wert) gesetzt werden, um solche negativen Werte zu vermeiden.

Die Differenz (R2-R1) repräsentiert die durch die zweite Menge des Kontrastmittels im Untersuchungsbereich hervorgerufene Kontrastverstärkung (Signalintensitätsverteilung).

Die Differenz (R2-R1) wird mit dem Verstärkungsfaktor α multipliziert und das Ergebnis der Multiplikation zu der ersten Repräsentation R1 addiert. Auf diese Weise wird die dritte Repräsentation R3 erzeugt. In dem in Fig. 1 gezeigten Beispiel beträgt der Verstärkungsfaktor α=3, d.h. die Differenz (R2-R1) wird dreimal zu der ersten Repräsentation R1 addiert.

Die dritte Repräsentation R3 kann einer Normalisierung unterzogen werden, das heißt, die Grauwerte/Farbwerte können mit einem Faktor multipliziert werden, so dass der Grauwert/Farbwert mit dem höchsten Wert beispielsweise durch den Grauton/Farbton "weiß" und der Grauwert/Farbwert mit dem geringsten Wert beispielsweise durch den Grauton/Farbton "schwarz" repräsentiert wird.

In dem in Fig. 1 gezeigten Beispiel besteht das erste Modell M1 also aus mathematischen Operationen, die Subtraktionen, Multiplikationen und Additionen auf Basis der Grauwerte/Farbwerte der einzelnen Bildelemente (z.B. Pixel, Voxel) ausführen.

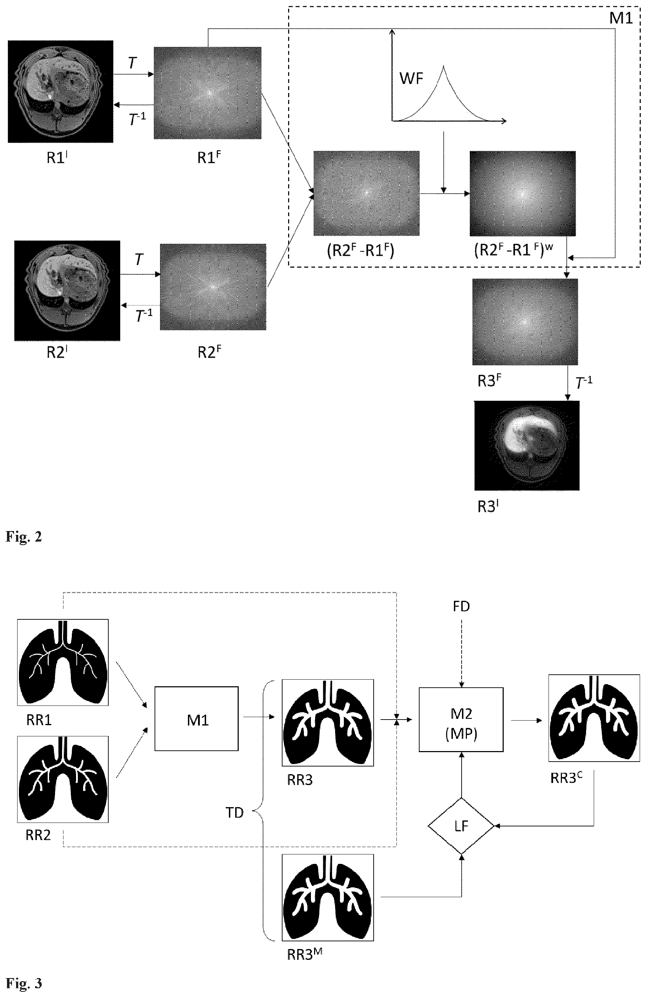

Fig. 2 zeigt schematisch ein weiteres Beispiel der Erzeugung einer dritten Repräsentation eines Untersuchungsbereichs eines Untersuchungsobjekts auf Basis einer ersten und einer zweiten Repräsentation des Untersuchungsbereichs des Untersuchungsobjekt mit Hilfe eines ersten Modells.

Fig. 2 zeigt einen Untersuchungsbereich eines Untersuchungsobjekts in Form von verschiedenen Repräsentationen.

Eine erste Repräsentation R1^{I} repräsentiert den Untersuchungsbereich im Ortsraum ohne Kontrastmittel oder nach der Applikation einer ersten Menge eines Kontrastmittels. Der in Fig. 2 gezeigte Untersuchungsbereich umfasst eine Leber eines Schweines. Die erste Repräsentation R1^{I} ist eine magnetresonanztomographische Aufnahme.

Die erste Ortsraum-Repräsentation R1^{I} lässt sich durch eine Transformation *T*, zum Beispiel eine Fourier-Transformation, in eine erste Repräsentation R1^{F} des Untersuchungsbereich im Frequenzraum umwandeln. Die erste Frequenzraum-Repräsentation R1^{F} repräsentiert denselben Untersuchungsbereich desselben Untersuchungsobjekts wie die erste Ortsraum-Repräsentation R1^{I}, ebenfalls ohne Kontrastmittel oder nach der Applikation der ersten Menge des Kontrastmittels.

Die erste Frequenzraum-Repräsentation R1^{F} lässt sich mittels einer Transformation *T*^{*-*1} in die erste Ortsraum-Repräsentation R1^{I} umwandeln, zum Beispiel durch eine inverse Fourier-Transformation. Die Transformation *T*^{*-*1} ist die zur Transformation *T* inverse Transformation.

Eine zweite Repräsentation R2^{I} repräsentiert denselben Untersuchungsbereich desselben Untersuchungsobjekts wie die erste Repräsentation R1^{I} im Ortsraum. Die zweite Ortsraum-Repräsentation R2^{I} repräsentiert den Untersuchungsbereich nach der Applikation einer zweiten Menge des Kontrastmittels. Die zweite Menge ist größer als die erste Menge (wobei die erste Menge wie beschrieben auch Null sein kann). Die zweite Repräsentation R2^{I} ist ebenfalls eine magnetresonanztomographische Aufnahme. Als Kontrastmittel wurde in dem in Fig. 2 dargestellten Beispiel das Dinatriumsalz der Gadoxetsäure (Gd-EOB-DTPA Dinatrium) als ein hepatobiliäres MRT-Kontrastmittel verwendet.

Die zweite Ortsraum-Repräsentation R2^{I} lässt sich durch die Transformation *T* in eine zweite Repräsentation R2^{F} des Untersuchungsbereichs im Frequenzraum umwandeln. Die zweite Frequenzraum-Repräsentation R2^{F} repräsentiert denselben Untersuchungsbereich desselben Untersuchungsobjekts wie die zweite Ortsraum-Repräsentation R2^{I}, ebenfalls nach der Applikation der zweiten Menge des Kontrastmittels.

Die zweite Frequenzraum-Repräsentation R2^{F} lässt sich mittels der Transformation *T*^{*-*1} in die zweite Ortsraum-Repräsentation R2^{I} umwandeln.

In dem in Fig. 2 gezeigten Beispiel werden die erste Frequenzraum-Repräsentation R1^{F} und die zweite Frequenzraum-Repräsentation R2^{F} einem ersten Modell M1 zugeführt. Das erste Modell M1 erzeugt auf Basis der ersten Frequenzraum-Repräsentation R1^{F} und der zweiten Frequenzraum-Repräsentation R2^{F} eine dritte Frequenzraum-Repräsentation R3^{F}. Die dritte Frequenzraum-Repräsentation R3^{F} kann durch eine Transformation *T*^{*-*1} (z.B. eine inverse Fourier-Transformation) in eine dritte Ortsraum-Repräsentation R3^{I} überführt werden.

Das in Fig. 2 gezeigte Modell M1 umfasst nicht die Transformation *T,* die die erste Ortsraum-Repräsentation R1^{I} in die erste Frequenzraum-Repräsentation R1^{F} überführt und die zweite Ortsraum-Repräsentation R2^{I} in die zweite Frequenzraum-Repräsentation R2^{F} überführt. Ebenso umfasst das in Fig. 2 gezeigte Modell M1 nicht die Transformation *T*⁻¹, die die dritte Frequenzraum-Repräsentation R3^{F} in die dritte Ortsraum-Repräsentation R3^{I} überführt. Es ist aber denkbar, dass die Transformation *T* und/oder die Transformation *T*^{*-*1} Bestandteil(e) des ersten Modells M1 sind, d.h. es ist denkbar, dass das erste Modell M1 die Transformation *T* und/oder die Transformation *T*^{*-*1} ausführt.

Mittels des ersten Modells M1 wird die erste Frequenzraum-Repräsentation R1^{F} von der zweiten Frequenzraum-Repräsentation R2^{F} subtrahiert (R2^{F} - R1^{F}). Das Ergebnis ist eine Repräsentation der durch Kontrastmittel im Untersuchungsbereich hervorgerufenen Signalintensitätsverteilung im Frequenzraum.

Die Differenz R2^{F} - R1^{F} wird mit einer Gewichtsfunktion WF multipliziert, die niedrige Frequenzen höher gewichtet als hohe Frequenzen. Im vorliegenden Fall werden die Amplituden der Grundschwingungen mit einem umso größeren Gewichtsfaktor multipliziert desto kleiner die Frequenz ist. Dieser Schritt ist ein optionaler Schritt, der ausgeführt werden kann, um das Signal-RauschVerhältnis in der dritten Repräsentation zu erhöhen, insbesondere dann, wenn der Verstärkungsfaktor α größere Werte (z.B. Werte größer als 3, 4 oder 5) annimmt. Das Ergebnis dieser frequenzabhängigen Gewichtung ist die gewichtete Repräsentation (R2^{F}-R1^{F})^{W}.

Kontrastinformationen werden in einer Frequenzraumdarstellung durch niedrige Frequenzen repräsentiert, während die höheren Frequenzen Informationen zu Feinstrukturen repräsentieren. Durch eine solche Gewichtung werden somit diejenigen Frequenzen, die einen höheren Beitrag zum Kontrast leisten, mit einem höheren Gewicht versehen werden als diejenigen Frequenzen, die einen geringeren Beitrag zum Kontrast leisten. Bildrauschen ist typischerweise in der Frequenzdarstellung gleichverteilt. Die frequenzabhängige Gewichtsfunktion hat die Wirkung eines Filters. Der Filter erhöht das Signalzu-Rauschen-Verhältnis, da die spektrale Rauschdichte für hohe Frequenzen verringert wird.

Bevorzugte Gewichtsfunktionen sind Hann-Funktion (auch als Hann Window bezeichnet) und Poisson-Funktion (Poisson Window).

Beispiele für weitere Gewichtsfunktionen sind zum Beispiel zu finden unter https://de.wikipedia.org/wiki/Fensterfunktion#Beispiele_von_Fensterfunktionen; F.J. Harris et al.: On the Use of Windows for Harmonic Analysis with the Discrete Fourier Transform, Proceedings oft he IEEE, VoL. 66, N. 1, 1978; https://docs.scipy.org/doc/scipy/reference/signal.windows.html; K. M. M Prabhu: Window Functions and Their Applications in Signal Processing, CRC Press, 2014, 978-1-4665-1583-3).

Die gewichtete Differenz (R2^{F}-R1^{F})^{W} wird in einem nächsten Schritt mit einem Verstärkungsfaktor α multipliziert und zu der ersten Frequenzraum-Repräsentation R1^{F} addiert. Das Ergebnis ist eine dritte Repräsentation R3^{F}=R1^{F}+α·(R2^{F}-R1^{F})^{W} des Untersuchungsbereichs des Untersuchungsobjekts im Frequenzraum. Die dritte Frequenzraum-Repräsentation R3^{F} wird in einem weiteren Schritt durch die Transformation *T*⁻¹ (z.B. eine inverse Fouriertransformation) in eine dritte Repräsentation R3^{I} des Untersuchungsbereichs des Untersuchungsobjekts im Ortsraum umgewandelt.

Die dritte Repräsentation R3^{I} repräsentiert den Untersuchungsbereich des Untersuchungsobjekts nach Applikation einer dritten Menge des Kontrastmittels. Die dritte Menge ist abhängig von dem Verstärkungsfaktor α. Ist der Verstärkungsfaktor beispielsweise 3 und ist die durch die Grauwerte/Farbwerte repräsentierte Signalintensitätsverteilung linear von der Menge des Kontrastmittels abhängig, dann entspricht die dritte Menge dem Dreifachen der Differenz der ersten Menge von der zweiten Menge.

Wird ein erstes Modell M1, wie es in Fig. 1 dargestellt ist, verwendet, um die dritte Repräsentation zu erzeugen, dann wird durch einen Verstärkungsfaktor, der größer als 1 ist (α > 1) nicht nur der Kontrast verstärkt, sondern es wird in demselben Umfang auch Rauschen verstärkt. Ein erstes Modell M1, wie es in Fig. 2 dargestellt ist, kann ein solches Rauschen durch die Gewichtung mit der Gewichtsfunktion im Frequenzraum zu einem gewissen Teil reduzieren.

Um Rauschen und/oder andere unerwünschte Artefakte in der dritten Repräsentation (weiter) zu reduzieren oder zu eliminieren, ist dem ersten Modell ein zweites Modell nachgeschaltet. Die dritte Repräsentation, die durch das erste Modell erzeugt wird, ist also nicht das Ergebnis der Erzeugung der synthetischen kontrastverstärkten Repräsentation der vorliegenden Offenbarung. Das zweite Modell dient der Korrektur der vom ersten Modell erzeugten dritten Repräsentation. Dabei kann der Begriff "Korrektur" das Reduzieren oder Eliminieren von Rauschen und/oder Artefakten bedeuten.

Gemäß der vorliegenden Offenbarung werden also zwei Modelle zum Erzeugen einer synthetischen kontrastverstärken radiologischen Aufnahme verwendet: ein erstes Modell und ein zweites Modell. Das erste Modell dient der Kontrastverstärkung (α > 1) oder der Kontrastminderung (α < 1). Das zweite Modell dient der Korrektur (z.B. Rauschunterdrückung, Unterdrückung von Artefakten). Das erste Modell kann auch als Synthese-Modell und das zweite Modell als Korrektur-Modell bezeichnet werden.

Mit anderen Worten: gemäß der vorliegenden Offenbarung werden zwei Modelle zum Erzeugen einer synthetischen kontrastverstärken radiologischen Aufnahme verwendet: ein erstes Modell und ein zweites Modell. Das erste Modell erzeugt einen "Vorschlag" einer synthetischen kontrastverstärken radiologischen Aufnahme; das zweite Modell optimiert diesen "Vorschlag". Der "Vorschlag" kann eine erste Annäherung an die synthetische kontrastverstärke radiologische Aufnahme sein. Das zweite Modell kann diese Annäherung so modifizieren (optimieren), dass das Ergebnis einer realen kontrastverstärken radiologischen Aufnahme entspricht.

Dem zweiten Modell wird die von dem ersten Modell erzeugte dritte Repräsentation als Eingabedaten zugeführt und das zweite Modell erzeugt auf Basis dieser Eingabedaten und auf Basis von Modellparametern eine korrigierte dritte Repräsentation. Neben der von dem ersten Modell erzeugten dritten Repräsentationen können dem zweiten Modell noch weitere Daten als Eingabedaten zugeführt werden (siehe unten).

Das zweite Modell ist ein Modell des maschinellen Lernens. Das zweite Modell ist anhand von Trainingsdaten trainiert worden, auf Basis einer von dem ersten Modell erzeugten dritten Repräsentation und Modellparametern eine korrigierte (z.B. modifizierte und/oder optimierte) dritte Repräsentation zu erzeugen.

Die Trainingsdaten umfassen für jedes Referenzobjekt einer Vielzahl an Referenzobjekten (i) eine von dem ersten Modell erzeugte Referenz-Repräsentation, die einen Referenzbereich des Referenzobjekts nach Applikation einer Referenzmenge des Kontrastmittels repräsentiert, und (ii) eine gemessene Referenz-Repräsentation des Referenzbereichs des Referenzobjekts nach Applikation der Referenzmenge des Kontrastmittels.

Der Begriff "Vielzahl" bedeutet mehr als zehn, vorzugsweise mehr als einhundert.

Der Begriff "Referenz" wird in dieser Beschreibung verwendet, um die Phase des Trainierens des zweiten Modells von der Phase des Verwendens des trainierten zweiten Modells zur Korrektur einer Repräsentation zu unterscheiden. Der Begriff "Referenz" hat ansonsten keinerlei einschränkende Bedeutung. Der Begriff "(Referenz-)Repräsentation" bedeutet, dass die entsprechende Aussage sowohl für eine Repräsentation eines Untersuchungsobjekts als auch für eine Referenz-Repräsentation eines Referenzobjekts gilt. Ein "Referenzobjekt" ist ein Objekt, von dem Daten (z.B. Referenz-Repräsentationen) zum Trainieren des zweiten Modells verwendet werden. Daten eines Untersuchungsobjekts werden hingegen verwendet, um das trainierte zweite Modell (in Kombination mit dem ersten Modell) zur Vorhersage zu nutzen. Jedes Referenzobjekt ist, wie das Untersuchungsobjekt, üblicherweise ein Lebewesen, vorzugsweise ein Säugetier, ganz besonders bevorzugt ein Mensch. Der "Referenzbereich" ist ein Teil des Referenzobjekts. Der Referenzbereich entspricht üblicherweise (aber nicht notwendigerweise) dem Untersuchungsbereich des Untersuchungsobjekts. Mit anderen Worten: für den Fall, dass der Untersuchungsbereich ein Organ oder ein Teil eines Organs (z.B. die Leber oder ein Teil der Leber) des Untersuchungsobjekts ist, ist der Referenzbereich eines jeden Referenzobjekts vorzugsweise das entsprechende Organ oder der entsprechende Teil des Organs des jeweiligen Referenzobjekts. Die "Referenzmenge" ist eine Menge an Kontrastmittel, die durch das erste Modell zumindest anteilig bestimmt (festgelegt) wird (zum Beispiel durch den Verstärkungsfaktor α). Die "Referenzmenge" kann der dritten Menge an Kontrastmittel entsprechen; die "Referenzmenge" kann aber auch verschieden von der dritten Menge sein.

Die Trainingsdaten, mit denen das zweite Modell trainiert wurde, umfassen also (i) Eingabedaten und (ii) Zieldaten. Das zweite Modell ist konfiguriert, auf Basis der Eingabedaten und auf Basis von Modellparametern Ausgabedaten zu erzeugen. Die Ausgabedaten werden mit den Zieldaten verglichen. Abweichungen zwischen den Ausgabedaten und den Zieldaten können in einem Optimierungsverfahren (z.B. einem Gradientenverfahren) durch Modifizieren von Modellparametern reduziert werden.

Die Eingabedaten umfassen für jedes Referenzobjekt der Vielzahl an Referenzobjekten eine von dem ersten Modell erzeugte Referenz-Repräsentation, die den Referenzbereich des Referenzobjekts nach Applikation der Referenzmenge des Kontrastmittels repräsentiert. Die Eingabedaten werden also mit Hilfe des ersten Modells erzeugt. Sie werden üblicherweise auf Basis einer ersten Referenz-Repräsentation und einer zweiten Referenz-Repräsentation erzeugt. Die erste Referenz-Repräsentation repräsentiert den Referenzbereich des jeweiligen Referenzobjekts ohne Kontrastmittel oder nach Applikation einer ersten Referenzmenge an Kontrastmittel. Die erste Referenzmenge kann der ersten Menge entsprechen. Die zweite Referenz-Repräsentation repräsentiert den Referenzbereich des jeweiligen Referenzobjekts nach Applikation einer zweiten Referenzmenge an Kontrastmittel. Die zweite Referenzmenge ist üblicherweise größer als die erste Referenzmenge. Die zweite Referenzmenge kann der zweiten Menge entsprechen. Die erste Referenz-Repräsentation und die zweite Referenz-Repräsentation werden dem ersten Modell zugeführt und das erste Modell erzeugt eine dritte Referenz-Repräsentation. Die dritte Referenz-Repräsentation repräsentiert den Referenzbereich des jeweiligen Referenzobjekts nach Applikation einer dritten Referenzmenge des Kontrastmittels. Die dritte Referenzmenge ist üblicherweise größer als die zweite Referenzmenge. Die dritte Referenzmenge kann der dritten Menge entsprechen. Die dritte Referenz-Repräsentation ist diejenige Referenzrepräsentation, die beim Trainieren des zweiten Modells dem zweiten Modell zugeführt wird.

Die Trainingsdaten umfassen ferner für jedes Referenzobjekt eine gemessene Referenz-Repräsentation als Zieldaten. Die gemessene Referenz-Repräsentation repräsentiert den Referenzbereich des jeweiligen Referenzobjekts nach der Applikation der dritten Referenzmenge des Kontrastmittels. Die gemessene Referenz-Repräsentation ist eine gemessene Repräsentation; sie repräsentiert den Referenzbereich des jeweiligen Referenzobjekts also so, wie er nach der Applikation der dritten Referenzmenge des Kontrastmittels tatsächlich ist (*ground truth*), und so, wie die vom ersten Modell erzeugte Referenz-Repräsentation den Referenzbereich repräsentieren sollte.

Das Trainieren des zweiten Modells umfasst für jedes Referenzobjekt der Vielzahl an Referenzobjekten:
∘ Zuführen der von dem ersten Modell erzeugten (dritten) Referenz-Repräsentation dem zweiten Modell,
∘ Empfangen einer korrigierten Referenz-Repräsentation von dem zweiten Modell,
∘ Reduzieren von Abweichungen zwischen der korrigierten Referenz-Repräsentation und der gemessenen Referenz-Repräsentation durch Modifizieren von Modellparametern.

Das Trainingsverfahren kann so lange durchgeführt werden, bis die Abweichungen ein vordefiniertes Minimum erreichen und/oder sich die Abweichungen durch Modifizieren von Modellparametern nicht mehr reduzieren lassen.

Das zweite Modell wird also trainiert, die von dem ersten Modell für verschiedene Referenzobjekte erzeugten Referenz-Repräsentationen so zu korrigieren, dass sie der jeweiligen gemessenen Referenz-Repräsentation möglichst nahekommen.

Das zweite Modell wird also z.B. trainiert, Rauschen und/oder Artefakte, die durch das erste Modell in den Referenz-Repräsentationen erzeugt werden, zu reduzieren oder zu eliminieren.

Fig. 3 zeigt beispielhaft und schematisch ein Verfahren zum Trainieren eines zweiten Modells. Das zweite Modell M2 wird anhand von Trainingsdaten TD trainiert. Die Trainingsdaten TD umfassen, für jedes Referenzobjekt einer Vielzahl an Referenzobjekten, eine von einem ersten Modell M1 erzeugte dritte Referenzrepräsentation RR3 eines Referenzbereichs des jeweiligen Referenzobjekts und eine gemessene dritte Referenzrepräsentation RR3^{M} des Referenzbereichs des Referenzobjekts. Die Trainingsdaten können optional noch weitere Daten umfassen (siehe unten).

In dem in Fig. 3 gezeigten Beispiel ist nur ein Satz an Trainingsdaten TD für ein einzelnes Referenzobjekt gezeigt.

Das Referenzobjekt ist ein Mensch; der Referenzbereich umfasst die Lunge des Menschen.

Die gemessene dritte Referenzrepräsentation RR3^{M} repräsentiert den Referenzbereich des Referenzobjekts nach Applikation einer dritten Menge eines Kontrastmittels. Es handelt sich bei der gemessenen dritten Referenzrepräsentation RR3^{M} um eine messtechnisch erzeugte Aufnahme; das heißt, die gemessene dritte Referenzrepräsentation RR3^{M} ist das Ergebnis einer radiologischen Untersuchung. Die gemessene dritte Referenzrepräsentation RR3^{M} kann beispielsweise eine CT-Aufnahme, eine MRT-Aufnahme, eine Ultraschallaufnahme oder eine andere radiologische Aufnahme sein.

Die dritte Referenzrepräsentation RR3 wird mittels des ersten Modells M1 erzeugt. Das erste Model M1 ist konfiguriert, auf Basis einer ersten Referenzrepräsentation RR1 und einer zweiten Referenzrepräsentation RR2 die dritte Referenzrepräsentation RR3 zu erzeugen.

Die erste Referenzrepräsentation RR1 repräsentiert den Referenzbereich des Referenzobjekts ohne Kontrastmittel oder nach Applikation einer ersten Menge des Kontrastmittels. Die zweite Referenzrepräsentation RR2 repräsentiert den Referenzbereich des Referenzobjekts nach Applikation einer zweiten Menge des Kontrastmittels. Die zweite Menge ist größer als die erste Menge. Die dritte Repräsentation RR3 repräsentiert den Referenzbereich des Referenzobjekts nach Applikation der dritten Menge des Kontrastmittels.

Die von dem ersten Modell M1 erzeugte dritte Referenzrepräsentation RR3 wird dem zweiten Modell M2 zugeführt. Optional können dem zweiten Modell noch weitere Eingabedaten zugeführt werden, z.B. die erste Referenzrepräsentation RR1 und/oder die zweite Referenzrepräsentation RR2 und/oder weitere/andere Daten FD. Die weiteren Eingabedaten können Daten sein, die das verwendete Kontrastmittel, die erste Menge des Kontrastmittels, die zweite Menge des Kontrastmittels, die dritte Menge des Kontrastmittels, den Verstärkungsfaktor, Aufnahmeparameter für die Erzeugung der ersten und/oder zweiten Repräsentation, das Referenzobjekt, den Referenzbereich und/oder andere Eigenschaften/Bedingungen spezifizieren. Die optionale Verwendung der weiteren Eingabedaten wird in Fig. 3 durch die gestrichelten Pfeile symbolisiert. Bei einer Verwendung von weiteren Eingabedaten sind diese weiteren Eingabedaten ebenfalls Trainingsdaten.

Das zweite Modell M2 ist konfiguriert, auf Basis der dritten Referenzrepräsentation RR3 und auf Basis von Modellparametern MP (und ggf. weiteren Eingabedaten) eine korrigierte dritte Repräsentation RR3^{C} zu erzeugen. Das zweite Modell M2 wird trainiert, eine korrigierte dritte Repräsentation RR3^{C} zu erzeugen, die der gemessenen dritten Repräsentation RR3^{M} möglichst nahekommt. Dazu wird die korrigierte dritte Repräsentation RR3^{C} mit der gemessenen dritten Repräsentation RR3^{M} verglichen. Mit Hilfe einer Fehlerfunktion LF werden Abweichungen zwischen der korrigierten dritten Repräsentation RR3^{C} und der gemessenen dritten Repräsentation RR3^{M} quantifiziert. In einem Optimierungsverfahren (z.B. einem Gradientenverfahren) werden Modellparameter MP so modifiziert, dass die Abweichungen und damit der mittels der Fehlerfunktion LF berechnete Fehler reduziert (minimiert) wird. Der Vorgang wird für weitere Trainingsdatensätze weiterer Referenzobjekte wiederholt, bis die Abweichungen auf ein vordefiniertes Minimum reduziert worden sind und/oder sich die Abweichungen durch Modifizieren von Modellparametern nicht weiter reduzieren lassen.

Das trainierte zweite Modell kann in einem Datenspeicher gespeichert, an ein separates Computersystem (z.B. über ein Netzwerk) übermittelt und/oder zur Erzeugung einer korrigierten dritten Repräsentation eines Untersuchungsbereichs eines Untersuchungsobjekts verwendet werden.

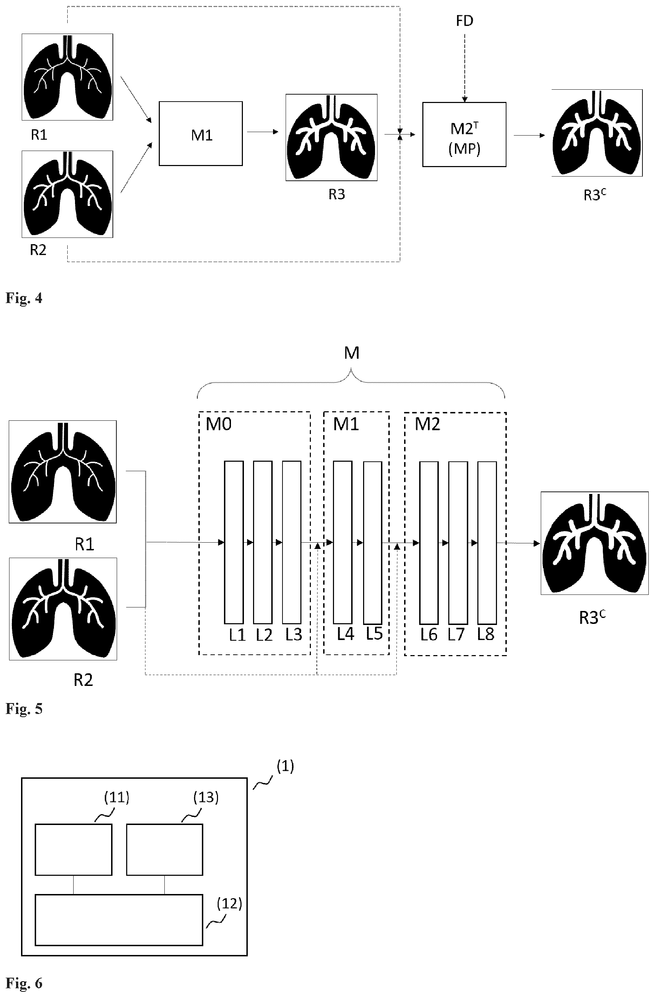

Fig. 4 zeigt beispielhaft und schematisch die Erzeugung einer korrigierten dritten Repräsentation auf Basis einer dritten Repräsentation mit Hilfe eines trainierten zweiten Modells.

Das trainierte zweite Modell M2^{T} kann beispielsweise wie in Bezug zu Fig. 3 beschrieben trainiert worden sein. Dem trainierten zweiten Modell M2^{T} wird eine dritte Repräsentation R3 eines Untersuchungsbereichs eines Untersuchungsobjekts zugeführt. Optional können dem trainierten zweiten Modell M2^{T} weitere Eingabedaten zugeführt werden. Die weiteren Eingabedatendaten können Daten sein, die das verwendete Kontrastmittel, die erste Menge des Kontrastmittels, die zweite Menge des Kontrastmittels, die dritte Menge des Kontrastmittels, den Verstärkungsfaktor, Aufnahmeparameter für die Erzeugung der ersten und/oder zweiten Repräsentation, das Untersuchungsobjekt, den Untersuchungsbereich und/oder andere Eigenschaften/Bedingungen spezifizieren. Die optionale Verwendung der weiteren Eingabedaten wird in Fig. 4 durch die gestrichelten Pfeile symbolisiert. Weitere Eingabedaten werden insbesondere dann verwendet, wenn solche Daten auch zum Trainieren des zweiten Modells verwendet wurden.

Das Untersuchungsobjekt ist in dem in Fig. 4 gezeigten Beispiel ein Mensch; der Untersuchungsbereich umfasst die Lunge des Menschen.

Die dritte Repräsentation R3 wird mit Hilfe eines ersten Modells M1 erzeugt. Das erste Modell M1 ist konfiguriert, die dritte Repräsentation auf Basis einer ersten Repräsentation R1 und einer zweiten Repräsentation R2 zu erzeugen. Die erste Repräsentation R1 repräsentiert den Untersuchungsbereich des Untersuchungsobjekts ohne Kontrastmittel oder nach Applikation einer ersten Menge eines Kontrastmittels. Die zweite Repräsentation R2 repräsentiert den Untersuchungsbereich des Untersuchungsobjekts nach Applikation einer zweiten Menge des Kontrastmittels. Die zweite Menge ist größer als die erste Menge. Die dritte Repräsentation R3 repräsentiert den Untersuchungsbereich des Untersuchungsobjekts nach Applikation einer dritten Menge eines Kontrastmittels. Die dritte Menge ist verschieden von der ersten Menge und der zweiten Menge. Im vorliegenden Beispiel ist die dritte Menge größer als die zweite Menge, d.h. das erste Modell erzeugt eine dritte Repräsentation R3, die gegenüber der zweiten Repräsentation R2 eine Kontrastverstärkung aufweist.

Das trainierte zweite Modell M2^{T} ist konfiguriert und trainiert, auf Basis der von dem ersten Modell M1 erzeugten dritten Repräsentation R3 (und ggf. weiteren Eingabedaten) eine korrigierte dritte Repräsentation R3^{C} zu erzeugen. Die korrigierte dritte Repräsentation R3^{C} ist eine gegenüber der dritten Repräsentation R3 verbesserte künstliche radiologische Aufnahme, da sie z.B. weniger Rauschen und/oder Artefakte umfasst.

Das zweite Modell kann ein künstliches neuronales Netzwerk sein oder ein solches umfassen.

Ein "künstliches neuronales Netzwerk" umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen der Eingabe-Repräsentationen. Üblicherweise gibt es ein Eingangsneuron für jedes Pixel oder Voxel einer Eingabe-Repräsentation, falls es sich bei der Repräsentation um eine Ortsraumdarstellung in Form einer Rastergrafik handelt, oder ein Eingangsneuron für jede Frequenz, die in der Eingabe-Repräsentation vorhanden ist, falls es sich bei der Repräsentation um eine Frequenzraumdarstellung handelt. Es können zusätzliche Eingangsneuronen für zusätzliche Eingangswerte (z.B. Informationen zum Untersuchungsbereich, zum Untersuchungsobjekt, zu Bedingungen, die bei der Erzeugung der Eingabe-Repräsentation herrschten, Informationen zu dem Zustand, den die Eingabe-Repräsentation repräsentiert, und/oder Informationen zu dem Zeitpunkt oder der Zeitspanne zu/in der die Eingabe-Repräsentation erzeugt worden ist) vorhanden sein.

Die Ausgangsneuronen dienen dazu, eine synthetische radiologische Aufnahme auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Das künstliche neuronale Netzwerk kann ein Convolutional Neural Network (kurz: CNN) sein oder ein solches umfassen.

Ein Convolutional Neural Network ist in der Lage, Eingabedaten in Form einer Matrix zu verarbeiten. Dies ermöglicht es, als Matrix dargestellte digitale radiologische Aufnahmen (z.B. Breite x Höhe x Farbkanäle) als Eingabedaten zu verwenden. Ein normales neuronales Netzwerk z.B. in Form eines Multi-Layer-Perceptrons (MLP) benötigt dagegen einen Vektor als Eingabe, d.h. um eine radiologische Aufnahme als Eingabe zu verwenden, müssten die Pixel oder Voxel der radiologischen Aufnahme in einer langen Kette hintereinander ausgerollt werden. Dadurch sind normale neuronale Netzwerke z.B. nicht in der Lage, Objekte in einer radiologischen Aufnahme unabhängig von der Position des Objekts in der Aufnahme zu erkennen. Das gleiche Objekt an einer anderen Position in der Aufnahme hätte einen völlig anderen Eingabevektor.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netzwerk kann insbesondere ein *Generative Adversarial Network* (GAN) für Superauflösung von Bildern (*image super-resolution* (SR)) sein (siehe z.B. C. Ledig et al.: Photo-Realistic Single Image Super-Resolution Using a Generative Adversarial Network, arXiv:1609.04802v5).

Das künstliche neuronale Netzwerk kann ein Transfomer-Netzwerk sein (siehe z.B. D. Karimi et al.: Convolution-Free Medical Image Segmentation using Transformers, arXiv:2102.13645 [eess.IV]).

Ist das erste Modell differenzierbar, dann können das erste Modell und das zweite Modell auch in einem gemeinsamen Modell zusammengefasst werden. In einem solchen Fall sind das erste Modell und das zweite Modell Bestandteile eines gemeinsamen Modells, wobei die von dem ersten Modell erzeugte dritte (Referenz-)Repräsentation direkt dem zweiten Modell zugeführt wird.

Das in dieser Beschreibung beispielsweise in Bezug zu Fig. 1 und Fig. 2 beschriebene erste Modell ist differenzierbar. Es kann beispielsweise als eine oder mehrere Schichten einem als neuronalen Netzwerk ausgeführten zweiten Modell vorangestellt werden. Diese eine oder mehreren Schichten können die Rechenoperationen mit den entsprechenden Rechenparametern (z.B. den Verstärkungsfaktor α) als feste (unveränderliche) Werte umfassen, während Modellparameter des zweiten Modells variabel sein können. Auf diese Weise kann das erste Modell in das Training des zweiten Modells einbezogen werden, wobei die Modellparameter des ersten Modells bei dem Training unverändert bleiben. In einem solchen Fall gibt das erste Modell mittels der unveränderlichen Modellparameter die physikalischen Grundlagen vor, die das zweite Modell berücksichtigen/akzeptieren/hinnehmen muss. Im Gegensatz zu einem völlig variablen Modell zum Erzeugen von künstlichen kontrastverstärkten radiologischen Aufnahmen, wie es beispielsweise in WO2019/074938A1 beschrieben ist, hat der hier beschriebene Ansatz den Vorteil, dass dem Modell die physikalischen Grundlagen, innerhalb derer es sich bewegen kann, in Form des ersten Modells als Wissen vorgegeben werden können. Auf diese Weise werden die erzeugten synthetischen radiologischen Aufnahmen realistischer.

Es ist auch möglich, in einem als neuronales Netzwerk ausgeführten gemeinsamen Modell umfassend das erste Modell und das zweite Modell dem ersten Modell eine oder mehrere weitere Schichten voranzustellen. Diese eine oder mehreren weiteren Schichten können beispielsweise trainierbare (veränderliche) Modellparameter umfassen, die für eine (verbesserte) Co-Registrierung der ersten (Referenz-)Repräsentation und der zweiten (Referenz-)Repräsentation sorgen. Die (verbesserte) Co-Registrierung kann dann ein Bestandteil des Trainings sein. Die eine oder mehreren weiteren, dem ersten Modell vorangestellten Schichten können auch trainierbare Modellparameter umfassen, die einen oder mehrere Modellparameter des ersten Modells lernen, z.B. einen optimierten Verstärkungsfaktor α und/oder Parameter der Gewichtsfunktion im Falle einer Gewichtung wie in Fig. 2 gezeigt. Diese eine oder mehreren, dem ersten Modell vorangestellten Schichten können beispielsweise eine Architektur wie ein DenseNet aufweisen (siehe z.B. G. Haung et al.: Densely Connected Convolutional Networks, arXiv:1608.06993v5).

Ein Modell umfassend das erste Modell und das zweite Modell kann in einem Ende-zu-Ende Verfahren trainiert werden.

Es ist ferner möglich, dem zweiten Modell nicht nur die von dem ersten Modell erzeugte (Referenz-)Repräsentation zuzuführen, sondern zusätzlich die erste (Referenz-)Repräsentation und/oder die zweite (Referenz-)Repräsentation. Auf diese Weise kann das zweite Modell trainiert werden, Artefakte in der dritten (Referenz-)Repräsentation, die auf eine unzureichende Co-Registrierung zurückzuführen sind, zu reduzieren oder zu eliminieren.

Fig. 5 zeigt beispielhaft und schematisch ein Modell umfassend ein erstes Modell und ein zweites Modell.

Das in Fig. 5 dargestellte Modell M weist verschiedene Verarbeitungsschichten L1, L2, L3, L4, L5, L6, L7 und L8 auf. Die Zahl der Verarbeitungsschichten wurde rein willkürlich gewählt; die dargestellten Verarbeitungsschichten dienen lediglich der Veranschaulichung. Die Verarbeitungsschichten L4 und L5 bilden das erste Modell M1; die Verarbeitungsschichten L6, L7 und L8 bilden das zweite Modell M2. Dem ersten Modell M1 sind die Verarbeitungsschichten L1, L2 und L3 vorangestellt; sie bilden ein initiales Modell M0.

Dem initialen Modell M0 werden eine erste Repräsentation R1 und eine zweite Repräsentation R2 zugeführt. Die erste Repräsentation R1 repräsentiert einen Untersuchungsbereich eines Untersuchungsobjekts ohne Kontrastmittel oder nach Applikation einer ersten Menge eines Kontrastmittels; die zweite Repräsentation R2 repräsentiert den Untersuchungsbereich des Untersuchungsobjekts nach Applikation einer zweiten Menge des Kontrastmittels; die zweite Menge ist größer als die erste Menge; das Untersuchungsobjekt ist ein Mensch; der Untersuchungsbereich umfasst die Lunge des Menschen.

Das initiale Modell M0 kann konfiguriert sein, eine Co-Registrierung der ersten Repräsentation R1 und der zweiten Repräsentation R2 durchzuführen und/oder Modellparameter des ersten Modells M1 zu ermitteln. Die co-registrierten Repräsentationen können über die Schicht L3 des initialen Modells M0 der Schicht L4 des ersten Modells M1 übergeben werden. Ebenso ist es möglich, dass an dieser Stelle von dem initialen Modell M0 ermittelte Modellparameter dem ersten Modell M1 übergeben werden, während die erste Repräsentation R1 und die zweite Repräsentation R2 dem ersten Modell M1 separat zugeführt werden (siehe gestrichelten Pfeil).

Das erste Modell M1 ist konfiguriert, auf Basis der (co-registrierten) Repräsentation R1 und der (co-registrierten) Repräsentation R2 eine dritte Repräsentation (in Fig. 5 nicht dargestellt) zu erzeugen. Die dritte Repräsentation repräsentiert den Untersuchungsbereich des Untersuchungsobjekts nach Applikation einer dritten Menge des Kontrastmittels; die dritte Menge ist verschieden von der ersten Menge und der zweiten Menge; vorzugsweise ist die dritte Menge größer als die zweite Menge.

Die von dem ersten Modell M1 erzeugte dritte Repräsentation wird über die Schicht L5 des ersten Modells M1 an die Schicht L6 des zweiten Modells M2 übergeben.

Dem zweiten Modell M2 können auch die optional co-registrierte erste Repräsentation R1 und/oder die optional co-registrierte zweite Repräsentation R2 zugeführt werden (siehe gestrichelten Pfeil).

Das zweite Modell M2 ist konfiguriert und trainiert, auf Basis der dritten Repräsentation (und ggf. auf Basis der (co-registrierten) ersten Repräsentation R1 und/oder der (co-registrierten) zweiten Repräsentation) eine korrigierte dritte Repräsentation R3^{C} zu erzeugen. Die korrigierte dritte Repräsentation R3^{C} weist gegenüber der dritten Repräsentation weniger Rauschen und/oder weniger Artefakte auf. Die korrigierte dritte Repräsentation R3^{C} kann über die Schicht L8 des zweiten Modells M2 ausgegeben werden.

Fig. 6 zeigt beispielhaft und schematisch ein Computersystem gemäß der vorliegenden Offenbarung.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Das in Fig. 6 gezeigte Computersystem (1) umfasst eine Empfangseinheit (11), eine Steuer- und Recheneinheit (12) und eine Ausgabeeinheit (13).

Die Steuer- und Recheneinheit (12) dient der Steuerung des Computersystems (1), der Koordinierung der Datenflüsse zwischen den Einheiten des Computersystems (1) und der Durchführung von Berechnungen.

Die Steuer- und Recheneinheit (12) ist konfiguriert:
- eine erste Repräsentation zu erzeugen oder die Empfangseinheit (11) zu veranlassen, die erste Repräsentation zu empfangen, wobei die erste Repräsentation einen Untersuchungsbereich eines Untersuchungsobjekts ohne Kontrastmittel oder nach Applikation einer ersten Menge eines Kontrastmittels repräsentiert,
- eine zweite Repräsentation zu erzeugen oder die Empfangseinheit (11) zu veranlassen, die zweite Repräsentation zu empfangen, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- die erste Repräsentation und die zweite Repräsentation einem ersten Modell zuzuführen, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
- die dritte Repräsentation einem zweiten Modell zuzuführen,
   ∘ wobei das zweite Modell in einem Trainingsverfahren auf Basis von Trainingsdaten trainiert wurde,
   ∘ wobei die Trainingsdaten für jedes Referenzobjekt einer Vielzahl an Referenzobjekten
      (i) eine von dem ersten Modell erzeugte Referenz-Repräsentation, die einen Referenzbereich des Referenzobjekts nach Applikation einer Referenzmenge des Kontrastmittels repräsentiert, und
      (ii) eine gemessene Referenz-Repräsentation des Referenzbereichs des Referenzobjekts nach Applikation der Referenzmenge des Kontrastmittels
      umfasst,
   ∘ wobei das Trainingsverfahren für jedes Referenzobjekt umfasst:
      ▪ Zuführen der von dem ersten Modell erzeugten Referenz-Repräsentation dem zweiten Modell,
      ▪ Empfangen einer korrigierten Referenz-Repräsentation von dem zweiten Modell,
      ▪ Reduzieren von Abweichungen zwischen der korrigierten Referenz-Repräsentation und der gemessenen Referenz-Repräsentation durch Modifizieren von Modellparametern,
- eine korrigierte dritte Repräsentation des Untersuchungsbereichs des Untersuchungsobjekts von dem zweiten Modell zu empfangen,
- die Ausgabeeinheit (13) zu veranlassen die korrigierte dritte Repräsentation auszugeben, zu speichern und/oder an ein separates Computersystem zu übermitteln.

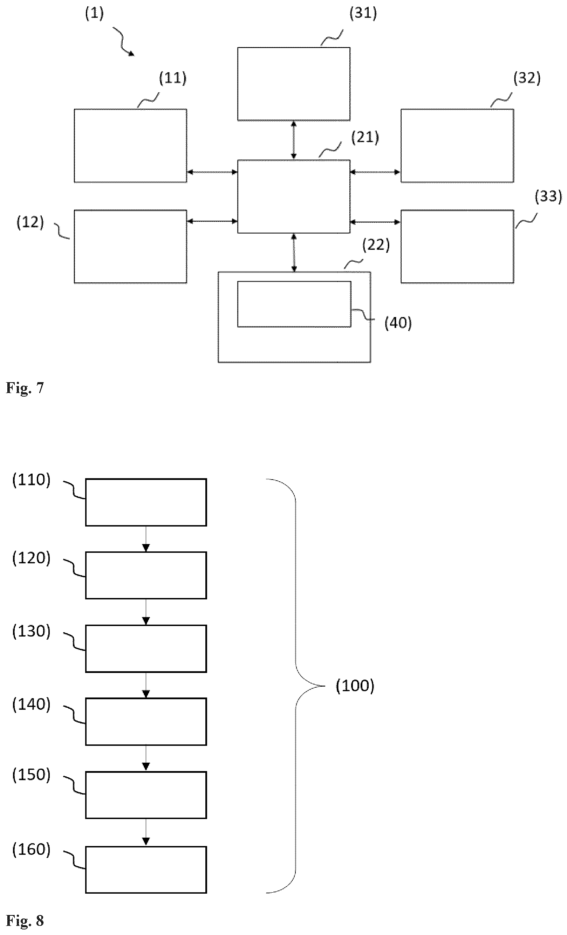

Fig. 7 zeigt beispielhaft und schematisch eine weitere Ausführungsform des Computersystems. Das Computersystem (1) umfasst eine Verarbeitungseinheit (21), die mit einem Speicher (22) verbunden ist. Die Verarbeitungseinheit (21) und der Speicher (22) bilden eine Steuer- und Recheneinheit, wie sie in Fig. 6 gezeigt ist.

Die Verarbeitungseinheit (21) (engl.: *processing unit*) kann einen oder mehrere Prozessoren allein oder in Kombination mit einem oder mehreren Speichern umfassen. Bei der Verarbeitungseinheit (21) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (21) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (21) kann konfiguriert sein, Computerprogramme auszuführen, die in einem Arbeitsspeicher der Verarbeitungseinheit (21) oder im Speicher (22) desselben oder eines anderen Computersystems gespeichert sein können.

Der Speicher (22) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (22) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (22) kann die Verarbeitungseinheit (21) auch mit einer oder mehreren Schnittstellen (11, 12, 31, 32, 33) verbunden sein, um Informationen anzuzeigen, zu übertragen und/oder zu empfangen. Die Schnittstellen können eine oder mehrere Kommunikationsschnittstellen (11, 32, 33) und/oder eine oder mehrere Benutzerschnittstellen (12, 31) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (31) umfassen. Eine Anzeige (31) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (11, 12) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (40) können im Speicher (22) gespeichert sein und von der Verarbeitungseinheit (21) ausgeführt werden, die dadurch programmiert wird, die in dieser Beschreibung beschriebenen Funktionen zu erfüllen. Das Abrufen, Laden und Ausführen von Anweisungen des Computerprogramms (40) kann sequenziell erfolgen, so dass jeweils ein Befehl abgerufen, geladen und ausgeführt wird. Das Abrufen, Laden und/oder Ausführen kann aber auch parallel erfolgen.

Das Computersystem der vorliegenden Offenbarung kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

Gegenstand der vorliegenden Erfindung ist auch ein Computerprogrammprodukt. Ein solches Computerprogrammprodukt umfasst einen nichtflüchtigen Datenträger wie beispielsweise eine CD, eine DVD, ein USB-Stick oder ein anderes Medium zum Speichern von Daten. Auf dem Datenträger ist ein Computerprogramm gespeichert. Das Computerprogramm kann in einen Arbeitsspeicher eines Computersystems (insbesondere in einen Arbeitsspeicher eines Computersystems der vorliegenden Offenbarung) geladen werden und dort das Computersystem dazu veranlassen, folgende Schritte ausführen:
- Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
- Zuführen der dritten Repräsentation einem zweiten Modell,
   ∘ wobei das zweite Modell in einem Trainingsverfahren auf Basis von Trainingsdaten trainiert wurde,
   ∘ wobei die Trainingsdaten für jedes Referenzobjekt einer Vielzahl an Referenzobjekten
      (i) eine von dem ersten Modell erzeugte Referenz-Repräsentation, die einen Referenzbereich des Referenzobjekts nach Applikation einer Referenzmenge des Kontrastmittels repräsentiert, und
      (ii) eine gemessene Referenz-Repräsentation des Referenzbereichs des Referenzobjekts nach Applikation der Referenzmenge des Kontrastmittels
      umfasst,
   ∘ wobei das Trainingsverfahren für jedes Referenzobjekt umfasst:
      ▪ Zuführen der von dem ersten Modell erzeugten Referenz-Repräsentation dem zweiten Modell,
      ▪ Empfangen einer korrigierten Referenz-Repräsentation von dem zweiten Modell,
      ▪ Reduzieren von Abweichungen zwischen der korrigierten Referenz-Repräsentation und der gemessenen Referenz-Repräsentation durch Modifizieren von Modellparametern,
- Empfangen einer korrigierten dritten Repräsentation des Untersuchungsbereichs des Untersuchungsobjekts von dem zweiten Modell,
- Ausgeben und/oder Speichern der korrigierten dritten Repräsentation und/oder Übermitteln der korrigierten dritten Repräsentation an ein separates Computersystem.

Das Computerprogramm kann auch als Download z.B. über eine Internetseite und/oder einen App-Store als Computerprogrammprodukt zum Kauf angeboten werden.

Das Computerprogrammprodukt kann auch in Kombination (in einer Zusammenstellung) mit dem Kontrastmittel vermarktet werden. Eine solche Zusammenstellung wird auch als Kit bezeichnet. Ein solches Kit umfasst das Kontrastmittel und das Computerprogrammprodukt. Es ist auch möglich, dass ein solches Kit das Kontrastmittel und Mittel umfasst, die es einen Käufer erlauben, das Computerprogramm zu beziehen, z.B. von einer Internetseite herunterzuladen. Diese Mittel können einen Link, d.h. eine Adresse der Internetseite umfassen, auf der das Computerprogramm bezogen, z.B. von der das Computerprogramm auf ein mit dem Internet verbundenes Computersystem heruntergeladen werden kann. Diese Mittel können einen Code (z.B. eine alphanumerische Zeichenkette oder einen QR-Code, oder einen DataMatrix-Code oder einen Barcode oder eine anderen optisch und/oder elektronisch lesbaren Code) umfassen, mit dem der Käufer Zugang zu dem Computerprogramm erhält. Ein solcher Link und/oder Code kann beispielsweise auf einer Verpackung des Kontrastmittels aufgedruckt und/oder auf einem Beipackzettel zum Kontrastmittel aufgedruckt sein. Ein Kit ist somit ein Kombinationsprodukt umfassend ein Kontrastmittel und ein Computerprogramm (z.B. in Form eines Zugangs zu dem Computerprogramm oder in Form von ausführbarem Programmcode auf einem Datenträger), das zusammen zum Kauf angeboten wird.

Fig. 8 zeigt beispielhaft und schematisch eine Ausführungsform des computer-implementierten Verfahrens in Form eines Ablaufschemas.

Das Verfahren (100) umfasst die Schritte:
(110) Bereitstellen einer ersten Repräsentation, wobei die erste Repräsentation einen Untersuchungsbereich eines Untersuchungsobjekts ohne Kontrastmittel oder nach Applikation einer ersten Menge eines Kontrastmittels repräsentiert,
(120) Bereitstellen einer zweiten Repräsentation, wobei die zweite Repräsentation den Untersuchungsbereich des Untersuchungsobjekts nach Applikation einer zweiten Menge des Kontrastmittels repräsentiert, wobei die zweite Menge größer als die erste Menge ist,
(130) Zuführen der ersten Repräsentation und der zweiten Repräsentation einem ersten Modell, wobei das erste Modell konfiguriert ist, auf Basis der ersten Repräsentation und der zweiten Repräsentation eine dritte Repräsentation zu erzeugen, wobei die dritte Repräsentation den Untersuchungsbereich des Untersuchungsobjekts nach Applikation einer dritten Menge des Kontrastmittels repräsentiert, wobei die dritte Menge verschieden von der ersten Menge und der zweiten Menge ist,
(140) Zuführen der dritten Repräsentation einem zweiten Modell,
   ∘ wobei das zweite Modell in einem Trainingsverfahren auf Basis von Trainingsdaten trainiert wurde,
   ∘ wobei die Trainingsdaten für jedes Referenzobjekt einer Vielzahl an Referenzobjekten
      (i) eine von dem ersten Modell erzeugte Referenz-Repräsentation, die einen Referenzbereich des Referenzobjekts nach Applikation einer Referenzmenge des Kontrastmittels repräsentiert, und
      (ii) eine gemessene Referenz-Repräsentation des Referenzbereichs des Referenzobjekts nach Applikation der Referenzmenge des Kontrastmittels
      umfasst,
   o wobei das Trainingsverfahren für jedes Referenzobjekt umfasst:
      ▪ Zuführen der von dem ersten Modell erzeugten Referenz-Repräsentation dem zweiten Modell,
      ▪ Empfangen einer korrigierten Referenz-Repräsentation von dem zweiten Modell,
      ▪ Reduzieren von Abweichungen zwischen der korrigierten Referenz-Repräsentation und der gemessenen Referenz-Repräsentation durch Modifizieren von Modellparametern,
(150) Empfangen einer korrigierten dritten Repräsentation des Untersuchungsbereichs des Untersuchungsobjekts von dem zweiten Modell,
(160) Ausgeben und/oder Speichern der korrigierten dritten Repräsentation und/oder Übermitteln der korrigierten dritten Repräsentation an ein separates Computersystem.

Die vorliegende Erfindung kann für verschiedene Zwecke verwendet werden. Einige Anwendungsbeispiele werden nachfolgend beschrieben, ohne die Erfindung auf diese Anwendungsbeispiele beschränken zu wollen.

Ein erstes Anwendungsbeispiel betrifft magnetresonanztomographische Untersuchungen zur Abgrenzung von intraaxialen Tumoren wie intrazerebralen Metastasen und malignen Gliomen. Wegen des infiltrativen Wachstums dieser Tumoren ist eine genaue Abgrenzung zwischen Tumor und gesundem Gewebe schwierig. Die Bestimmung der Ausdehnung eines Tumors ist jedoch für eine operative Entfernung entscheidend. Die Unterscheidung zwischen Tumoren und gesundem Gewebe wird durch Applikation eines extrazellulären erleichtert; nach intravenöser Gabe einer Standarddosis von 0,1 mmol/kg Körpergewicht des extrazellulären MRT-Kontrastmittels Gadobutrol lassen sich intraaxiale Tumoren deutlich besser abgrenzen. Bei höheren Dosen wird der Kontrast zwischen Läsion und gesundem Hirngewebe weiter erhöht; die Nachweisrate von Hirnmetastasen steigt linear mit der Dosis des Kontrastmittels an (siehe z.B. M. Hartmann et al.: *Does the administration of a high dose of a paramagnetic contrast medium (Gadovist) improve the diagnostic value of magnetic resonance tomography in glioblastomas?* doi: 10.1055/s-2007-1015623).

Dabei können eine einzelne Dreifachdosis oder eine zweite Folgedosis bis zu einer Gesamtdosis von 0,3 mmol/kg Körpergewicht verabreicht werden. Dadurch werden der Patient und die Umgebung zusätzlichem Gadolinium ausgesetzt, und im Falle eines zweiten Scans entstehen weitere zusätzliche Kosten.

Die vorliegende Erfindung kann eingesetzt werden, um eine Kontrastmitteldosis, die über die Standardmenge hinausgeht, zu vermeiden. Es können eine erste MRT-Aufnahme ohne Kontrastmittel oder mit einer geringeren Menge als die Standardmenge und eine zweite MRT-Aufnahme mit der Standardmenge erzeugt werden. Auf Basis dieser erzeugten MRT-Aufnahmen kann wie in dieser Offenbarung beschrieben, eine synthetische MRT-Aufnahme erzeugt werden, bei der sich der Kontrast zwischen Läsionen und gesundem Gewebe durch Verändern des Verstärkungsfaktors α in weiten Grenzen variieren lässt. Dabei können Kontraste erreicht werden, die sonst nur durch Applikation einer Menge an Kontrastmittel erzielt werden können, die höher als die Standardmenge ist.

Ein weiteres Anwendungsbeispiel betrifft die Reduzierung der Menge an MRT-Kontrastmittel in einer magnetresonanztomographischen Untersuchung. Gadolinium-haltige Kontrastmittel wie Gadobutrol werden für eine Vielzahl an Untersuchungen eingesetzt. Sie dienen der Kontrastverstärkung bei Untersuchungen des Schädels, der Wirbelsäule, der Brust oder anderen Untersuchungen. Im zentralen Nervensystem hebt Gadobutrol Bereiche mit einer gestörten Blut-Hirn-Schranke und/oder abnormalen Gefäßen hervor. Im Brustgewebe macht Gadobutrol das Vorhandensein und Ausmaß einer bösartigen Brusterkrankung sichtbar. Gadobutrol wird auch in der kontrastverstärkten Magnetresonanzangiographie zur Diagnose von Schlaganfällen, zum Nachweis der Tumordurchblutung und zum Nachweis einer fokalen zerebralen Ischämie eingesetzt.

Aufgrund der zunehmenden Umweltbelastung, der Kostenbelastung des Gesundheitssystems und der Befürchtung akuter Nebenwirkungen und möglicher langfristiger Gesundheitsrisiken, insbesondere bei wiederholter und langfristiger Exposition, wird eine Dosisreduzierung von Gadolinium-haltigen Kontrastmitteln angestrebt. Dies kann durch die vorliegende Erfindung erreicht werden.

Es können eine erste MRT-Aufnahme ohne Kontrastmittel und eine zweite MRT-Aufnahme mit einer Kontrastmittelmenge erzeugt werden, die geringer als die Standardmenge ist. Auf Basis dieser erzeugten MRT-Aufnahmen kann wie in dieser Offenbarung beschrieben, eine synthetische MRT-Aufnahme erzeugt werden, bei der sich der Kontrast durch Verändern des Verstärkungsfaktors α in weiten Grenzen variieren lässt. Dabei kann mit einer geringeren Kontrastmittelmenge als der Standardmenge ein Kontrast erreicht werden, der dem Kontrast nach der Applikation der Standardmenge entspricht.

Ein weiteres Anwendungsbeispiel betrifft die Detektion, Identifizierung und/oder Charakterisierung von Läsionen in der Leber mit Hilfe eines hepatobiliären Kontrastmittels wie beispielsweise Primovist^{®}.

Primovist^{®} wird intravenös (i.v.) in einer Standarddosis von 0,025 mmol/kg Körpergewicht verabreicht. Diese Standarddosis ist geringer als die Standarddosis von 0,1 mmol/kg Körpergewicht bei extrazellulären MR-Kontrastmitteln. Im Vergleich zur kontrastverstärkten MRT mit extrazellulären Gadolinium-haltigen Kontrastmitteln ermöglicht Primovist^{®} eine dynamische T1w-Mehrphasenbildgebung. Aufgrund der niedrigeren Dosis von Primovist^{®} und der Beobachtung vorübergehender Bewegungsartefakte, die kurz nach der intravenösen Verabreichung auftreten können, wird die Kontrastverstärkung von Primovist^{®} in der arteriellen Phase jedoch von Radiologen als geringer wahrgenommen als die die Kontrastverstärkung von extrazellulären MRT-Kontrastmitteln. Die Beurteilung der Kontrastverstärkung in der arteriellen Phase und der Vaskularität von fokalen Leberläsionen ist für die genaue Charakterisierung der Läsion aber von entscheidender Bedeutung.

Mit Hilfe der vorliegenden Erfindung kann der Kontrast insbesondere in der arteriellen Phase erhöht werden, ohne dass eine höhere Dosis verabreicht werden muss.

Es können eine erste MRT-Aufnahme ohne Kontrastmittel und eine zweite MRT-Aufnahme während der arteriellen Phase nach der Applikation einer Kontrastmittelmenge erzeugt werden, die der Standardmenge entspricht. Auf Basis dieser erzeugten MRT-Aufnahmen kann wie in dieser Offenbarung beschrieben, eine synthetische MRT-Aufnahme erzeugt werden, bei der sich der Kontrast in der arteriellen Phase durch Verändern des Verstärkungsfaktors α in weiten Grenzen variieren lässt. Dabei können Kontraste erreicht werden, die sonst nur durch Applikation einer Menge an Kontrastmittel erzielt werden können, die höher als die Standardmenge ist.

Ein weiteres Anwendungsbeispiel betrifft die Verwendung von MRT-Kontrastmitteln in computertomographischen Untersuchungen.

MRT-Kontrastmittel entfalten üblicherweise in einer CT-Untersuchung eine geringere kontraststeigernde Wirkung als CT-Kontrastmittel. Dennoch kann es vorteilhaft sein, ein MRT-Kontrastmittel in einer CT-Untersuchung einzusetzen. Als Beispiel sei eine minimalinvasive Intervention in der Leber eines Patienten aufgeführt, bei der ein Chirurg den Eingriff mittels eines CT-Scanners verfolgt. Die Computertomographie (CT) hat gegenüber der Magnetresonanztomographie den Vorteil, dass während der Erzeugung von CT-Aufnahmen eines Untersuchungsbereichs eines Untersuchungsobjekts chirurgische Eingriffe in dem Untersuchungsbereich in größerem Umfang möglich sind. Es gibt hingegen nur wenige interventionelle Bestecke und chirurgische Geräte, die MRTtauglich sind. Zudem ist der Zugang zum Patienten durch die in der MRT verwendeten Magneten eingeschränkt. Während ein Chirurg also einen Eingriff in dem Untersuchungsbereich vornimmt, kann er den Untersuchungsbereich mit Hilfe der CT bildhaft darstellen und den Eingriff auf einem Monitor verfolgen.

Möchte ein Chirurg beispielsweise einen Eingriff in der Leber eines Patienten vornehmen, um z.B. eine Biopsie an einer Leberläsion durchzuführen oder einen Tumor zu entfernen, so ist in einer CT-Aufnahme der Leber der Kontrast zwischen einer Leberläsion oder dem Tumor und gesundem Lebergewebe nicht so ausgeprägt wie in einer MRT-Aufnahme nach der Applikation eines hepatobiliären Kontrastmittels. In der CT sind derzeit keine hepatobiliären CT-spezifischen Kontrastmittel bekannt und/oder zugelassen. Die Verwendung eines MRT-Kontrastmittels, insbesondere eines hepatobiliären MRT-Kontrastmittels in der Computertomographie kombiniert also die Möglichkeit der Differenzierung zwischen gesundem und erkranktem Lebergewebe und die Möglichkeit der Durchführung eines Eingriffs bei gleichzeitiger Darstellung der Leber.

Dabei kann die vergleichsweise geringe, durch das MRT-Kontrastmittel erzielte Kontrastverstärkung mit Hilfe der vorliegenden Erfindung erhöht werden, ohne dass eine höhere Dosis als die Standarddosis verabreicht werden muss.

Es können eine erste CT-Aufnahme ohne MRT-Kontrastmittel und eine zweite CT-Aufnahme nach der Applikation eines MRT-Kontrastmittels erzeugt werden, dessen Menge der Standardmenge entspricht. Auf Basis dieser erzeugten CT-Aufnahmen kann wie in dieser Offenbarung beschrieben, eine synthetische CT-Aufnahme erzeugt werden, bei der sich der durch das MRT-Kontrastmittel hervorgerufene Kontrast durch Verändern des Verstärkungsfaktors α in weiten Grenzen variieren lässt. Dabei können Kontraste erreicht werden, die sonst nur durch Applikation einer Menge an MRT-Kontrastmittel erzielt werden können, die höher als die Standardmenge ist.

## Patentansprüche

1. Computer-implementiertes Verfahren umfassend:
- Bereitstellen einer ersten Repräsentation (R1, R1^{I}, R1^{F}), wobei die erste Repräsentation (R1, R1^{I}, R1^{F}) einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation (R2, R2^{I}, R2^{F}), wobei die zweite Repräsentation (R2, R2^{I}, R2^{F}) den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation (R1, R1^{I}, R1^{F}) und der zweiten Repräsentation (R2, R2^{I}, R2^{F}) einem ersten Modell (M1), wobei das erste Modell (M1) konfiguriert ist, auf Basis der ersten Repräsentation (R1, R1^{I}, R1^{F}) und der zweiten Repräsentation (R2, R2^{I}, R2^{F}) eine dritte Repräsentation (R3, R3^{I}, R3^{F}) zu erzeugen, wobei die dritte Repräsentation (R3, R3^{I}, R3^{F}) den Untersuchungsbereich des Untersuchungsobjekts nach Applikation einer dritten Menge des Kontrastmittels repräsentiert, wobei die dritte Menge verschieden von der ersten Menge und der zweiten Menge ist,
wobei das Erzeugen der dritten Repräsentation (R3, R3^{I}, R3^{F}) durch das erste Modell (M1) umfasst:
∘ Subtrahieren der ersten Repräsentation (R1, R1^{I}, R1^{F}) von der zweiten Repräsentation (R2, R2^{I}, R2^{F}),
∘ Multiplizieren des Ergebnisses des Subtrahierens mit einem Verstärkungsfaktor α,
∘ Addieren des Ergebnisses des Multiplizierens auf die erste Repräsentation (R1, R1^{I}, R1^{F}),
- Zuführen der dritten Repräsentation (R3, R3^{I}, R3^{F}) einem zweiten Modell (M2),
∘ wobei das zweite Modell (M2) in einem Trainingsverfahren auf Basis von Trainingsdaten (TD) trainiert wurde,
∘ wobei die Trainingsdaten (TD) für jedes Referenzobjekt einer Vielzahl an Referenzobjekten
(i) eine von dem ersten Modell erzeugte Referenz-Repräsentation (RR3), die einen Referenzbereich des Referenzobjekts nach Applikation einer Referenzmenge des Kontrastmittels repräsentiert, und
(ii) eine gemessene Referenz-Repräsentation (RR3^{M}) des Referenzbereichs des Referenzobjekts nach Applikation der Referenzmenge des Kontrastmittels umfasst,
o wobei das Trainingsverfahren für jedes Referenzobjekt umfasst:
▪ Zuführen der von dem ersten Modell (M1) erzeugten Referenz-Repräsentation (RR3) dem zweiten Modell (M2), wobei das zweite Modell (M2) auf Basis der von dem ersten Modell (M1) erzeugten Referenz-Repräsentation (RR3) und auf Basis von Modellparametern (MP) eine korrigierte Referenz-Repräsentation (RR3^{C}) erzeugt,
▪ Empfangen der korrigierten Referenz-Repräsentation (RR3^{C}) von dem zweiten Modell (M2),
▪ Reduzieren von Abweichungen zwischen der korrigierten Referenz-Repräsentation (RR3^{C}) und der gemessenen Referenz-Repräsentation (RR3^{M}) durch Modifizieren der Modellparameter (MP),
- Empfangen einer korrigierten dritten Repräsentation (R3^{C}) des Untersuchungsbereichs des Untersuchungsobjekts von dem zweiten Modell (M2),
- Ausgeben und/oder Speichern der korrigierten dritten Repräsentation (R3^{C}) und/oder Übermitteln der korrigierten dritten Repräsentation (R3^{C}) an ein separates Computersystem.

2. Verfahren gemäß Anspruch 1, wobei das Erzeugen der dritten Repräsentation (R3, R3^{I}, R3^{F}) durch das erste Modell (M1) umfasst:
- Multiplizieren einer Frequenzraum-Repräsentation (R2^{F}-R1^{F}) einer Differenz der ersten Repräsentation (R1, R1^{I}, R1^{F}) von der zweiten Repräsentation (R2, R2^{I}, R2^{F}) mit einer frequenzabhängigen Gewichtsfunktion (WF) und dabei Erhalten einer gewichteten Repräsentation (R2^{F}-R1^{F})^{W},
- Multiplizieren der gewichteten Repräsentation (R2^{F}-R1^{F})^{W} mit einem Verstärkungsfaktor α,
- Addieren der mit dem Verstärkungsfaktor α multiplizierten gewichteten Repräsentation (R2^{F}-R1^{F})^{W} auf die erste Repräsentation (R1, R1^{I}, R1^{F}

3. Verfahren gemäß Anspruch 2, wobei die frequenzabhängige Gewichtsfunktion (WF) eine Hann-Fensterfunktion oder eine Poisson-Fensterfunktion ist.

4. Verfahren gemäß einem der Ansprüche 1 bis 3, wobei der Verstärkungsfaktor α größer als 1 ist, vorzugsweise größer als 2 ist.

5. Verfahren gemäß einem der Ansprüche 1 bis 3, wobei der Verstärkungsfaktor α größer als Null und kleiner als 1 ist.

6. Verfahren gemäß einem der Ansprüche 1 bis 3, wobei der Verstärkungsfaktor α kleiner als Null ist.

7. Verfahren gemäß einem der Ansprüche 1 bis 6, wobei die dritte Menge und die Referenzmenge größer als eine Standardmenge des Kontrastmittels sind.

8. Verfahren gemäß einem der Ansprüche 1 bis 7, wobei das zweite Modell (M2) ein künstliches neuronales Netzwerk ist, wobei das erste Modell (M1) eine oder mehrere Verarbeitungsschichten (L4, L5) umfasst, die dem zweiten Modell (M2) vorangestellt sind.

9. Verfahren gemäß Anspruch 8, wobei dem zweiten Modell (M2) eine oder mehrere weitere Verarbeitungsschichten (L1, L2, L3) vorangestellt sind, wobei die eine oder die mehreren weiteren Verarbeitungsschichten ein initiales Modell (M0) bilden, wobei das initiale Modell (M0) konfiguriert ist, den Verstärkungsfaktor α und/oder Parameter der frequenzabhängigen Gewichtsfunktion (WF) auf Basis der ersten Repräsentation (R1, R1^{I}, R1^{F}) und/oder der zweiten Repräsentation (R2, R2^{I}, R2^{F}) zu ermitteln und/oder eine Co-Registrierung der ersten Repräsentation (R1, R1^{I}, R1^{F}) und der zweiten Repräsentation (R2, R2^{I}, R2^{F}) durchzuführen.

10. Verfahren gemäß einem der Ansprüche 1 bis 9, wobei das Untersuchungsobjekt und jedes Referenzobjekt ein Mensch oder ein Tier, vorzugsweise ein Säugetier ist,
wobei der Untersuchungsbereich ein Teil des Untersuchungsobjekts ist und der Referenzbereich ein Teil des Referenzobjekts ist, wobei der Referenzbereich dem Untersuchungsbereich entspricht.

11. Verfahren gemäß einem der Ansprüche 1 bis 10, wobei die erste Repräsentation (R1, R1^{I}, R1^{F}) und die zweite Repräsentation (R2, R2^{I}, R2^{F}) das Ergebnis einer radiologischen Untersuchung, vorzugsweise einer MRT-Untersuchung und/oder einer CT-Untersuchung sind.

12. Verfahren nach einem der Ansprüche 1 bis 11, wobei das Kontrastmittel
- einen Gd³⁺-Komplex einer Verbindung der Formel (I) 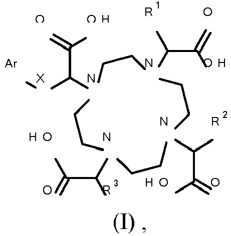
umfasst, wobei
Ar eine Gruppe ausgewählt aus 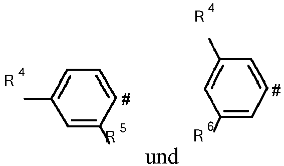 darstellt,
wobei # die Anknüpfung zu X darstellt,
X eine Gruppe darstellt, die aus
CH₂, (CH₂)₂, (CH₂)₃, (CH₂)₄ und *-(CH₂)₂-O-CH₂-^{#} ausgewählt wird,
wobei * die Anknüpfung zu Ar darstellt und ^{#} die Anknüpfung zum Essigsäurerest darstellt, R¹, R² und
R³ unabhängig voneinander ein Wasserstoffatom oder eine Gruppe ausgewählt aus C₁-C₃-Alkyl, -CH₂OH, -(CH₂)₂OH und -CH₂OCH₃ darstellen, R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon, oder
- einen Gd³⁺-Komplex einer Verbindung der Formel (II) 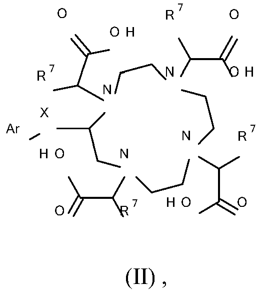
umfasst, wobei
Ar eine Gruppe ausgewählt aus 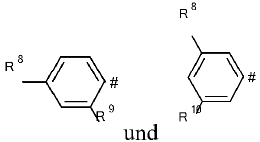 darstellt,
wobei # die Anknüpfung zu X darstellt,
X eine Gruppe darstellt, die aus CH₂, (CH₂)₂, (CH₂)₃, (CH₂)₄ und *-(CH₂)₂-O-CH₂-^{#} ausgewählt wird, wobei * die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon, oder
das Kontrastmittel eine der folgenden Substanzen umfasst:
- Gadolinium(III) 2-[4,7,10-tris(carboxymethyl)-1,4,7, 10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen[(±)-4-carboxy-5,8,11-tris(carboxymethyl)-1-phenyl-2-oxa-5,8,11-triazatridecan-13-oato(5-)]gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl} amino)acetyl]-amino}methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(2S)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

13. Computersystem (1) umfassend
• eine Empfangseinheit (11),
• eine Steuer- und Recheneinheit (12), und
• eine Ausgabeeinheit (13),
wobei die Steuer- und Recheneinheit (12) konfiguriert ist,
- eine erste Repräsentation (R1, R1^{I}, R1^{F}) zu erzeugen oder die Empfangseinheit (11) zu veranlassen, die erste Repräsentation (R1, R1^{I}, R1^{F}) zu empfangen, wobei die erste Repräsentation (R1, R1^{I}, R1^{F}) einen Untersuchungsbereich eines Untersuchungsobjekts ohne Kontrastmittel oder nach Applikation einer ersten Menge eines Kontrastmittels repräsentiert,
- eine zweite Repräsentation (R2, R2^{I}, R2^{F}) zu erzeugen oder die Empfangseinheit (11) zu veranlassen, die zweite Repräsentation (R2, R2^{I}, R2^{F}) zu empfangen, wobei die zweite Repräsentation (R2, R2^{I}, R2^{F}) den Untersuchungsbereich des Untersuchungsobjekts nach Applikation einer zweiten Menge des Kontrastmittels repräsentiert, wobei die zweite Menge größer als die erste Menge ist,
- die erste Repräsentation (R1, R1^{I}, R1^{F}) und die zweite Repräsentation (R2, R2^{I}, R2^{F}) einem ersten Modell (M1) zuzuführen, wobei das erste Modell (M1) konfiguriert ist, auf Basis der ersten Repräsentation (R1, R1^{I}, R1^{F}) und der zweiten Repräsentation (R2, R2^{I}, R2^{F}) eine dritte Repräsentation (R3, R3^{I}, R3^{F}) zu erzeugen, wobei die dritte Repräsentation (R3, R3^{I}, R3^{F}) den Untersuchungsbereich des Untersuchungsobjekts nach Applikation einer dritten Menge des Kontrastmittels repräsentiert, wobei die dritte Menge verschieden von der ersten Menge und der zweiten Menge ist,
wobei das Erzeugen der dritten Repräsentation (R3, R3^{I}, R3^{F}) durch das erste Modell (M1) umfasst:
∘ Subtrahieren der ersten Repräsentation (R1, R1^{I}, R1^{F}) von der zweiten Repräsentation (R2, R2^{I}, R2^{F}),
∘ Multiplizieren des Ergebnisses des Subtrahierens mit einem Verstärkungsfaktor α,
∘ Addieren des Ergebnisses des Multiplizierens auf die erste Repräsentation (R1, R1^{I}, R1^{F}),
- die dritte Repräsentation (R3, R3^{I}, R3^{F}) einem zweiten Modell (M2) zuzuführen,
∘ wobei das zweite Modell (M2) in einem Trainingsverfahren auf Basis von Trainingsdaten (TD) trainiert wurde,
∘ wobei die Trainingsdaten (TD) für jedes Referenzobjekt einer Vielzahl an Referenzobjekten
(i) eine von dem ersten Modell (M1) erzeugte Referenz-Repräsentation (RR3), die einen Referenzbereich des Referenzobjekts nach Applikation einer Referenzmenge des Kontrastmittels repräsentiert, und
(ii) eine gemessene Referenz-Repräsentation (RR3^{M}) des Referenzbereichs des Referenzobjekts nach Applikation der Referenzmenge des Kontrastmittels umfasst,
∘ wobei das Trainingsverfahren für jedes Referenzobjekt umfasst:
▪ Zuführen der von dem ersten Modell (M1) erzeugten Referenz-Repräsentation (RR3) dem zweiten Modell (M2), wobei das zweite Modell (M2) auf Basis der von dem ersten Modell (M1) erzeugten Referenz-Repräsentation (RR3) und auf Basis von Modellparametern (MP) eine korrigierte Referenz-Repräsentation (RR3^{C}) erzeugt,
▪ Empfangen der korrigierten Referenz-Repräsentation (RR3^{C}) von dem zweiten Modell (M2),
▪ Reduzieren von Abweichungen zwischen der korrigierten Referenz-Repräsentation (RR3^{C}) und der gemessenen Referenz-Repräsentation (RR3^{M}) durch Modifizieren der Modellparameter (MP),
- eine korrigierte dritte Repräsentation (R3^{C}) des Untersuchungsbereichs des Untersuchungsobjekts von dem zweiten Modell (M2) zu empfangen,
- die Ausgabeeinheit (13) zu veranlassen die korrigierte dritte Repräsentation (R3^{C}) auszugeben, zu speichern und/oder an ein separates Computersystem zu übermitteln.

14. Computerprogrammprodukt umfassend einen Datenträger, auf dem ein Computerprogramm (40) gespeichert ist, wobei das Computerprogramm (40) in einen Arbeitsspeicher (22) eines Computersystems (1) geladen werden kann und dort das Computersystem (1) dazu veranlasst, folgende Schritte auszuführen:
- Bereitstellen einer ersten Repräsentation (R1, R1^{I}, R1^{F}), wobei die erste Repräsentation (R1, R1^{I}, R1^{F}) einen Untersuchungsbereich eines Untersuchungsobjekts ohne Kontrastmittel oder nach Applikation einer ersten Menge eines Kontrastmittels repräsentiert,
- Bereitstellen einer zweiten Repräsentation (R2, R2^{I}, R2^{F}), wobei die zweite Repräsentation (R2, R2^{I}, R2^{F}) den Untersuchungsbereich des Untersuchungsobjekts nach Applikation einer zweiten Menge des Kontrastmittels repräsentiert, wobei die zweite Menge größer als die erste Menge ist,
- Zuführen der ersten Repräsentation (R1, R1^{I}, R1^{F}) und der zweiten Repräsentation (R2, R2^{I}, R2^{F}) einem ersten Modell (M1), wobei das erste Modell (M1) konfiguriert ist, auf Basis der ersten Repräsentation (R1, R1^{I}, R1^{F}) und der zweiten Repräsentation (R2, R2^{I}, R2^{F}) eine dritte Repräsentation (R3, R3^{I}, R3^{F}) zu erzeugen, wobei die dritte Repräsentation (R3, R3^{I}, R3^{F}) den Untersuchungsbereich des Untersuchungsobjekts nach Applikation einer dritten Menge des Kontrastmittels repräsentiert, wobei die dritte Menge verschieden von der ersten Menge und der zweiten Menge ist,
wobei das Erzeugen der dritten Repräsentation (R3, R3^{I}, R3^{F}) durch das erste Modell (M1) umfasst:
∘ Subtrahieren der ersten Repräsentation (R1, R1^{I}, R1^{F}) von der zweiten Repräsentation (R2, R2^{I}, R2^{F}),
∘Multiplizieren des Ergebnisses des Subtrahierens mit einem Verstärkungsfaktor α,
∘ Addieren des Ergebnisses des Multiplizierens auf die erste Repräsentation (R1, R1^{I}, R1^{F}),
- Zuführen der dritten Repräsentation (R3, R3^{I}, R3^{F}) einem zweiten Modell (M2),
∘ wobei das zweite Modell (M2) in einem Trainingsverfahren auf Basis von Trainingsdaten (TD) trainiert wurde,
∘ wobei die Trainingsdaten (TD) für jedes Referenzobjekt einer Vielzahl an Referenzobjekten
(i) eine von dem ersten Modell erzeugte Referenz-Repräsentation (RR3), die einen Referenzbereich des Referenzobjekts nach Applikation einer Referenzmenge des Kontrastmittels repräsentiert, und
(ii) eine gemessene Referenz-Repräsentation (RR3^{M}) des Referenzbereichs des Referenzobjekts nach Applikation der Referenzmenge des Kontrastmittels umfasst,
∘ wobei das Trainingsverfahren für jedes Referenzobjekt umfasst:
▪ Zuführen der von dem ersten Modell (M1) erzeugten Referenz-Repräsentation (RR3) dem zweiten Modell (M2), wobei das zweite Modell (M2) auf Basis der von dem ersten Modell (M1) erzeugten Referenz-Repräsentation (RR3) und auf Basis von Modellparametern (MP) eine korrigierte Referenz-Repräsentation (RR3^{C}) erzeugt,
▪ Empfangen der korrigierten Referenz-Repräsentation (RR3^{C}) von dem zweiten Modell (M2),
▪ Reduzieren von Abweichungen zwischen der korrigierten Referenz-Repräsentation (RR3^{C}) und der gemessenen Referenz-Repräsentation (RR3^{M}) durch Modifizieren der Modellparameter (MP),
- Empfangen einer korrigierten dritten Repräsentation (R3^{C}) des Untersuchungsbereichs des Untersuchungsobjekts von dem zweiten Modell (M2),
- Ausgeben und/oder Speichern der korrigierten dritten Repräsentation (R3^{C}) und/oder Übermitteln der korrigierten dritten Repräsentation (R3^{C}) an ein separates Computersystem.

15. Verwendung eines Kontrastmittels in einem radiologischen Untersuchungsverfahren, wobei das radiologische Untersuchungsverfahren das computer-implementierte Verfahren gemäß Anspruch 1 umfasst.

16. Verwendung gemäß Anspruch 15, wobei das radiologische Untersuchungsverfahren eine magnetresonanztomographische Untersuchung oder eine computertomographische Untersuchung ist und wobei das Kontrastmittel
- einen Gd³⁺-Komplex einer Verbindung der Formel (I) 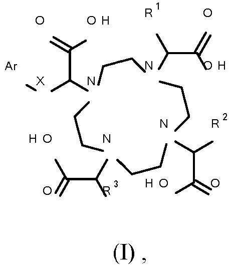
umfasst, wobei
Ar eine Gruppe ausgewählt aus 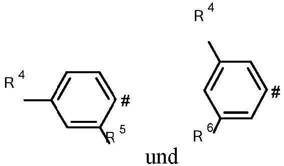 darstellt,
wobei # die Anknüpfung zu X darstellt,
X eine Gruppe darstellt, die aus
CH₂, (CH₂)₂, (CH₂)₃, (CH₂)₄ und *-(CH₂)₂-O-CH₂-^{#} ausgewählt wird,
wobei * die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon, oder
- einen Gd³⁺-Komplex einer Verbindung der Formel (II) 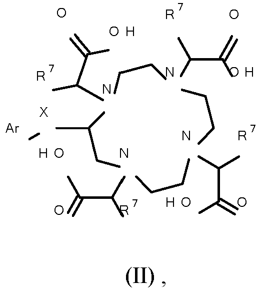
umfasst, wobei
Ar eine Gruppe ausgewählt aus 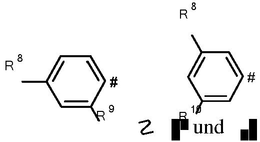 darstellt,
wobei # die Anknüpfung zu X darstellt,
X eine Gruppe darstellt, die aus CH₂, (CH₂)₂, (CH₂)₃, (CH₂)₄ und *-(CH₂)₂-O-CH₂-^{#} ausgewählt
wird, wobei * die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon, oder
- das Kontrastmittel eine der folgenden Substanzen umfasst:
- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen[(±)-4-carboxy-5,8,11-tris(carboxymethyl)-1-phenyl-2-oxa-5,8,11-triazatridecan-13-oato(5-)]gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazahepta-decan-2-yl}-1,4,7,10-tetraazacyclododecan-1-yllacetat,
- 2,2',2"-(10-{1-carboxy-2-[2-(4-ethoxyphenyl)ethoxylethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxyl phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(2S)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

17. Kit umfassend ein Computerprogrammprodukt gemäß Anspruch 14 und ein Kontrastmittel, wobei das Kontrastmittel vorzugsweise
- einen Gd³⁺-Komplex einer Verbindung der Formel (I) 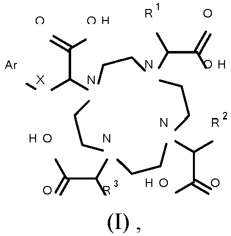
umfasst, wobei
Ar eine Gruppe ausgewählt aus 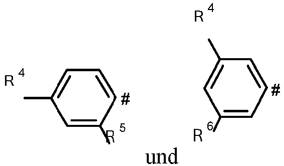 darstellt,
wobei # die Anknüpfung zu X darstellt,
X eine Gruppe darstellt, die aus
CH₂, (CH₂)₂, (CH₂)₃, (CH₂)₄ und *-(CH₂)₂-O-CH₂-^{#} ausgewählt wird,
wobei * die Anknüpfung zu Ar darstellt und ^{#} die Anknüpfung zum Essigsäurerest darstellt, R¹, R² und
R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und
R⁶ ein Wasserstoffatom darstellt,
oder ein Stereoisomer, Tautomer, Hydrat, Solvat oder Salz davon, oder eine Mischung davon, oder
- einen Gd³⁺-Komplex einer Verbindung der Formel (II) 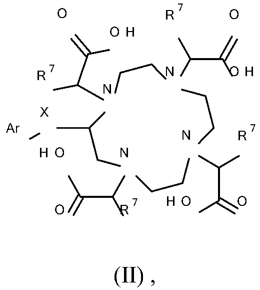
umfasst, wobei
Ar eine Gruppe ausgewählt aus 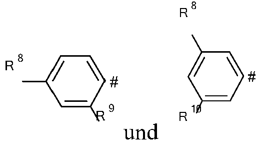 darstellt,
wobei # die Anknüpfung zu X darstellt,
X eine Gruppe darstellt, die aus CH₂, (CH₂)₂, (CH₂)₃, (CH₂)₄ und *-(CH₂)₂-O-CH₂-^{#} ausgewählt wird, wobei * die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon, oder
- das Kontrastmittel eine der folgenden Substanzen umfasst:
- Gadolinium(III) 2-[4,7,10-tris(carboxymethyl)-1,4,7,10-tetrazacyclododec-1-yl]essigsäure,
- Gadolinium(III) Ethoxybenzyl-diethylenetriaminepentaessigsäure,
- Gadolinium(III) 2-[3,9-bis[1-carboxylato-4-(2,3-dihydroxypropylamino)-4-oxobutyl]-3,6,9,15-tetrazabicyclo[9.3.1]pentadeca-1(15),11,13-trien-6-yl]-5-(2,3-dihydroxypropylamino)-5-oxopentanoat,
- Dihydrogen[(±)-4-carboxy-5,8,11-tris(carboxymethyl)-1-phenyl-2-oxa-5,8,11-triazatridecan-13-oato(5-)]gadolinat(2-),
- Tetragadolinium-[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl} amino)acetyl]-amino}methyl)-4,7,11,14-tetraazahepta-decan-2-yl}-1,4,7,10-tetraazacyclododecan-1-yl]acetat,
- 2,2',2"-(10-{1-carboxy-2-[2-(4-ethoxyphenyl)ethoxy]ethyl}-1,4,7,10-tetraazacyclododecan-1,4,7-triyl)triacetat,
- Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
- Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
- Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat)
- Gadolinium 2,2',2"-{10-[(1S)-4-(4-butoxyphenyl)-1-carboxybutyl]-1,4,7,10-tetraazacyclododecan-1,4,7-triyl}triacetat,
- Gadolinium(III) 5,8-bis(carboxylatomethyl)-2-[2-(methylamino)-2-oxoethyl]-10-oxo-2,5,8,11-tetraazadodecan-1-carboxylat-Hydrat
- Gadolinium(III) 2-[4-(2-hydroxypropyl)-7,10-bis(2-oxido-2-oxoethyl)-1,4,7,10-tetrazacyclododec-1-yl]acetat,
- Gadolinium(III) 2,2',2"-(10-((2R,3S)-1,3,4-trihydroxybutan-2-yl)-1,4,7,10-tetraazacyclododecan-1,4,7-triyl)triacetat,
- Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetat,
- Gadolinium-2,2',2"-[10-(carboxymethyl)-2-(4-ethoxybenzyl)-1,4,7,10-tetraazacyclododecane-1,4,7-triyl]triacetat.

## Claims

1. A computer-implemented method comprising:
- providing a first representation (R1, R1^{I}, R1^{F}), wherein the first representation (R1, R1^{I}, R1^{F}) represents an examination region of an examination object without contrast agent or after administration of a first amount of a contrast agent,
- providing a second representation (R2, R2^{I}, R2^{F}), wherein the second representation (R2, R2^{I}, R2^{F}) represents the examination region of the examination object after administration of a second amount of the contrast agent, wherein the second amount is greater than the first amount,
- feeding the first representation (R1, R1^{I}, R1^{F}) and the second representation (R2, R2^{I}, R2^{F}) to a first model (M1), wherein the first model (M1) is configured to generate, on the basis of the first representation (R1, R1^{I}, R1^{F}) and the second representation (R2, R2^{I}, R2^{F}), a third representation (R3, R3^{I}, R3^{F}), wherein the third representation (R3, R3^{I}, R3^{F}) represents the examination region of the examination object after administration of a third amount of the contrast agent, wherein the third amount is different from the first amount and the second amount,
wherein generating the third representation (R3, R3^{I}, R3^{F}) by the first model (M1) comprises:
∘ subtracting the first representation (R1, R1^{I}, R1^{F}) from the second representation (R2, R2^{I}, R2^{F}),
∘ multiplying the result of the subtracting by a gain factor α,
∘ adding the result of the multiplying to the first representation (R1, R1^{I}, R1^{F}),
- feeding the third representation (R3, R3^{I}, R3^{F}) to a second model (M2),
∘ wherein the second model (M2) has been trained in a training method on the basis of training data (TD),
∘ wherein the training data (TD) comprise, for each reference object of a plurality of reference objects,
(i) a reference representation (RR3) generated by the first model, which represents a reference region of the reference object after administration of a reference amount of the contrast agent, and
(ii) a measured reference representation (RR3^{M}) of the reference region of the reference object after administration of the reference amount of the contrast agent
∘ wherein the training method comprises, for each reference object:
▪ feeding the reference representation (RR3) generated by the first model (M1) to the second model (M2), wherein the second model (M2) generates, on the basis of the reference representation (RR3) generated by the first model (M1) and on the basis of model parameters (MP), a corrected reference representation (RR3^{C}),
▪ receiving the corrected reference representation (RR3^{C}) from the second model (M2),
▪ reducing deviations between the corrected reference representation (RR3^{C}) and the measured reference representation (RR3^{M}) by modifying the model parameters (MP),
- receiving a corrected third representation (R3^{C}) of the examination region of the examination object from the second model (M2),
- outputting and/or storing the corrected third representation (R3^{C}) and/or transmitting the corrected third representation (R3^{C}) to a separate computer system.

2. The method according to claim 1, wherein generating the third representation (R3, R3^{I}, R3^{F}) by the first model (M1) comprises:
- multiplying a frequency-space representation (R2^{F}-R1^{F}) of a difference of the first representation (R1, R1^{I}, R1^{F}) from the second representation (R2, R2^{I}, R2^{F}) by a frequency-dependent weighting function (WF), thereby obtaining a weighted representation (R2^{F}-R1^{F})^{W},
- multiplying the weighted representation (R2^{F}-R1^{F})^{W} by a gain factor α,
- adding, after multiplication by the gain factor α, the weighted representation (R2^{F}-R1^{F})^{W} to the first representation (R1, R1^{I}, R1^{F}).

3. The method according to claim 2, wherein the frequency-dependent weighting function (WF) is a Hann window function or a Poisson window function.

4. The method according to any one of claims 1 to 3, wherein the gain factor α is greater than 1, preferably greater than 2.

5. The method according to any one of claims 1 to 3, wherein the gain factor α is greater than zero and less than 1.

6. The method according to any one of claims 1 to 3, wherein the gain factor α is less than zero.

7. The method according to any one of claims 1 to 6, wherein the third amount and the reference amount are greater than a standard amount of the contrast agent.

8. The method according to any one of claims 1 to 7, wherein the second model (M2) is an artificial neural network, wherein the first model (M1) comprises one or more processing layers (L4, L5) which precede the second model (M2).

9. The method according to claim 8, wherein one or more further processing layers (L1, L2, L3) precede the second model (M2), wherein the one or more further processing layers form an initial model (M0), wherein the initial model (M0) is configured to determine the gain factor α and/or parameters of the frequency-dependent weighting function (WF) on the basis of the first representation (R1, R1^{I}, R1^{F}) and/or the second representation (R2, R2^{I}, R2^{F}) and/or to perform a co-registration of the first representation (R1, R1^{I}, R1^{F}) and the second representation (R2, R2^{I}, R2^{F}).

10. The method according to any one of claims 1 to 9, wherein the examination object and each reference object is a human or an animal, preferably a mammal,
wherein the examination region is a part of the examination object and the reference region is a part of the reference object, wherein the reference region corresponds to the examination region.

11. The method according to any one of claims 1 to 10, wherein the first representation (R1, R1^{I}, R1^{F}) and the second representation (R2, R2^{I}, R2^{F}) are the result of a radiological examination, preferably an MRI examination and/or a CT examination.

12. The method according to any one of claims 1 to 11, wherein the contrast agent comprises
- a Gd³⁺ complex of a compound of formula (I) 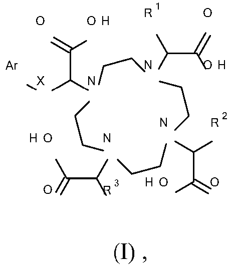 wherein
Ar represents a group selected from 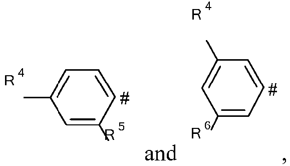
wherein # represents the point of attachment to X,
X represents a group selected from
CH₂, (CH₂)₂, (CH₂)₃, (CH₂)₄ and *-(CH₂)₂-O-CH₂-^{#} ,
wherein * represents the point of attachment to Ar and ^{#} represents the point of attachment to the acetic acid residue,
R¹, R² and R³ independently of one another represent a hydrogen atom or a group selected from C₁-C₃-alkyl, -CH₂OH, -(CH₂)₂OH and -CH₂OCH₃,
R⁴ represents a group selected from C₂-C₄-alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ represents a hydrogen atom,
and
R⁶ represents a hydrogen atom,
or a stereoisomer, tautomer, hydrate, solvate or salt thereof, or a mixture thereof, or
- a Gd³⁺ complex of a compound of formula (II) 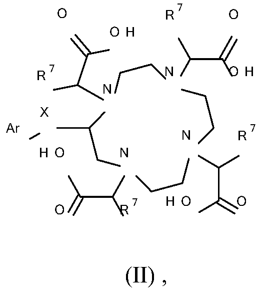 wherein
Ar represents a group selected from 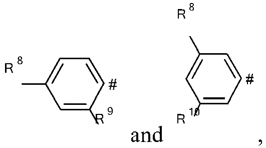
wherein # represents the point of attachment to X,
X represents a group selected from CH₂, (CH₂)₂, (CH₂)₃, (CH₂)₄ and *-(CH₂)₂-O-CH₂-^{#}, wherein * represents the point of attachment to Ar and ^{#} represents the point of attachment to the acetic acid residue,
R⁷ represents a hydrogen atom or a group selected from C₁-C₃-alkyl, -CH₂OH, -(CH₂)₂OH and -CH₂OCH₃;
R⁸ represents a group selected from C₂-C₄-alkoxy, (H₃C-CH₂O)-(CH₂)₂-O-, (H₃C-CH₂O)-(CH₂)₂-O-(CH₂)₂-O- and (H₃C-CH₂O)-(CH₂)₂-O-(CH₂)₂-O-(CH₂)₂-O-;
R⁹ and R¹⁰ independently of one another represent a hydrogen atom;
or a stereoisomer, tautomer, hydrate, solvate or salt thereof, or a mixture thereof, or
the contrast agent comprises one of the following substances:
- gadolinium(III) 2-[4,7,10-tris(carboxymethyl)-1,4,7,10-tetraazacyclododecan-1-yl]acetic acid,
- gadolinium(III) ethoxybenzyl diethylenetriaminepentaacetic acid,
- gadolinium(III) 2-[3,9-bis[1-carboxylato-4-(2,3-dihydroxypropylamino)-4-oxobutyl]-3,6,9,15-tetraazabicyclo[9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)]gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]amino}methyl)-4,7,11,14-tetraazaheptadecan-2-yl}-1,4,7,10-tetraazacyclododecan-1-yl]acetate,
- 2,2',2"-(10-{1-carboxy-2-[2-(4-ethoxyphenyl)ethoxy]ethyl}-1,4,7,10-tetraazacyclododecane-1,4,7-triyl)triacetate,
- gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl } triacetate,
- gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl } triacetate,
- gadolinium (2S,2'S,2"S)-2,2',2"- {10-[(1 S)-1-carboxy-4- {4-[2-(2-ethoxyethoxy)ethoxy]phenyl}butyl]-1,4,7,10-tetraazacyclododecane-1,4,7-triyl}tris(3-hydroxypropanoate),
- gadolinium 2,2',2"-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate,
- gadolinium(III) 2-[4-(2-hydroxypropyl)-7,10-bis(2-oxido-2-oxoethyl)-1,4,7,10-tetraazacyclododecan-1-yl]acetate,
- gadolinium(III) 2,2',2"-[10-((2R,3S)-1,3,4-trihydroxybutan-2-yl)-1,4,7,10-tetraazacyclododecane-1,4,7-triyl]triacetate,
- gadolinium 2,2',2"-{(2*S*)-10-(carboxymethyl)-2-[4-(2-ethoxyethoxy)benzyl]-1,4,7,10-tetraazacyclododecane-1,4,7-triyl } triacetate,
- gadolinium 2,2',2"-[10-(carboxymethyl)-2-(4-ethoxybenzyl)-1,4,7,10-tetraazacyclododecane-1,4,7-triyl]triacetate.

13. A computer system (1) comprising
• a receiving unit (11),
• a control and computing unit (12), and
• an output unit (13),
wherein the control and computing unit (12) is configured
- to generate a first representation (R1, R1^{I}, R1^{F}) or to cause the receiving unit (11) to receive the first representation (R1, R1^{I}, R1^{F}), wherein the first representation (R1, R1^{I}, R1^{F}) represents an examination region of an examination object without contrast agent or after administration of a first amount of a contrast agent,
- to generate a second representation (R2, R2^{I}, R2^{F}) or to cause the receiving unit (11) to receive the second representation (R2, R2^{I}, R2^{F}), wherein the second representation (R2, R2^{I}, R2^{F}) represents the examination region of the examination object after administration of a second amount of the contrast agent, wherein the second amount is greater than the first amount,
- to feed the first representation (R1, R1^{I}, R1^{F}) and the second representation (R2, R2^{I}, R2^{F}) to a first model (M1), wherein the first model (M1) is configured to generate, on the basis of the first representation (R1, R1^{I}, R1^{F}) and the second representation (R2, R2^{I}, R2^{F}), a third representation (R3, R3^{I}, R3^{F}), wherein the third representation (R3, R3^{I}, R3^{F}) represents the examination region of the examination object after administration of a third amount of the contrast agent, wherein the third amount is different from the first amount and the second amount,
wherein generating the third representation (R3, R3^{I}, R3^{F}) by the first model (M1) comprises:
∘ subtracting the first representation (R1, R1^{I}, R1^{F}) from the second representation (R2, R2^{I}, R2^{F}),
∘ multiplying the result of the subtracting by a gain factor α,
∘ adding the result of the multiplying to the first representation (R1, R1^{I}, R1^{F}),
- to feed the third representation (R3, R3^{I}, R3^{F}) to a second model (M2),
∘ wherein the second model (M2) has been trained in a training method on the basis of training data (TD),
∘ wherein the training data (TD) comprise, for each reference object of a plurality of reference objects,
(i) a reference representation (RR3) generated by the first model (M1), which represents a reference region of the reference object after administration of a reference amount of the contrast agent, and
(ii) a measured reference representation (RR3^{M}) of the reference region of the reference object after administration of the reference amount of the contrast agent
∘ wherein the training method comprises, for each reference object:
▪ feeding the reference representation (RR3) generated by the first model (M1) to the second model (M2), wherein the second model (M2) generates, on the basis of the reference representation (RR3) generated by the first model (M1) and on the basis of model parameters (MP), a corrected reference representation (RR3^{C}),
▪ receiving the corrected reference representation (RR3^{C}) from the second model (M2),
▪ reducing deviations between the corrected reference representation (RR3^{C}) and the measured reference representation (RR3^{M}) by modifying the model parameters (MP),
- to receive a corrected third representation (R3^{C}) of the examination region of the examination object from the second model (M2),
- to cause the output unit (13) to output, store and/or transmit the corrected third representation (R3^{C}) to a separate computer system.

14. A computer program product comprising a data carrier on which a computer program (40) is stored, wherein the computer program (40) can be loaded into a main memory (22) of a computer system (1) and causes the computer system (1) there to execute the following steps:
- providing a first representation (R1, R1^{I}, R1^{F}), wherein the first representation (R1, R1^{I}, R1^{F}) represents an examination region of an examination object without contrast agent or after administration of a first amount of a contrast agent,
- providing a second representation (R2, R2^{I}, R2^{F}), wherein the second representation (R2, R2^{I}, R2^{F}) represents the examination region of the examination object after administration of a second amount of the contrast agent, wherein the second amount is greater than the first amount,
- feeding the first representation (R1, R1^{I}, R1^{F}) and the second representation (R2, R2^{I}, R2^{F}) to a first model (M1), wherein the first model (M1) is configured to generate, on the basis of the first representation (R1, R1^{I}, R1^{F}) and the second representation (R2, R2^{I}, R2^{F}), a third representation (R3, R3^{I}, R3^{F}), wherein the third representation (R3, R3^{I}, R3^{F}) represents the examination region of the examination object after administration of a third amount of the contrast agent, wherein the third amount is different from the first amount and the second amount,
wherein generating the third representation (R3, R3^{I}, R3^{F}) by the first model (M1) comprises:
∘ subtracting the first representation (R1, R1^{I}, R1^{F}) from the second representation (R2, R2^{I}, R2^{F}),
∘ multiplying the result of the subtracting by a gain factor α,
∘ adding the result of the multiplying to the first representation (R1, R1^{I}, R1^{F}),
- feeding the third representation (R3, R3^{I}, R3^{F}) to a second model (M2),
∘ wherein the second model (M2) has been trained in a training method on the basis of training data (TD),
∘ wherein the training data (TD) comprise, for each reference object of a plurality of reference objects,
(i) a reference representation (RR3) generated by the first model, which represents a reference region of the reference object after administration of a reference amount of the contrast agent, and
(ii) a measured reference representation (RR3^{M}) of the reference region of the reference object after administration of the reference amount of the contrast agent
∘ wherein the training method comprises, for each reference object:
▪ feeding the reference representation (RR3) generated by the first model (M1) to the second model (M2), wherein the second model (M2) generates, on the basis of the reference representation (RR3) generated by the first model (M1) and on the basis of model parameters (MP), a corrected reference representation (RR3^{C}),
▪ receiving the corrected reference representation (RR3^{C}) from the second model (M2),
▪ reducing deviations between the corrected reference representation (RR3^{C}) and the measured reference representation (RR3^{M}) by modifying the model parameters (MP),
- receiving a corrected third representation (R3^{C}) of the examination region of the examination object from the second model (M2),
- outputting and/or storing the corrected third representation (R3^{C}) and/or transmitting the corrected third representation (R3^{C}) to a separate computer system.

15. Use of a contrast agent in a radiological examination method, wherein the radiological examination method comprises the computer-implemented method according to claim 1.

16. The use according to claim 15, wherein the radiological examination method is a magnetic resonance imaging examination or a computed tomography examination, and wherein the contrast agent comprises
- a Gd³⁺ complex of a compound of formula (I) 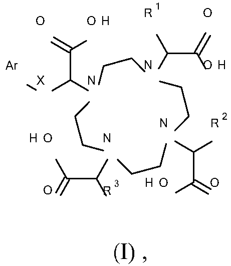 wherein
Ar represents a group selected from 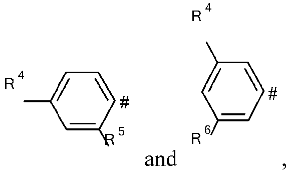
wherein _{#} represents the point of attachment to X,
X represents a group selected from
CH₂, (CH₂)₂, (CH₂)₃, (CH₂)₄ and *-(CH₂)₂-O-CH₂-^{#} ,
wherein * represents the point of attachment to Ar and ^{#} represents the point of attachment to the acetic acid residue,
R¹, R² and R³ independently of one another represent a hydrogen atom or a group selected from C₁-C₃-alkyl, -CH₂OH, -(CH₂)₂OH and -CH₂OCH₃,
R⁴ represents a group selected from C₂-C₄-alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ represents a hydrogen atom,
and
R⁶ represents a hydrogen atom,
or a stereoisomer, tautomer, hydrate, solvate or salt thereof, or a mixture thereof, or
- a Gd³⁺ complex of a compound of formula (II) 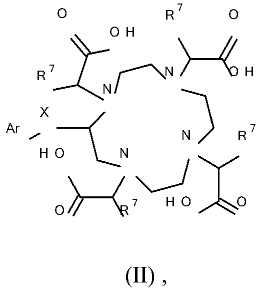 wherein
Ar represents a group selected from 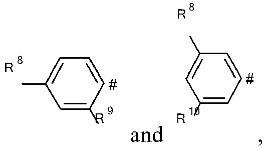
wherein _{#} represents the point of attachment to X,
X represents a group selected from CH₂, (CH₂)₂, (CH₂)₃, (CH₂)₄ and *-(CH₂)₂-O-CH₂-^{#}, wherein * represents the point of attachment to Ar and ^{#} represents the point of attachment to the acetic acid residue,
R⁷ represents a hydrogen atom or a group selected from C₁-C₃-alkyl, -CH₂OH, -(CH₂)₂OH and -CH₂OCH₃;
R⁸ represents a group selected from C₂-C₄-alkoxy, (H₃C-CH₂O)-(CH₂)₂-O-, (H₃C-CH₂O)-(CH₂)₂-O-(CH₂)₂-O- and (H₃C-CH₂O)-(CH₂)₂-O-(CH₂)₂-O-(CH₂)₂-O-;
R⁹ and R¹⁰ independently of one another represent a hydrogen atom;
or a stereoisomer, tautomer, hydrate, solvate or salt thereof, or a mixture thereof, or
- the contrast agent comprises one of the following substances:
- gadolinium(III) 2-[4,7,10-tris(carboxymethyl)-1,4,7,10-tetraazacyclododecan-1-yl]acetic acid,
- gadolinium(III) ethoxybenzyl diethylenetriaminepentaacetic acid,
- gadolinium(III) 2-[3,9-bis[1-carboxylato-4-(2,3-dihydroxypropylamino)-4-oxobutyl]-3,6,9,15-tetraazabicyclo[9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)]gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]amino}methyl)-4,7,11,14-tetraazaheptadecan-2-yl}-1,4,7,10-tetraazacyclododecan-1-yl]acetate,
- 2,2',2"-(10-{1-carboxy-2-[2-(4-ethoxyphenyl)ethoxy]ethyl1,4,7,10-tetraazacyclododecane-1,4,7-triyl)triacetate,
- gadolinium 2,2',2"-{10-[l-carboxy-2-{4-[2-(2-ethoxyethoxy)ethoxy]phenyl}ethyl]-1,4,7,10-tetraazacyclododecane-1,4,7-triyl } triacetate,
- gadolinium 2,2',2"-{10-[(1R)-1-carboxy-2-{4-[2-(2-ethoxyethoxy)ethoxy]phenyl}ethyl]-1,4,7,10-tetraazacyclododecane- 1,4,7-triyl } triacetate,
- gadolinium (2S,2'S,2"S)-2,2',2"- {10-[(1 S)-1-carboxy-4- {4-[2-(2-ethoxyethoxy)ethoxy]phenyl}butyl]-1,4,7,10-tetraazacyclododecane-1,4,7-triyl}tris(3-hydroxypropanoate),
- gadolinium 2,2',2"-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate,
- gadolinium(III) 2-[4-(2-hydroxypropyl)-7,10-bis(2-oxido-2-oxoethyl)-1,4,7,10-tetraazacyclododecan-1-yl]acetate,
- gadolinium(III) 2,2',2"-[10-((2R,3S)-1,3,4-trihydroxybutan-2-yl)-1,4,7,10-tetraazacyclododecane-1,4,7-triyl]triacetate,
- gadolinium 2,2',2"-{(2S)-10-(carboxymethyl)-2-[4-(2-ethoxyethoxy)benzyl]-1,4,7,10-tetraazacyclododecane-1,4,7-triyl}triacetate,
- gadolinium 2,2',2"-[10-(carboxymethyl)-2-(4-ethoxybenzyl)-1,4,7,10-tetraazacyclododecane-1,4,7-triyl]triacetate.

17. A kit comprising a computer program product according to claim 14 and a contrast agent, wherein the contrast agent preferably comprises
- a Gd³⁺ complex of a compound of formula (I) 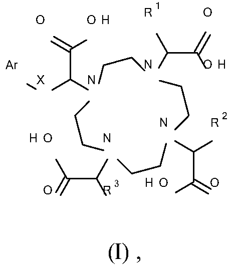 wherein
Ar represents a group selected from 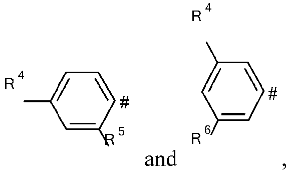
wherein _{#} represents the point of attachment to X,
X represents a group selected from
CH₂, (CH₂)₂, (CH₂)₃, (CH₂)₄ and *-(CH₂)₂-O-CH₂-^{#} ,
wherein * represents the point of attachment to Ar and ^{#} represents the point of attachment to the acetic acid residue,
R¹, R² and R³ independently of one another represent a hydrogen atom or a group selected from C₁-C₃-alkyl, -CH₂OH, -(CH₂)₂OH and -CH₂OCH₃,
R⁴ represents a group selected from C₂-C₄-alkoxy, (H₃C-CH₂)-O-(CH₂)₂-O-, (H₃C-CH₂)-O-(CH₂)₂-O-(CH₂)₂-O- and (H₃C-CH₂)-O-(CH₂)₂-O-(CH₂)₂-O-(CH₂)₂-O-,
R⁵ represents a hydrogen atom,
and
R⁶ represents a hydrogen atom,
or a stereoisomer, tautomer, hydrate, solvate or salt thereof, or a mixture thereof, or
- a Gd³⁺ complex of a compound of formula (II) 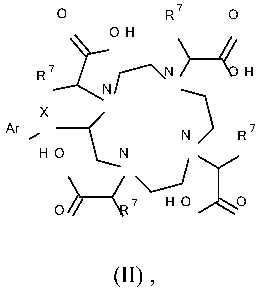 wherein
Ar represents a group selected from 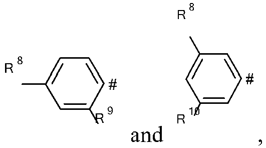
wherein _{#} represents the point of attachment to X,
X represents a group selected from CH₂, (CH₂)₂, (CH₂)₃, (CH₂)₄ and *-(CH₂)₂-O-CH₂-^{#}, wherein * represents the point of attachment to Ar and ^{#} represents the point of attachment to the acetic acid residue,
R⁷ represents a hydrogen atom or a group selected from C₁-C₃-alkyl, -CH₂OH, -(CH₂)₂OH and -CH₂OCH₃;
R⁸ represents a group selected from C₂-C₄-alkoxy, (H₃C-CH₂O)-(CH₂)₂-O-, (H₃C-CH₂0)-(CH₂)₂-O-(CH₂)₂-O- and (H₃C-CH₂O)-(CH₂)₂-O-(CH₂)₂-O-(CH₂)₂-O-;
R⁹ and R¹⁰ independently of one another represent a hydrogen atom;
or a stereoisomer, tautomer, hydrate, solvate or salt thereof, or a mixture thereof, or
- the contrast agent comprises one of the following substances:
- gadolinium(III) 2-[4,7,10-tris(carboxymethyl)-1,4,7,10-tetraazacyclododecan-1-yl]acetic acid,
- gadolinium(III) ethoxybenzyl diethylenetriaminepentaacetic acid,
- gadolinium(III) 2-[3,9-bis[1-carboxylato-4-(2,3-dihydroxypropylamino)-4-oxobutyl]-3,6,9,15-tetraazabicyclo[9.3.1]pentadeca-1(15),11,13-trien-6-yl]-5-(2,3-dihydroxypropylamino)-5-oxopentanoate,
- dihydrogen [(±)-4-carboxy-5,8,11-tris(carboxymethyl)-1-phenyl-2-oxa-5,8,11-triazatridecan-13-oato(5-)]gadolinate(2-),
- tetragadolinium [4,10-bis(carboxylatomethyl)-7-{3,6,12,15-tetraoxo-16-[4,7,10-tris(carboxylatomethyl)-1,4,7,10-tetraazacyclododecan-1-yl]-9,9-bis({[({2-[4,7,10-tris(carboxylatomethyl)-1,4,7,10-tetraazacyclododecan-1-yl]propanoyl}amino)acetyl]amino}methyl)-4,7,11,14-tetraazaheptadecan-2-yl}-1,4,7,10-tetraazacyclododecan-1-yl]acetate,
- 2,2',2"-(10-{1-carboxy-2-[2-(4-ethoxyphenyl)ethoxy]ethyl}-1,4,7,10-tetraazacyclododecane-1,4,7-triyl)triacetate,
- gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl } triacetate,
- gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl } triacetate,
- gadolinium (2S,2'S,2"S)-2,2',2"-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate),
- gadolinium 2,2',2"-{10-[(1S)-4-(4-butoxyphenyl)-1-carboxybutyl]-1,4,7,10-tetraazacyclododecane-1,4,7-triyl}triacetate,
- gadolinium(III) 5,8-bis(carboxylatomethyl)-2-[2-(methylamino)-2-oxoethyl]-10-oxo-2,5,8,11-tetraazadodecane-1-carboxylate hydrate,
- gadolinium(III) 2-[4-(2-hydroxypropyl)-7,10-bis(2-oxido-2-oxoethyl)-1,4,7,10-tetraazacyclododecan-1-yl]acetate,
- gadolinium(III) 2,2',2"-[10-((2R,3S)-1,3,4-trihydroxybutan-2-yl)-1,4,7,10-tetraazacyclododecane-1,4,7-triyl]triacetate,
- gadolinium 2,2',2"-{(2S)-10-(carboxymethyl)-2-[4-(2-ethoxyethoxy)benzyl]-1,4,7,10-tetraazacyclododecane-1,4,7-triyl}triacetate,
- gadolinium 2,2',2"-[10-(carboxymethyl)-2-(4-ethoxybenzyl)-1,4,7,10-tetraazacyclododecane-1,4,7-triyl]triacetate.

## Revendications

1. Procédé mis en œuvre par ordinateur, comprenant :
- la fourniture d'une première représentation (R1, R1^{I}, R1^{F}), la première représentation (R1, R1^{I}, R1^{F}) représentant une région d'examen d'un objet d'examen sans agent de contraste ou après administration d'une première quantité d'un agent de contraste,
- la fourniture d'une deuxième représentation (R2, R2^{I}, R2^{F}), la deuxième représentation (R2, R2^{I}, R2^{F}) représentant la région d'examen de l'objet d'examen après administration d'une deuxième quantité de l'agent de contraste, la deuxième quantité étant supérieure à la première quantité,
- l'introduction de la première représentation (R1, R1^{I}, R1^{F}) et de la deuxième représentation (R2, R2^{I}, R2^{F}) dans un premier modèle (M1), le premier modèle (M1) étant configuré pour générer, sur la base de la première représentation (R1, R1^{I}, R1^{F}) et de la deuxième représentation (R2, R2^{I}, R2^{F}), une troisième représentation (R3, R3^{I}, R3^{F}), la troisième représentation (R3, R3^{I}, R3^{F}) représentant la région d'examen de l'objet d'examen après administration d'une troisième quantité de l'agent de contraste, la troisième quantité étant différente de la première quantité et de la deuxième quantité,
la génération de la troisième représentation (R3, R3^{I}, R3^{F}) par le premier modèle (M1) comprenant :
∘ la soustraction de la première représentation (R1, R1^{I}, R1^{F}) de la deuxième représentation (R2, R2^{I}, R2^{F}),
∘ la multiplication du résultat de la soustraction par un facteur d'amplification α,
∘ l'addition du résultat de la multiplication à la première représentation (R1, R1^{I}, R1^{F}),
- l'introduction de la troisième représentation (R3, R3^{I}, R3^{F}) dans un deuxième modèle (M2),
∘ le deuxième modèle (M2) ayant été entraîné dans un procédé d'entraînement sur la base de données d'entraînement (TD),
∘ les données d'entraînement (TD) comprenant, pour chaque objet de référence d'une pluralité d'objets de référence,
(i) une représentation de référence (RR3) générée par le premier modèle, qui représente une région de référence de l'objet de référence après administration d'une quantité de référence de l'agent de contraste, et
(ii) une représentation de référence mesurée (RR3^{M}) de la région de référence de l'objet de référence après administration de la quantité de référence de l'agent de contraste,
∘ le procédé d'entraînement comprenant, pour chaque objet de référence :
▪ l'introduction de la représentation de référence (RR3) générée par le premier modèle (M1) dans le deuxième modèle (M2), le deuxième modèle (M2) générant, sur la base de la représentation de référence (RR3) générée par le premier modèle (M1) et sur la base de paramètres de modèle (MP), une représentation de référence corrigée (RR3^{C}),
▪ la réception de la représentation de référence corrigée (RR3^{C}) en provenance du deuxième modèle (M2),
▪ la réduction des écarts entre la représentation de référence corrigée (RR3^{C}) et la représentation de référence mesurée (RR3^{M}) par modification des paramètres de modèle (MP),
- la réception d'une troisième représentation corrigée (R3^{C}) de la région d'examen de l'objet d'examen en provenance du deuxième modèle (M2),
- l'émission et/ou le stockage de la troisième représentation corrigée (R3^{C}) et/ou la transmission de la troisième représentation corrigée (R3^{C}) à un système informatique séparé.

2. Procédé selon la revendication 1, dans lequel la génération de la troisième représentation (R3, R3^{I}, R3^{F}) par le premier modèle (M1) comprenant :
- la multiplication d'une représentation dans l'espace fréquentiel (R2^{F}-R1^{F}) d'une différence entre la première représentation (R1, R1^{I}, R1^{F}) de la deuxième représentation (R2, R2^{I}, R2^{F}) par une fonction de pondération dépendant de la fréquence (WF), permettant d'obtenir une représentation pondérée (R2^{F}- R1^{F})^{W},
- la multiplication de la représentation pondérée (R2^{F}-R1^{F})^{W} par un facteur d'amplification α,
- l'addition, après multiplication par le facteur d'amplification α, de la représentation pondérée (R2^{F}-R1^{F})^{W} à la première représentation (R1, R1^{I}, R1^{F}).

3. Procédé selon la revendication 2, dans lequel la fonction de pondération dépendant de la fréquence (WF) est une fonction de fenêtre de Hann ou une fonction de fenêtre de Poisson.

4. Procédé selon l'une quelconque des revendications 1 à 3, dans lequel le facteur d'amplification α est supérieur à 1, de préférence supérieur à 2.

5. Procédé selon l'une quelconque des revendications 1 à 3, dans lequel le facteur d'amplification α est supérieur à zéro et inférieur à 1.

6. Procédé selon l'une quelconque des revendications 1 à 3, dans lequel le facteur d'amplification α est inférieur à zéro.

7. Procédé selon l'une quelconque des revendications 1 à 6, dans lequel la troisième quantité et la quantité de référence sont supérieures à une quantité standard de l'agent de contraste.

8. Procédé selon l'une quelconque des revendications 1 à 7, dans lequel le deuxième modèle (M2) est un réseau neuronal artificiel, le premier modèle (M1) comprenant une ou plusieurs couches de traitement (L4, L5) qui précèdent le deuxième modèle (M2).

9. Procédé selon la revendication 8, dans lequel une ou plusieurs couches de traitement supplémentaires (L1, L2, L3) précèdent le deuxième modèle (M2), la ou les couches de traitement supplémentaires formant un modèle initial (M0), le modèle initial (M0) étant configuré pour déterminer le facteur d'amplification α et/ou des paramètres de la fonction de pondération dépendant de la fréquence (WF) sur la base de la première représentation (R1, R1^{I}, R1^{F}) et/ou de la deuxième représentation (R2, R2^{I}, R2^{F}) et/ou pour effectuer un co-recalage de la première représentation (R1, R1^{I}, R1^{F}) et de la deuxième représentation (R2, R2^{I}, R2^{F}).

10. Procédé selon l'une quelconque des revendications 1 à 9, dans lequel l'objet d'examen et chaque objet de référence sont un être humain ou un animal, de préférence un mammifère,
la région d'examen étant une partie de l'objet d'examen et la région de référence étant une partie de l'objet de référence, la région de référence correspondant à la région d'examen.

11. Procédé selon l'une quelconque des revendications 1 à 10, dans lequel la première représentation (R1, R1^{I}, R1^{F}) et la deuxième représentation (R2, R2^{I}, R2^{F}) sont le résultat d'un examen radiologique, de préférence d'un examen IRM et/ou d'un examen TDM.

12. Procédé selon l'une quelconque des revendications 1 à 11, dans lequel l'agent de contraste comprend
- un complexe de Gd³⁺ d'un composé de formule (I) 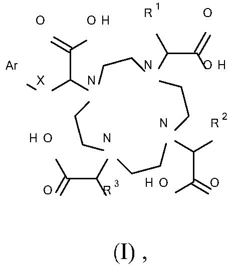 dans laquelle
Ar représente un groupe choisi parmi 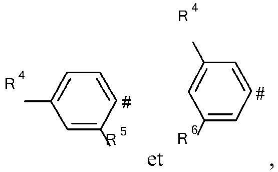
où # représente le point de rattachement à X,
X représente un groupe choisi parmi
CH₂, (CH₂)₂, (CH₂)₃, (CH₂)₄ et *-(CH₂)₂-O-CH₂-^{#},
où * représente le point de rattachement à Ar et ^{#} représente le point de rattachement au reste acide acétique,
R¹, R² et R³ représentent, indépendamment les uns des autres, un atome d'hydrogène ou un groupe choisi parmi C₁-C₃-alkyle, -CH₂OH, -(CH₂)₂OH et -CH₂OCH₃,
R⁴ représente un groupe choisi parmi C₂-C₄-alcoxy, (H₃C-CH₂)-O-(CH₂)₂-O-, (H₃C-CH₂)-O-(CH₂)₂-O-(CH₂)₂-O- et (H₃C-CH₂)-O-(CH₂)₂-O-(CH₂)₂-O-(CH₂)₂-O-,
R⁵ représente un atome d'hydrogène,
et
R⁶ représente un atome d'hydrogène,
ou un stéréoisomère, un tautomère, un hydrate, un solvate ou un sel de celui-ci, ou un mélange de ceux-ci, ou
- un complexe de Gd³⁺ d'un composé de formule (II) 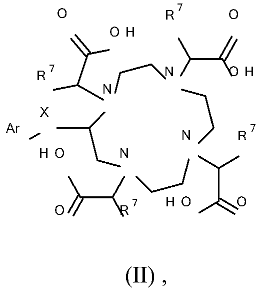 dans laquelle
Ar représente un groupe choisi parmi 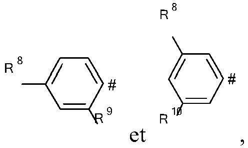
où # représente le point de rattachement à X,
X représente un groupe choisi parmi CH₂, (CH₂)₂, (CH₂)₃, (CH₂)₄ et *-(CH₂)₂-O-CH₂-^{#}, où * représente le point de rattachement à Ar et ^{#} représente le point de rattachement au reste acide acétique,
R⁷ représente un atome d'hydrogène ou un groupe choisi parmi C₁-C₃-alkyle, -CH₂OH, -(CH₂)₂OH et -CH₂OCH₃ ;
R⁸ représente un groupe choisi parmi C₂-C₄-alcoxy, (H₃C-CH₂O)-(CH₂)₂-O-, (H₃C-CH₂O)-(CH₂)₂-O-(CH₂)₂-O- et (H₃C-CH₂O)-(CH₂)₂-O-(CH₂)₂-O-(CH₂)₂-O- ;
R⁹ et R¹⁰ représentent, indépendamment l'un de l'autre, un atome d'hydrogène ;
ou un stéréoisomère, un tautomère, un hydrate, un solvate ou un sel de celui-ci, ou un mélange de ceux-ci, ou
l'agent de contraste comprend l'une des substances suivantes :
- acide 2-[4,7,10-tris(carboxyméthyl)-1,4,7,10-tétraazacyclododécan-1-yl]acétique de gadolinium(III),
- acide éthoxybenzyl-diéthylènetriaminepentaacétique de gadolinium(III),
- 2-[3,9-bis[1-carboxylato-4-(2,3-dihydroxypropylamino)-4-oxobutyl]-3,6,9,15-tétraazabicyclo[9.3.1]pentadéca-1(15),11,13-trién-6-yl]-5-(2,3-dihydroxypropylamino)-5-oxopentanoate de gadolinium(III),
- [(±)-4-carboxy-5,8,11-tris(carboxyméthyl)-1-phényl-2-oxa-5,8,11-triazatridécan-13-oato(5-)]gadolinate(2-) de dihydrogène,
- [4,10-bis(carboxylatométhyl)-7-{3,6,12,15-tétraoxo-16-[4,7,10-tris(carboxylatométhyl)-1,4,7,10-tétraazacyclododécan-1-yl]-9,9-bis({[({2-[4,7,10-tris(carboxylatométhyl)-1,4,7,10-tétraazacyclododécan-1-yl]propanoyl}amino)acétyl]amino}méthyl)-4,7,11,14-tétraazaheptadécan-2-yl}-1,4,7,10-tétraazacyclododécan-1-yl]acétate de tétragadolinium,
- 2,2',2"-(10-{1-carboxy-2-[2-(4-éthoxyphényl)éthoxy]éthyl}-1,4,7,10-tétraazacyclododécane-1,4,7-triyl)triacétate,
- 2,2',2"-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
- 2,2',2"-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,
- (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
- 2,2',2"-{10-[(1S)-4-(4-butoxyphényl)-1-carboxybutyl]-1,4,7,10-tétraazacyclododécane-1,4,7-triyl}triacétate de gadolinium,
- 5,8-bis(carboxylatométhyl)-2-[2-(méthylamino)-2-oxoéthyl]-10-oxo-2,5,8,11-tétraazadodécane-1-carboxylate de gadolinium(III) hydraté,
- 2-[4-(2-hydroxypropyl)-7,10-bis(2-oxido-2-oxoéthyl)-1,4,7,10-tétraazacyclododécan-1-yl]acétate de gadolinium(III),
- 2,2',2"-[10-((2R,3S)-1,3,4-trihydroxybutan-2-yl)-1,4,7,10-tétraazacyclododécane-1,4,7-triyl]triacétate de gadolinium(III),
- 2,2',2"-{(2*S*)-10-(carboxyméthyl)-2-[4-(2-éthoxyéthoxy)benzyl]-1,4,7,10-tétraazacyclododécane-1,4,7-triyl}triacétate de gadolinium,
- 2,2',2"-[10-(carboxyméthyl)-2-(4-éthoxybenzyl)-1,4,7,10-tétraazacyclododécane-1,4,7-triyl]triacétate de gadolinium.

13. Système informatique (1) comprenant
• une unité de réception (11),
• une unité de commande et de calcul (12), et
• une unité de sortie (13),
l'unité de commande et de calcul (12) étant configurée
- pour générer une première représentation (R1, R1^{I}, R1^{F}) ou pour amener l'unité de réception (11) à recevoir la première représentation (R1, R1^{I}, R1^{F}), la première représentation (R1, R1^{I}, R1^{F}) représentant une région d'examen d'un objet d'examen sans agent de contraste ou après administration d'une première quantité d'un agent de contraste,
- pour générer une deuxième représentation (R2, R2^{I}, R2^{F}) ou pour amener l'unité de réception (11) à recevoir la deuxième représentation (R2, R2^{I}, R2^{F}), la deuxième représentation (R2, R2^{I}, R2^{F}) représentant la région d'examen de l'objet d'examen après administration d'une deuxième quantité de l'agent de contraste, la deuxième quantité étant supérieure à la première quantité,
- pour introduire la première représentation (R1, R1^{I}, R1^{F}) et la deuxième représentation (R2, R2^{I}, R2^{F}) dans un premier modèle (M1), le premier modèle (M1) étant configuré pour générer, sur la base de la première représentation (R1, R1^{I}, R1^{F}) et de la deuxième représentation (R2, R2^{I}, R2^{F}), une troisième représentation (R3, R3^{I}, R3^{F}), la troisième représentation (R3, R3^{I}, R3^{F}) représentant la région d'examen de l'objet d'examen après administration d'une troisième quantité de l'agent de contraste, la troisième quantité étant différente de la première quantité et de la deuxième quantité,
la génération de la troisième représentation (R3, R3^{I}, R3^{F}) par le premier modèle (M1) comprenant :
∘ la soustraction de la première représentation (R1, R1^{I}, R1^{F}) de la deuxième représentation (R2, R2^{I}, R2^{F}),
∘ la multiplication du résultat de la soustraction par un facteur d'amplification α,
∘ l'addition du résultat de la multiplication à la première représentation (R1, R1^{I}, R1^{F}),
- pour introduire la troisième représentation (R3, R3^{I}, R3^{F}) dans un deuxième modèle (M2),
∘ le deuxième modèle (M2) ayant été entraîné dans un procédé d'entraînement sur la base de données d'entraînement (TD),
∘ les données d'entraînement (TD) comprenant, pour chaque objet de référence d'une pluralité d'objets de référence,
(i) une représentation de référence (RR3) générée par le premier modèle (M1), qui représente une région de référence de l'objet de référence après administration d'une quantité de référence de l'agent de contraste, et
(ii) une représentation de référence mesurée (RR3^{M}) de la région de référence de l'objet de référence après administration de la quantité de référence de l'agent de contraste,
∘ le procédé d'entraînement comprenant, pour chaque objet de référence :
▪ l'introduction de la représentation de référence (RR3) générée par le premier modèle (M1) dans le deuxième modèle (M2), le deuxième modèle (M2) générant, sur la base de la représentation de référence (RR3) générée par le premier modèle (M1) et sur la base de paramètres de modèle (MP), une représentation de référence corrigée (RR3^{C}),
▪ la réception de la représentation de référence corrigée (RR3^{C}) en provenance du deuxième modèle (M2),
▪ la réduction des écarts entre la représentation de référence corrigée (RR3^{C}) et la représentation de référence mesurée (RR3^{M}) par modification des paramètres de modèle (MP),
- pour recevoir une troisième représentation corrigée (R3^{C}) de la région d'examen de l'objet d'examen en provenance du deuxième modèle (M2),
- pour amener l'unité de sortie (13) à émettre, stocker et/ou transmettre la troisième représentation corrigée (R3^{C}) à un système informatique séparé.

14. Produit programme d'ordinateur comprenant un support de données sur lequel est stocké un programme d'ordinateur (40), le programme d'ordinateur (40) pouvant être chargé dans une mémoire de travail (22) d'un système informatique (1) et y amenant le système informatique (1) à exécuter les étapes suivantes :
- la fourniture d'une première représentation (R1, R1^{I}, R1^{F}), la première représentation (R1, R1^{I}, R1^{F}) représentant une région d'examen d'un objet d'examen sans agent de contraste ou après administration d'une première quantité d'un agent de contraste,
- la fourniture d'une deuxième représentation (R2, R2^{I}, R2^{F}), la deuxième représentation (R2, R2^{I}, R2^{F}) représentant la région d'examen de l'objet d'examen après administration d'une deuxième quantité de l'agent de contraste, la deuxième quantité étant supérieure à la première quantité,
- l'introduction de la première représentation (R1, R1^{I}, R1^{F}) et de la deuxième représentation (R2, R2^{I}, R2^{F}) dans un premier modèle (M1), le premier modèle (M1) étant configuré pour générer, sur la base de la première représentation (R1, R1^{I}, R1^{F}) et de la deuxième représentation (R2, R2^{I}, R2^{F}), une troisième représentation (R3, R3^{I}, R3^{F}), la troisième représentation (R3, R3^{I}, R3^{F}) représentant la région d'examen de l'objet d'examen après administration d'une troisième quantité de l'agent de contraste, la troisième quantité étant différente de la première quantité et de la deuxième quantité,
la génération de la troisième représentation (R3, R3^{I}, R3^{F}) par le premier modèle (M1) comprenant :
∘ la soustraction de la première représentation (R1, R1^{I}, R1^{F}) de la deuxième représentation (R2, R2^{I}, R2^{F}),
∘ la multiplication du résultat de la soustraction par un facteur d'amplification α,
∘ l'addition du résultat de la multiplication à la première représentation (R1, R1^{I}, R1^{F}),
- l'introduction de la troisième représentation (R3, R3^{I}, R3^{F}) dans un deuxième modèle (M2),
∘ le deuxième modèle (M2) ayant été entraîné dans un procédé d'entraînement sur la base de données d'entraînement (TD),
∘ les données d'entraînement (TD) comprenant, pour chaque objet de référence d'une pluralité d'objets de référence,
(i) une représentation de référence (RR3) générée par le premier modèle, qui représente une région de référence de l'objet de référence après administration d'une quantité de référence de l'agent de contraste, et
(ii) une représentation de référence mesurée (RR3^{M}) de la région de référence de l'objet de référence après administration de la quantité de référence de l'agent de contraste,
∘ le procédé d'entraînement comprenant, pour chaque objet de référence :
▪ l'introduction de la représentation de référence (RR3) générée par le premier modèle (M1) dans le deuxième modèle (M2), le deuxième modèle (M2) générant, sur la base de la représentation de référence (RR3) générée par le premier modèle (M1) et sur la base de paramètres de modèle (MP), une représentation de référence corrigée (RR3^{C}),
▪ la réception de la représentation de référence corrigée (RR3^{C}) en provenance du deuxième modèle (M2),
▪ la réduction des écarts entre la représentation de référence corrigée (RR3^{C}) et la représentation de référence mesurée (RR3^{M}) par modification des paramètres de modèle (MP),
- la réception d'une troisième représentation corrigée (R3^{C}) de la région d'examen de l'objet d'examen en provenance du deuxième modèle (M2),
- l'émission et/ou le stockage de la troisième représentation corrigée (R3^{C}) et/ou la transmission de la troisième représentation corrigée (R3^{C}) à un système informatique séparé.

15. Utilisation d'un agent de contraste dans un procédé d'examen radiologique, le procédé d'examen radiologique comprenant le procédé mis en œuvre par ordinateur selon la revendication 1.

16. Utilisation selon la revendication 15, dans laquelle le procédé d'examen radiologique est un examen d'imagerie par résonance magnétique ou un examen de tomodensitométrie, et dans laquelle l'agent de contraste comprend
- un complexe de Gd³⁺ d'un composé de formule (I) 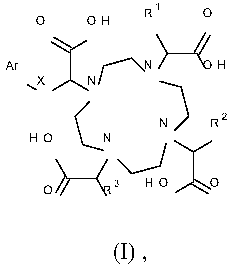 dans laquelle
Ar représente un groupe choisi parmi 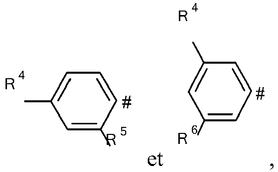
où # représente le point de rattachement à X,
X représente un groupe choisi parmi
CH₂, (CH₂)₂, (CH₂)₃, (CH₂)₄ et *-(CH₂)₂-O-CH₂-^{#} ,
où * représente le point de rattachement à Ar et ^{#} représente le point de rattachement au reste acide acétique,
R¹, R² et R³ représentent, indépendamment les uns des autres, un atome d'hydrogène ou un groupe choisi parmi C₁-C₃-alkyle, -CH₂OH, -(CH₂)₂OH et -CH₂OCH₃,
R⁴ représente un groupe choisi parmi C₂-C₄-alcoxy, (H₃C-CH₂)-O-(CH₂)₂-O-, (H₃C-CH₂)-O-(CH₂)₂-O-(CH₂)₂-O- et (H₃C-CH₂)-O-(CH₂)₂-O-(CH₂)₂-O-(CH₂)₂-O-,
R⁵ représente un atome d'hydrogène,
et
R⁶ représente un atome d'hydrogène,
ou un stéréoisomère, un tautomère, un hydrate, un solvate ou un sel de celui-ci, ou un mélange de ceux-ci, ou
- un complexe de Gd³⁺ d'un composé de formule (II) 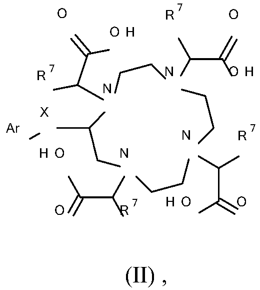 dans laquelle
Ar représente un groupe choisi parmi 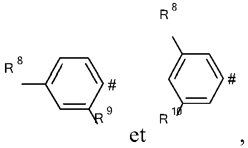
où # représente le point de rattachement à X,
X représente un groupe choisi parmi CH₂, (CH₂)₂, (CH₂)₃, (CH₂)₄ et *-(CH₂)₂-O-CH₂-^{#}, où * représente le point de rattachement à Ar et ^{#} représente le point de rattachement au reste acide acétique,
R⁷ représente un atome d'hydrogène ou un groupe choisi parmi C₁-C₃-alkyle, -CH₂OH, -(CH₂)₂OH et -CH₂OCH₃ ;
R⁸ représente un groupe choisi parmi C₂-C₄-alcoxy, (H₃C-CH₂O)-(CH₂)₂-O-, (H₃C-CH₂O)-(CH₂)₂-O-(CH₂)₂-O- et (H₃C-CH₂O)-(CH₂)₂-O-(CH₂)₂-O-(CH₂)₂-O- ;
R⁹ et R¹⁰ représentent, indépendamment l'un de l'autre, un atome d'hydrogène ;
ou un stéréoisomère, un tautomère, un hydrate, un solvate ou un sel de celui-ci, ou un mélange de ceux-ci, ou
- l'agent de contraste comprend l'une des substances suivantes :
- acide 2-[4,7,10-tris(carboxyméthyl)-1,4,7,10-tétraazacyclododécan-1-yl]acétique de gadolinium(III),
- acide éthoxybenzyl-diéthylènetriaminepentaacétique de gadolinium(III),
- 2-[3,9-bis[1-carboxylato-4-(2,3-dihydroxypropylamino)-4-oxobutyl]-3,6,9,15-tétraazabicyclo[9.3.1]pentadéca-1(15),11,13-trién-6-yl]-5-(2,3-dihydroxypropylamino)-5-oxopentanoate de gadolinium(III),
- [(±)-4-carboxy-5,8,11-tris(carboxyméthyl)-1-phényl-2-oxa-5,8,11-triazatridécan-13-oato(5-)]gadolinate(2-) de dihydrogène,
- [4,10-bis(carboxylatométhyl)-7-{3,6,12,15-tétraoxo-16-[4,7,10-tris(carboxylatométhyl)-1,4,7,10-tétraazacyclododécan-1-yl]-9,9-bis({[({2-[4,7,10-tris(carboxylatométhyl)-1,4,7,10-tétraazacyclododécan-1-yl]propanoyl}amino)acétyl]amino}méthyl)-4,7,11,14-tétraazaheptadécan-2-yl}-1,4,7,10-tétraazacyclododécan-1-yl]acétate de tétragadolinium,
- 2,2',2"-(10-{1-carboxy-2-[2-(4-éthoxyphényl)éthoxy]éthyl}-1,4,7,10-tétraazacyclododécane-1,4,7-triyl)triacétate,
- 2,2',2"-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
- 2,2',2"-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,
- (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
- 2,2',2"-{10-[(1S)-4-(4-butoxyphényl)-1-carboxybutyl]-1,4,7,10-tétraazacyclododécane-1,4,7-triyl}triacétate de gadolinium,
- 5,8-bis(carboxylatométhyl)-2-[2-(méthylamino)-2-oxoéthyl]-10-oxo-2,5,8,11-tétraazadodécane-1-carboxylate de gadolinium(III) hydraté,
- 2-[4-(2-hydroxypropyl)-7,10-bis(2-oxido-2-oxoéthyl)-1,4,7,10-tétraazacyclododécan-1-yl]acétate de gadolinium(III),
- 2,2',2"-[10-((2R,3S)-1,3,4-trihydroxybutan-2-yl)-1,4,7,10-tétraazacyclododécane-1,4,7-triyl]triacétate de gadolinium(III),
- 2,2',2"-{(2*S*)-10-(carboxyméthyl)-2-[4-(2-éthoxyéthoxy)benzyl]-1,4,7,10-tétraazacyclododécane-1,4,7-triyl}triacétate de gadolinium,
- 2,2',2"-[10-(carboxyméthyl)-2-(4-éthoxybenzyl)-1,4,7,10-tétraazacyclododécane-1,4,7-triyl]triacétate de gadolinium.

17. Kit comprenant un produit programme d'ordinateur selon la revendication 14 et un agent de contraste, l'agent de contraste comprenant de préférence
- un complexe de Gd³⁺ d'un composé de formule (I) 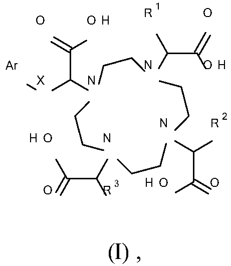 dans laquelle
Ar représente un groupe choisi parmi 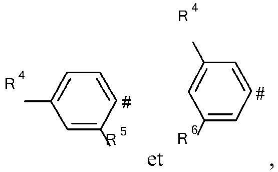
où # représente le point de rattachement à X,
X représente un groupe choisi parmi
CH₂, (CH₂)₂, (CH₂)₃, (CH₂)₄ et *-(CH₂)₂-O-CH₂-^{#},
où * représente le point de rattachement à Ar et ^{#} représente le point de rattachement au reste acide acétique,
R¹, R² et R³ représentent, indépendamment les uns des autres, un atome d'hydrogène ou un groupe choisi parmi C₁-C₃-alkyle, -CH₂OH, -(CH₂)₂OH et -CH₂OCH₃,
R⁴ représente un groupe choisi parmi C₂-C₄-alcoxy, (H₃C-CH₂)-O-(CH₂)₂-O-, (H₃C-CH₂)-O-(CH₂)₂-O-(CH₂)₂-O- et (H₃C-CH₂)-O-(CH₂)₂-O-(CH₂)₂-O-(CH₂)₂-O-,
R⁵ représente un atome d'hydrogène,
et
R⁶ représente un atome d'hydrogène,
ou un stéréoisomère, un tautomère, un hydrate, un solvate ou un sel de celui-ci, ou un mélange de ceux-ci, ou
- un complexe de Gd³⁺ d'un composé de formule (II) 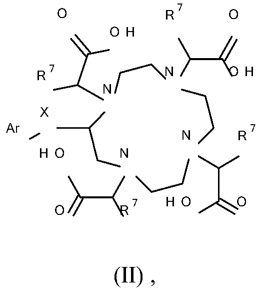 dans laquelle
Ar représente un groupe choisi parmi 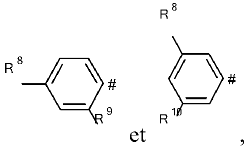
où # représente le point de rattachement à X,
X représente un groupe choisi parmi CH₂, (CH₂)₂, (CH₂)₃, (CH₂)₄ et *-(CH₂)₂-O-CH₂-^{#}, où * représente le point de rattachement à Ar et ^{#} représente le point de rattachement au reste acide acétique,
R⁷ représente un atome d'hydrogène ou un groupe choisi parmi C₁-C₃-alkyle, -CH₂OH, -(CH₂)₂OH et -CH₂OCH₃ ;
R⁸ représente un groupe choisi parmi C₂-C₄-alcoxy, (H₃C-CH₂O)-(CH₂)₂-O-, (H₃C-CH₂O)-(CH₂)₂-O-(CH₂)₂-O- et (H₃C-CH₂O)-(CH₂)₂-O-(CH₂)₂-O-(CH₂)₂-O- ;
R⁹ et R¹⁰ représentent, indépendamment l'un de l'autre, un atome d'hydrogène ;
ou un stéréoisomère, un tautomère, un hydrate, un solvate ou un sel de celui-ci, ou un mélange de ceux-ci, ou
- l'agent de contraste comprend l'une des substances suivantes :
- acide 2-[4,7,10-tris(carboxyméthyl)-1,4,7,10-tétraazacyclododécan-1-yl]acétique de gadolinium(III),
- acide éthoxybenzyl-diéthylènetriaminepentaacétique de gadolinium(III),
- 2-[3,9-bis[1-carboxylato-4-(2,3-dihydroxypropylamino)-4-oxobutyl]-3,6,9,15-tétraazabicyclo[9.3.1]pentadéca-1(15),11,13-trién-6-yl]-5-(2,3-dihydroxypropylamino)-5-oxopentanoate de gadolinium(III),
- [(±)-4-carboxy-5,8,11-tris(carboxyméthyl)-1-phényl-2-oxa-5,8,11-triazatridécan-13-oato(5-)]gadolinate(2-) de dihydrogène,
- [4,10-bis(carboxylatométhyl)-7-{3,6,12,15-tétraoxo-16-[4,7,10-tris(carboxylatométhyl)-1,4,7,10-tétraazacyclododécan-1-yl]-9,9-bis({[({2-[4,7,10-tris(carboxylatométhyl)-1,4,7,10-tétraazacyclododécan-1-yl]propanoyl}amino)acétyl]amino}méthyl)-4,7,11,14-tétraazaheptadécan-2-yl}-1,4,7,10-tétraazacyclododécan-1-yl]acétate de tétragadolinium,
- 2,2',2"-(10-{1-carboxy-2-[2-(4-éthoxyphényl)éthoxy]éthyl}-1,4,7,10-tétraazacyclododécane-1,4,7-triyl)triacétate,
- 2,2',2"-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
- 2,2',2"-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,
- (2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
- 2,2',2"-{10-[(1S)-4-(4-butoxyphényl)-1-carboxybutyl]-1,4,7,10-tétraazacyclododécane-1,4,7-triyl}triacétate de gadolinium,
- 5,8-bis(carboxylatométhyl)-2-[2-(méthylamino)-2-oxoéthyl]-10-oxo-2,5,8,11-tétraazadodécane-1-carboxylate de gadolinium(III) hydraté,
- 2-[4-(2-hydroxypropyl)-7,10-bis(2-oxido-2-oxoéthyl)-1,4,7,10-tétraazacyclododécan-1-yl]acétate de gadolinium(III),
- 2,2',2"-[10-((2R,3S)-1,3,4-trihydroxybutan-2-yl)-1,4,7,10-tétraazacyclododécane-1,4,7-triyl]triacétate de gadolinium(III),
- 2,2',2"-{(2*S*)-10-(carboxyméthyl)-2-[4-(2-éthoxyéthoxy)benzyl]-1,4,7,10-tétraazacyclododécane-1,4,7-triyl}triacétate de gadolinium,
- 2,2',2"-[10-(carboxyméthyl)-2-(4-éthoxybenzyl)-1,4,7,10-tétraazacyclododécane-1,4,7-triyl]triacétate de gadolinium.
